(19) Europäisches Patentamt
European Patent Office
Office européen des brevets

(11) EP 3 581 176 A1

(12) EUROPEAN PATENT APPLICATION

(43) Date of publication:
18.12.2019 Bulletin 2019/51

(51) Int Cl.:
*A61K 9/51* (2006.01) *A61K 38/28* (2006.01)

(21) Application number: 18382412.7

(22) Date of filing: 12.06.2018

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: Universidad de Navarra
31009 Pamplona (Navarra) (ES)

(72) Inventors:
- BROTONS CANTÓ, Ana
E-31008 Pamplona, Navarra (ES)
- GAMAZO DE LA RASILLA, Carlos
E-31008 Pamplona, Navarra (ES)
- INCHAURRAGA CASADAMÓN, Laura
E-31008 Pamplona, Navarra (ES)
- IRACHE GARRETA, Juan Manuel
E-31008 Pamplona, Navarra (ES)
- MARTÍN ARBELLÁ, Nekane
E-31008 Pamplona, Navarra (ES)
- MATÍAS MORO, José
E-31008 Pamplona, Navarra (ES)

(74) Representative: ABG Intellectual Property Law, S.L.
Avenida de Burgos, 16D
Edificio Euromor
28036 Madrid (ES)

# (54) CORE-SHELL ZEIN NANOPARTICLES FOR THE ENCAPSULATION OF COMPOUNDS, PROCESS FOR THEIR PREPARATION AND USES THEREOF

(57) The present invention relates to a core-shell nanoparticle, wherein the core is solid and comprises a zein matrix and a basic amino acid; and the shell comprises a conjugate obtained by reaction of a poly (methyl vinyl ether-co-maleic anhydride) copolymer with a compound having a reactive primary amine group. It also relates to a process for their preparation as well as their use in immunotherapy and in the treatment of disease, such as diabetes.



Printed by Jouve, 75001 PARIS (FR)

**Description**

**FIELD OF THE INVENTION**

The present invention is comprised in the nanotechnology sector, and more particularly in the field of nanosystems for delivery of active ingredients. It relates to mucus-penetrating (MPP) core-shell zein nanoparticles, as well as to a process for obtaining them and pharmaceutical applications thereof.

**BACKGROUND**

Localized delivery of therapeutics via biodegradable nanoparticles often provides advantages over systemic drug administration, including reduced systemic side effects and controlled drug levels at target sites. However, controlled drug delivery at mucosal surfaces has been limited by the presence of the protective mucus layer.

Mucus is a viscoelastic gel that coats all exposed epithelial surfaces not covered by skin, such as respiratory, gastrointestinal, nasopharyngeal, female reproductive tracts and the surface of eye. Mucus efficiently traps conventional particulate drug delivery systems via steric and/or adhesive interactions. As a result of mucus turnover, most therapeutics delivered locally to mucosal surfaces suffer from poor retention and distribution, which limits their efficacy. In addition, some mucosal surfaces, such as those of the mouth, stomach, intestines, colon and vagina, exhibit highly folded epithelial surfaces that are inaccessible to conventional mucoadhesive particles and also to many small molecule drugs and therapeutics. Without maximal distribution with penetration into these deep recesses, much of the epithelium is left susceptible and/or untreated. Additionally, penetration into the folds, presumably containing a much more slowly cleared mucus layer, allows for increased residence time at the epithelial surface.

Zein is the major storage protein found in the corn grain seed and is particularly rich in glutamic acid (21-26%) and non-polar amino acids such as leucine (20%), proline (10%) and alanine (10%), but deficient in basic and acidic amino acids. It is a globular protein belonging to the prolamine group since it tends to have a large number of proline and glutamine amino acids and is characterized by its high insolubility in water. Actually, zein is not a single protein but a mixture of four main fractions ($\alpha$-, $\beta$-, $\gamma$- and $\delta$-zein), differentiated by their solubility and sequence. Alpha-zein is the most abundant (around 80% of total zein) and includes two prolamin groups with apparent molecular weights of 24 and 27 kDa. Beta-zein consists of a methionine-rich polypeptide of 17 kDa and constitutes up to 10% of the total zein; whereas gamma-zein is also composed of two peptides of 27 and 18 kDa. Finally, delta-zein is a minor fraction and has a molecular weight of about 10 kDa. In recent years, this protein has become very important in the scientific and industrial field due to its particular physicochemical properties and to its molecular structure since it has amphiphilic characteristics and can form different self-assembled structures according to the hydrophilic-lipophilic compounds present in the medium (Wang et al., Food Biophysics 2008;3:174-181). Therefore, zein offers a number of potential advantages as a raw material of films, since it is capable of forming hard and hydrophobic coatings with excellent flexibility and compressibility characteristics which are furthermore resistant to microbial attack.

As a result of these properties, new applications have been found for zein as a an adhesive, biodegradable plastic, chewing gum, coating for food products, fiber, cosmetic powders, microencapsulator for pesticides and inks, etc. (Muthuselvi and Dhathathreyan, Colloids and Surfaces B: Biointerfaces 2006;51:39-43). This protein is also used by the pharmaceutical industry to coat capsules for the purpose of protecting, releasing in a controlled manner and masking unwanted tastes and aromas (Shukla and Cheryan, Industrial Crops and Products 2001;13:171-192). Furthermore, it has been proposed for the microencapsulation of insulin, heparin, ivermectin and gitoxin. Stable microparticles/microspheres, even in high humidity and heat conditions, which are furthermore resistant to bacterial attack are generally achieved (US5679377).

Peñalva R, et al (Journal of Drug Delivery Science and Technology, 2015, 30, 450-457) discloses zein nanoparticles encapsulating folic acid which undergo localized release of the folic acid when submitted to intestinal conditions. The authors postulate that this is due to the mucoadhesive properties of the zein nanoparticles described therein. WO 2012007628 A1 discloses nanoparticles comprising a zein matrix and a basic amino acid which form a system for encapsulating and stabilizing biologically active compounds (BACs) for their application in food, in cosmetic and in pharmacy.

Accordingly, nanotechnology has a great potential for revolutionizing the food, cosmetic and pharmaceutical industries, since it allows encapsulating BACs, e.g., essential oils, antioxidants, minerals, prebiotics, flavours, vitamins, etc., for the purpose of obtaining various benefits, for example, increasing the shelf life of the product, reducing the amount of BACs to be used, controlling the release thereof, increasing the bioavailability thereof, producing mucosal vaccines, masking unwanted tastes, and increasing the mucus-penetration capabilities of otherwise inaccessible BACs. The latter is evidenced by the work of X. Li et al in Biomaterials, 2013, 34, 9678-9687, where mucus-penetrating nanoparticles (MPP) made of chitosan with a hydrophilic chain and polyethylene oxide outer layer are shown to have improved intestinal mucus penetration.

H.H. Salman et al., Vaccine, 2007, 25, 8123-8132, disclose nanoparticles made of the copolymer of methyl vinyl ether and maleic anhydride (Gantrez ® AN-119) covered with thiamine. These nanoparticles are reported to encapsulate ovalbumin and to possess mucoadhesive properties with higher intestine affinity over the stomach.

Laura Inchaurraga et al, European Journal of Pharmaceutics and Biopharmaceutics, 2015, 97, 280-289 also disclose Gantrez ® AN-119 nanoparticles, but coated with the mucus-penetrating poly(ethylene glycol) hydrophilic chain. The coating of Gantrez nanoparticles with polyethylene glycol (PEG) was shown to inhibit their mucoadhesive properties and shifted their affinity from the surface of the stomach mucosa to the interior of the mucosa of the small intestine. Similarly, international patent application WO 2016087340 A1 also discloses Gantrez™ AN-119 particles with polyethylene glycol (PEG) as mucus-penetrating capable carriers for the release of biologically active compounds (BACs) through the intestinal mucosa.

Other mucus-penetrating nanoparticles are reported, for example, nanoparticles made of poly(acrylic acid) (PAA) decorated with mucolytic enzymes (Sousa I.P. et al, European Journal of Pharmaceutics and Biopharmaceutics, 2015, 97, 257-264). These nanoparticles are shown once again to display higher affinity towards the intestine mucosa in comparison to the stomach mucosa.

However, the nanoparticle carriers described so far have little residence time inside the stomach, precluding their application in situations where slow release of biologically active compounds inside the gastrointestinal (GI) tract is desirable, such as for vaccination purposes. A vaccine must be cost-effective and safe. However, the majority of vaccines are administered by inoculation with needles, with the concomitant side effects derived from accidents during manipulation including the risk of transmission of blood-borne diseases. Nanoparticles can be exploited as mucosal adjuvants since they can enhance the delivery of the loaded antigen to the gut lymphoid cells due to their ability to be captured and internalized by cells of the mucosal immune system.

Therefore, a need still exists for providing safe and convenient pharmaceutically acceptable carriers capable of enduring longer times inside the gastrointestinal tract while capable of penetrating the intestinal mucosa and releasing biologically active compounds therein.

## SUMMARY OF THE INVENTION

The inventors have surprisingly found a way to provide mucus-penetrating core-shell zein nanoparticles which selectivity can be tuned for the stomach mucosa or for the intestine mucosa. The particles of the invention are also carriers of biologically active compounds (BACs) meaning that the present invention now provides a solution to the problem of extended release administration of BACs within the gastrointestinal tract.

Accordingly, in a first aspect, the invention is directed to a core-shell nanoparticle, wherein:

the core is solid and comprises a zein matrix and at least one basic amino acid; and
the shell comprises a conjugate obtained by reaction of a poly (methyl vinyl ether-co-maleic anhydride) copolymer with a compound having a reactive primary amine group.

In a second aspect of the invention, there is disclosed a process for producing a core-shell nanoparticle as described above, said process comprising the steps of:

a. preparing a hydroalcoholic solution comprising a zein and at least one basic amino acid;

b. adding water to the solution obtained in step (a);

c. reacting a poly (methyl vinyl ether-co-maleic anhydride) copolymer with a compound having a reactive primary amine group in an organic solvent to form a conjugate; and

d. adding the conjugate obtainable in step (c) to the solution obtained in step (b).

In a third aspect, the invention relates to a core-shell nanoparticle obtainable by the process of the invention, as described above.

Various tests performed by the inventors have shown that the provision of a shell layer comprising a conjugate derived from poly (methyl vinyl ether-co-maleic anhydride) copolymer and a compound having a reactive primary amine group onto the solid core of zein and basic amino acid gives rise to nanoparticles which are not only capable of penetrating the mucus layer of intestine, but also reside within the GI tract for longer periods of time. Once ingested, the particles of the invention remain in both stomach and intestine, instead of selectively moving on to the intestine.

Furthermore, the inventors have studied and shown herein that the nanoparticles of the invention are capable of carrying BACs and act on certain metabolic pathways of the host.

Thus, in a fourth aspect of the invention, there is described a composition comprising at least one core-shell nanoparticle of the invention and/or at least one core-shell nanoparticle obtainable by the process of the invention as described above, and a food, nutraceutical, cosmeceutical, or pharmaceutically acceptable vehicle or carrier. In a particular embodiment, said composition of the invention is a pharmaceutical composition.
In a fifth aspect, the invention relates to the nanoparticles of the invention or to the nanoparticles obtainable by the process of the invention or to a pharmaceutical composition comprising said nanoparticles for use in medicine.
Particularly, the nanoparticles of the invention have the ability to modulate the immune response when they are administered to a subject, which allows their use in immunotherapy and vaccination.
Therefore, another aspect of the invention refers to a vaccine or an immunotherapeutic composition comprising at least one core-shell nanoparticle, wherein:

the core is solid and comprises a zein matrix, at least one basic amino acid, and an allergen or an antigen and/or an immunomodulating agent; and

the shell comprises a conjugate obtained by reaction of a poly (methyl vinyl ether-co-maleic anhydride) copolymer with a compound having a reactive primary amine group.

In a particular embodiment, the compound having a reactive primary amine group is mannosamine.
In another aspect, the invention refers to the core-shell nanoparticles of the invention or to the core-shell nanoparticles obtainable by the process of the invention or to a vaccine or immunotherapeutic composition as defined above for use in immunotherapy.
Furthermore, the nanoparticles of the invention also have shown to be capable of controlling the glycaemia in an animal model of diabetes type 1 when they are loaded with compound capable of controlling the glycaemia.
Thus, another aspect of the invention refers to a pharmaceutical composition comprising at least one core-shell nanoparticle, wherein:

the core is solid and comprises a zein matrix, at least one basic amino acid and a compound capable of controlling the glycaemia; and

the shell comprises a conjugate obtained by reaction of a poly (methyl vinyl ether-co-maleic anhydride) copolymer with a compound having a reactive primary amine group;

and a pharmaceutically acceptable carrier or excipient.
In a particular embodiment, the compound having a reactive primary amine group is thiamine.
Finally, another aspect of the invention refers to the core-shell nanoparticles of the invention or to the core-share nanoparticles obtainable by the process of the invention or a pharmaceutical composition as defined in the previous two paragraphs for use in the prevention or treatment of diabetes.

**DESCRIPTION OF THE DRAWINGS**

Figure 1: IR spectra of Gantrez AN polymer (G) and Gantrez-Thiamine conjugate (G-T).

Figure 2: IR spectra of (top to bottom) Gantrez (G), Mannosamine (M), and Gantrez-Mannosamine conjugate (GM). Number 1 shows the typical bands of the anhydride groups. Number 2 illustrates the band corresponding to C-N stretching. Number 3 shows the amide group formed by the binding of mannosamine to the poly(anhydride) chain.

Figure 3: $^1$H-NMR spectra from a) Gantrez AN and b) the conjugate between this copolymer and mannosamine (GM).

Figure 4: Concanavalin A agglutination assay. The turbidity change was reported at 405 nm after 50 seconds, upon incubation of 200 μL (1 mg/mL) of Gantrez-Mannosamine (GM) and control Gantrez polymer (G), with 50 μL Con A (1 mg/mL). Reference controls: (M) Con A + MAN as a mixture of 200 μL mannosamine (0.25 mg/mL) and 50 μL Con A (1 mg/mL); and (Con A) Concanavalin A (0.125 mg/mL). Data reported as mean ± SD (n=3).

Figure 5: IR spectra of (top to bottom) Mannosamine, Gantrez polymer, and Gantrez-mannosamine conjugate (GM2). Number 1 shows the characteristic bands of the anhydride groups. Number 2 shows the band corresponding to the new amide group binding.

Figure 6: Tomography electron microscopy of (A) "naked" core-only zein nanoparticles (NPZ) and (B) zein nanoparticles coated with a shell of Gantrez-Thiamine conjugate (GT-NPZ).

Figure 7: IR spectra of zein nanoparticles (NPZ) and zein nanoparticles coated with a shell of Gantrez-Mannosamine conjugate (GM-NPZ).

Figure 8: Concanavalin A agglutination assay to confirm the presence of mannosamine on the surface of the zein nanoparticles (NPZ or GM-NPZ). Data expressed as mean ±SD, n=3. The turbidity change was reported at 405 nm after 50 seconds, upon incubation of 200 μL (1 mg/mL) of nanoparticles with 50 μL concanvalin A (1 mg/mL). Controls: Mannosamine (M): mixture of 200 μL mannosamine (0.25 mg/mL) and 50 μL Con A (1 mg/mL). ConA: concanavalin A (0.200 mg/mL).

Figure 9: Volume rendered fused SPECT-CT images from representative animals 2 h after administration of $^{99m}$Tc-labelled nanoparticles by oral gavage. NPZ: "naked" core-only nanoparticles; GT-NPZ2: zein nanoparticles coated with a shell of Gantrez-Thiamine (GT) conjugate at a GT/zein ratio (w/w) of 5%.

Figure 10: Fluorescence microscopic visualisation of zein nanoparticles containing a shell of Gantrez-Thiamine (GT) conjugate at a GT/zein ratio (w/w) of 2.5 % and 5 % (GT-NPZ1 and GT-NPZ2 respectively) and control ones, "naked" core-only nanoparticles (NPZ), in a longitudinal section of the ileum of rats 2 hours post administration. A and B: NPZ; C and D: GT-NPZ1; E and F: GT-NPZ2.

Figure 11: Volume rendered fused SPECT-CT images from representative animals from 1 to 8 h after administration of $^{99m}$Tc-labelled nanoparticles by oral gavage. NPZ: "naked" core-only zein nanoparticles; GM-NPZ2 zein nanoparticles coated with a shell of Gantrez-Mannosamine (GM) conjugate at a GM/zein ratio of 0.025 (2.5%).

Figure 12: *In vitro* protein release profile from zein nanoparticles coated with a shell of Gantrez-Mannosamine (GM) conjugate after 24 hours in simulated gastric fluid (SGF) and simulated intestinal fluid (SIF) medium at 37°C.

Figure 13: Cumulative survival rate 40 minutes after challenge with 2 mg of peanut extract. Mice were immunized with one of the following treatments: PE: free peanut extract; PE-NPZ: "naked" core-only zein nanoparticles loading PE; PE-GM-NP: GM nanoparticles loading PE; PE-GM-NPZ: zein nanoparticles coated with a shell of Gantrez-Mannosamine (GM) conjugate and loading PE. A group of sensitized untreated mice (No OIT; control +) and a group of non-sensitized mice (NO sensitization; control -) were also included. Data show individual temperature and mean.

Figure 14: Mouse mast cell protease 1 sera levels after 15 minutes challenge with peanut extract. Mice were immunized with one of the following treatments: PE: free peanut extract; PE-GM-NPZ: zein nanoparticles coated with a shell of Gantrez-Mannosamine (GM) conjugate and loading PE. A group of sensitized untreated mice (No OIT; control +) and a group of non-sensitized mice (NO sensitization; control -) were also included. Data show individual temperature and mean.

Figure 15: OMV-loaded zein nanoparticles. Scanning electron micrograph of spraydried nanoparticles of zein coated with a shell of Gantrez-Mannosamine (GM) conjugate loading OMV from *E. coli* (OMV-GM-NPZ) (A). Antigenicity of the outer membrane vesicles components after encapsulation into nanoparticles (B). Immunoblotting of free OMVs (1) or OMVs extracted from OMV-GM-NPZ (2) against with a pool serum from immunized pigs with OMVs from *E. coli.*

Figure 16: Immunogenicity of OMVs from *E. coli*: OMV-specific antibodies. Serum OMV-specific antibody profile (IgG1, IgG2 or IgA) (A) and fecal OMV-specific IgA profile (B). Data expressed as the mean OD405 nm ± SD at the indicated dilutions (* P<0.05 for immunized mice vs non immunized control group). No-imm: non immunization; F4-GM-NPZ: nanoparticles of zein coated with a shell of Gantrez-Mannosamine (GM) conjugate loading OMVs derived from the serotype F4 of *E. Coli*; F18-GM-NPZ: nanoparticles of zein coated with a shell of Gantrez-Mannosamine (GM) conjugate loading OMVs derived from the serotype F18 of *E. coli;* F4: Free OMVs derived from the serotype F4 of *E. coli;* F18: Free OMVs derived from the serotype F18 of *E. coli.*

Figure 17: Immunogenicity of OMVs from *E. coli*: Labile toxin-specific antibodies. Serum LT-specific antibody profile (IgG1, IgG2) and fecal LT-specific IgA profile. Data are expressed as the mean OD405 nm ± SD at the indicated dilutions (* P<0.05 OMV-GM-NPZ vs OMV-free immunized mice). No-imm: non immunization; F4-GM-NPZ: nanoparticles of zein coated with a shell of Gantrez-Mannosamine (GM) conjugate loading OMVs derived from the

serotype F4 of *E. Coli*; F18-GM-NPZ: nanoparticles of zein coated with a shell of Gantrez-Mannosamine (GM) conjugate loading OMVs derived from the serotype F18 of *E. coli*; F4: Free OMVs derived from the serotype F4 of *E. coli;* F18: Free OMVs derived from the serotype F18 of *E. coli.*

Figure 18A and 18B: Splenic cytokine profile after immunization with free or OMV containing nanoparticles for cytokines IFNγ, IL-17a, IL-10 and IL-22 (A) and for cytokines IL-2, IL-4, IL-6 and TNFα (B). No-imm: non immunization; F4-GM-NPZ: nanoparticles of zein coated with a shell of Gantrez-Mannosamine (GM) conjugate loading OMVs derived from the serotype F4 of *E. Coli*; F18-GM-NPZ: nanoparticles of zein coated with a shell of Gantrez-Mannosamine (GM) conjugate loading OMVs derived from the serotype F18 of *E. coli;* F4: Free OMVs derived from the serotype F4 of *E. coli;* F18: Free OMVs derived from the serotype F18 of *E. coli.*

Figure 19A and 19B: Immunogenicity of OMVs from *E. coli*: OMV-specific antibodies. The immunizations were performed orally in eight weeks pregnant sows at week 0 and 5 by a single (NPI) or a double dose (NPII) of outer membrane vesicles encapsulated into nanoparticles. Serum (IgG, IgA or IgM) **(A)** and fecal OMV-specific antibody profile **(B)** were studied the weeks 0, 5 and 7. Data are expressed as the mean OD405 nm ± SD at the indicated dilutions. No-imm: non immunization; NPI: nanoparticles of zein coated with a shell of Gantrez-Mannosamine (GM) conjugate loading 50 mg F4-F18 OMV and 0.2 mg β-toxin; NPII: nanoparticles of zein coated with a shell of Gantrez-Mannosamine (GM) conjugate loading 100 mg F4-F18 OMV and 0.4 mg β-toxin); SUISENG: commercial intramuscular vaccine.

Figure 20A and 20B: Immunogenicity of β-toxin from *C. perfringens:* β-toxin-specific antibodies. The immunizations were performed orally in eight weeks pregnant sows at weeks 0 and 5 by a single (NPI) or a double dose (NPII) of β-toxin encapsulated into GM-NPZ nanoparticles. Serum (IgG, IgA or IgM) **(A)** and fecal β-toxin-specific antibody profile **(B)** were studied the weeks 0, 5 and 7. Data are expressed as the mean OD405 nm ± SD at the indicated dilutions. No-imm: non immunization; NPI: nanoparticles of zein coated with a shell of Gantrez-Mannosamine (GM) conjugate loading 50 mg F4-F18 OMV and 0.2 mg β-toxin; NPII: nanoparticles of zein coated with a shell of Gantrez-Mannosamine (GM) conjugate loading 100 mg F4-F18 OMV and 0.4 mg β-toxin); SUISENG: commercial intramuscular vaccine.

Figure 21A and 21B: Groups of eight weeks pregnant sows were immunized orally with GM-NPZ based formulations (F4-F18OMV formulated with β-toxin of Clostridium *perfringens*), and compared with the commercial vaccine SUISENG that was administered intramuscularly. Two different doses were used: NPI (50 mg OMV + 0.2 mg β-toxin) and NPII (100 mg OMV + 0.4 mg β-toxin). First dose of vaccines were administered to the sows between 7 and 8 weeks gestation. The second dose was administered between 13 and 14 weeks gestation, 5 weeks after receiving the first dose. A control group of non-immunized sows were also included (No Inm). The evolution of the elicited serum (IgG, IgA and IgM) and fecal (IgG, IgA and IgM) specific antibodies against OMV of E. coli F4 and F18 **(A),** or against β-toxin **(B),** was determined by indirect ELISA 0, 5 and 7 weeks after the first immunization.

Figure 22A and 22B: Analysis of antibodies titre in piglet serum. Groups of eight weeks pregnant sows were immunized orally with GM-NPZ based formulations (F4-F18-OMV formulated with β-toxin of Clostridium *perfringens*), and compared with the commercial vaccine SUISENG that was administered intramuscularly. Two different doses were used: NPI (50 mg OMV + 0.2 mg β-toxin) and NPII (100 mg OMV + 0.4 mg β-toxin). First dose of vaccines were administered to the sows between 7 and 8 weeks gestation. The second dose was administered between 13 and 14 weeks gestation, 5 weeks after receiving the first dose. A control group of non-immunized sows were also included. Serum samples from born piglets were taken and OMV-specific (A) and β-toxin-specific (B) antibody profile (IgG, IgA or IgM) was measured one week after birth. Data are expressed as the mean OD405 nm ± SD at the indicated dilutions.

Figure 23: Tomography Electron Microscopy of zein nanoparticles loading insulin: "naked" core-only zein nanoparticles: (A), or zein nanoparticles coated with a shell of Gantrez-Thiamine (GT) conjugate at a GT/zein ratio of 2.5 % w/w (I-GT-NPZ1) (B), 5.0 % w/w (I-GT-NPZ2) (C) and 10 % w/w (I-GT-NPZ3) (D).

Figure 24: *In vitro* release profiles of the different formulations when incubated in simulated gastric fluid (SGF) and simulated intestinal fluid (SIF): I-NPZ: "naked" core-only zein nanoparticles loaded with insulin (black squares); I-GT-NPZ1: zein nanoparticles loaded with insulin and coated with a shell of Gantrez -Thiamine (GT) conjugate at a ratio GT/zein (w/w) of 2.5% (white squares); I-GT-NPZ2: zein nanoparticles loaded with insulin and coated with a shell of Gantrez -Thiamine conjugate (GT) at a ratio GT/zein (w/w) of 5%(white circles); and I-GT-NPZ3: zein nanoparticles loaded with insulin and coated with a shell of Gantrez -Thiamine (GT) conjugate at a ratio GT/zein

(w/w) of 10% (white triangles).

Figure 25: Plasma glucose levels of diabetic rats treated with different formulations of recombinant human insulin (insulin): i) subcutaneous administration (s.c.) of insulin (circles); ii) oral administration (p.o.) of insulin (triangles); and iii) oral administration (p.o.) of insulin loaded in zein nanoparticles coated with a shell of Gantrez-Thiamine (GT) conjugate at a ratio GT/zein (w/w) of 5% (I-GT-NPZ2, squares). Data represents the mean±S.D., n=6 per group.

Figure 26: Blood insulin levels vs. time after the oral administration of the different formulations of recombinant human insulin (insulin): i) subcutaneous (s.c.) administration of insuline (circles); and ii) insuline loaded in zein nanoparticles coated with a shell of Gantrez-Thiamine (GT) conjugate at a ratio GT/zein (w/w) of 5% (I-GT-NPZ2) (squares). Data expressed as mean ± SD. N=4.

## DETAILED DESCRIPTION OF THE INVENTION

Unless defined otherwise, all technical and scientific terms used herein have the same meaning as commonly understood to one of ordinary skill in the art to which this disclosure belongs.

The term "core-shell nanoparticles" is widely recognized in the art. In the present application, it refers to nanoparticles in which a core (i.e., the inner layer or central part of the nanoparticle) is coated with an outer layer (the shell). The term "particle core" or simply "core" refers to the inner part of a core-shell nanoparticle. The term "particle shell" or simply "shell", refers to the outer layer of the core-shell nanoparticle. In the context of the present invention, the core and the shell are made of different materials, therefore distinguishable materials. Also, as used herein, "nanoparticle" refers to colloidal systems of the type of spheres or similar shapes with an average size less than 1 micrometer (μm), preferably of the order of 10 to 900 nanometers (nm).

As used herein, "average size" refers to the average diameter of the population of nanoparticles which move together in an aqueous medium. The average size of these systems can be measured by standard processes known by the person skilled in the art, and which are described, for example, in the experimental part (see below). In a preferred embodiment, the average size of the nanoparticles of the inventions ranges from 100 to 400 nm.

In the context of the present invention the term "zein" includes any globular protein belonging to the group of prolamines; said protein is generally synthesized during the development of the endosperm (nutritive tissue formed in the embryo sac of seed plants and usually forms a food deposit for the embryo of the seeds of various angiosperm plants). Zein can be obtained from any suitable source, although it is preferably obtained from corn. Various methods and techniques for extracting zein from corn endosperm are known; commercial zein is generally extracted from corn gluten meal (US 2009/0258050).

In the present invention, the source or the grade of zein is not limited to a single zein and, in fact, any zein can be used to put the present invention into practice. By way of illustration, the commercial zeins which can be used in the present invention include, but are not limited to, the zein supplied by Sigma-Aldrich (product number Z 3625); Wako Puras Chemical Industries (product numbers 261-00015, 264-01281 and 260-01283); Spectrum Chemical (product numbers Z1131 and ZE105); ScienceLab units SLZ1150; SJZ Chem-Pharma Company (product name ZEIN (GLIDZIN); Arco Organics (catalog numbers 17931-0000, 17931-1000, and 17931-5000); and Freeman Industries, zein regular grade F4000, zein regular grade F4400, zein special grade F6000, etc. In a particular embodiment, the commercial zein supplied by Sigma-Aldrich (product number Z 3625), obtained from corn, is used.

As used herein, the term "zein" includes both native zein and modified zein. In a particular embodiment, the zein of the invention is native zein. In another particular embodiment of the invention, the zein of the invention is modified zein. The term "modified zein" includes any zein having an amino acid sequence which is normally not naturally-occurring, but which behaves similarly to authentic zeins and which are soluble in alcohol. Amino acid substitutions, especially those which do not substantially modify the hydrophobicity, may be introduced. By way of illustration, amino acid substitutions can be performed within the repeated sections, or a single amino acid can be substituted, and substitutions can also be performed in the segments connecting the domains of repeated sequences. Insertions and substitutions can also be introduced in the carboxyl terminus and the amino terminus of the zein molecule. Additionally, deletions can be performed in the amino acid sequence provided that the resulting protein is functionally equivalent to zein, i.e., that it maintains its properties.

In the nanoparticles of the invention, the term "zein matrix" is to be interpreted as a zein material, as defined above, wherein the amino acids comprised in the zein form a solid continuous network capable of encapsulating a biologically active compound. Said biologically active compound can thus be distributed in the zein matrix. In a particular embodiment, said biologically active compound(s) is/are homogeneously distributed throughout the zein matrix.

In a particular embodiment, the "zein matrix" is a zein obtained from corn. A non-limiting example of the former is the zein supplied by Sigma-Aldrich (product number Z 3625).

Although the term "zein" in the present invention includes both native zein and modified zein, it never embraces

a zein which comprises an Arginine, Histidine or Lysine amount greater than 5 g per 100 g of protein (5% w/w).

The core of the nanoparticles of the invention further comprises at least one free amino acid bearing basic group which improves the solubility of the zein matrix in hydroalcoholic solutions. As used herein, a "basic amino acid" refers to an organic molecule containing an amino group ($-NH_2$) and a carboxyl group (-COOH) with positive, electrically charged, side chains at pH 7.4; said basic amino acid is preferably a basic alpha-amino acid such as lysine, arginine and histidine and is not covalently bound to the zein matrix. Thus, in a particular embodiment, the core-shell nanoparticle of the invention is a nanoparticle wherein the basic amino acid is selected from the group formed by arginine, lysine, histidine and mixtures thereof. Preferably, the basic amino acid is lysine.

As mentioned above, the incorporation of a free basic amino acid dramatically improves the solubility of the zein matrix. In a particular embodiment, the incorporation of a free basic amino acid improves the solubility of the zein matrix in a 1:1 water-ethanol hydroalcoholic solution. This improvement ultimately helps in the solubilisation of biologically active compounds (BACs), both hydrophilic and hydrophobic BACs.

In the present invention, the mass ratio between the amount of free basic amino acid and zein is 1:3-60, preferably 1:4-10, more preferably 1:5-8. In a particular embodiment of the invention, the mass ratio between the amount of free basic amino acid and zein is 1:6.7. The latter can also be expressed as 15 mg of free basic amino acid for each 100 mg of zein.

The core-shell nanoparticle of the invention comprises a shell, said shell comprising a conjugate obtained by reaction of a poly (methyl vinyl ether-co-maleic anhydride) copolymer with a compound having a reactive primary amine group.

As it is used herein, "poly (methyl vinyl ether-co-maleic anhydride)" (PVM/MA) refers to the copolymer of methyl vinyl ether and maleic anhydride. Said PVM/MA copolymer is a known product that can be obtained by conventional methods, for example by means of polymerizing acetylene with maleic anhydride, or it can be obtained on the market. In this sense, the International Specialty Products (ISP) company produces PVM/MA copolymers having different molecular weights marketed under the trade name Gantrez® AN. Therefore, the expressions "PVM/MA", "poly(anhydride)", "Gantrez® AN", "Gantrez AN", or simply "Gantrez", are synonyms, and are indistinctly used in this description.

The Gantrez® AN copolymer contains alternating units of methylvinylether and maleic anhydride, having the formula:

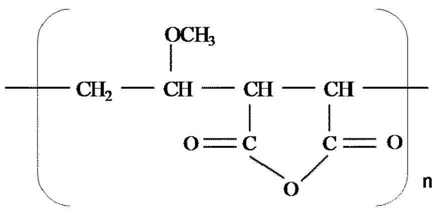

The fundamental character of this polymer is that a maleic anhydride unit (hence the "AN" in "Gantrez® AN") is adjacent to a methylvinylether unit and vice versa, resulting in a true alternating copolymer. The anhydride groups in Gantrez® AN structure allow the chemical interaction of this polymer with nucleophiles through a nucleophilic substitution reaction mechanism.

Gantrez® AN copolymer is supplied as a water-insoluble white powder, and is widely employed for pharmaceutical and medical purposes. The oral toxicity of this copolymer is quite low ($LD_{50}$ in guinea pigs is about 8-9 g/kg) so it has also been used as material for the preparation of nanoparticulate carriers.

In a particular embodiment of the invention, the PVM/MA copolymer has a molecular weight comprised in the range from 80 to 2,500 kDa, preferably from 85 to 2,000 kDa, more preferably from 90 to 220 kDa. In a preferred embodiment, the PVM/MA copolymer has a molecular weight of 130 kDa.

The preparation of polymer conjugates using a PVM/MA copolymer can be achieved by grafting said polymer with a molecule containing reactive primary amine groups ($NH_2$). Without wishing to be bound by any theory, the latter reactive groups are believed to react with the maleic anhydride moiety of the PVM/MA copolymer, opening the ring and forming an amide covalent bond. Illustrative, non-limitative examples of amides of PVM/MA are those wherein the degree of substitution is at least 5%, 10%, 15%, 20%, 25%, 30%, 35%, 40%, 45%, 50%, 55%, 60%, 65%, 70%, 75%, 80%, 85%, 90% or 95%.

The inventors have found that by covering the solid core of zein matrix and at least one basic amino acid with a conjugate obtained by reaction of PVM/MA with a reactive primary amine group, the resulting nanoparticles have the ability to penetrate the mucus layer, which facilitates access to enterocyte cellules where a biologically active ingredient can be delivered.

In a particular embodiment of the invention, the compound having a reactive primary amine group is selected from mannosamine and thiamine.

The nanoparticles of the invention can be used to encapsulate a biologically active compound (BAC).

Therefore, in another particular embodiment, the core-shell nanoparticle of the invention further comprises a BAC. In this case, the core-shell nanoparticle of the invention is occasionally identified in this description as "loaded nanoparticle of the invention" or "loaded core-shell nanoparticle of the invention" to differentiate it from other core-shell nanoparticles of the invention which do not contain BACs (occasionally identified as "empty (core-shell) nanoparticles of the invention"). Also, in the context of the present invention, the "nanoparticle of the invention" is to be identified with the "core-shell nanoparticle" defined in the claims.

More particularly, the BAC is encapsulated or loaded in the solid core of the nanoparticle, being distributed throughout the zein matrix, preferably homogeneously distributed.

As used herein, a "biologically active compound" or "BAC" refers to a compound having therapeutic activity; said compound can be fat-soluble or water-soluble. Non-limiting illustrative examples of BACs according to the present invention include amino acids, antimicrobial agents, flavouring agents, preservatives, sweeteners, steroids, drugs, hormones, lipids, peptides, polynucleotides, polysaccharides, proteins, proteoglycans, flavours, vitamins, fatty acids, antibodies, bacteria, etc.

More particularly, the biologically active compound is selected from allergens, antigens, immunomodulating agents and compounds capable of controlling the glycaemia.

In another particular embodiment of the invention, the compound having a reactive primary amine group is mannosamine.

Thus, in a particular embodiment the invention refers to a core-shell nanoparticle, wherein:

the core is solid and comprises a zein matrix and at least one basic amino acid; and

the shell comprises a conjugate obtained by reaction of a poly (methyl vinyl ether-co-maleic anhydride) copolymer with mannosamine.

Said nanoparticles may further comprise in the core thereof an allergen or an antigen and/or an immunomodulating agent as biologically active compound and release them in a controlled manner. In a particular embodiment, said immunomodulating agent is an immunostimulating agent.

The term "allergen" refers to a substance to which a subject is sensitive and which causes an immune reaction, for example allergenic pollen extracts, allergenic insect extracts, allergenic fungi extracts, allergenic food or food product extracts, components present in saliva, insect pincers or stingers inducing a sensitivity reaction in a subject, components present in plants or in animal epithelium inducing a sensitivity reaction in a subject, etc. Virtually, any allergen can be used in preparing allergen-loaded nanoparticles of the invention; nevertheless, in a particular embodiment, said allergen is a food product extract, particularly a tree nut extract (i.e. an extract from walnut, almond, hazelnut, cashew, pistachio, Brazil nuts or peanut), and more particularly a peanut extract. Peanut extracts are widely used in experimental allergenic models.

The term "antigen" refers to a native or recombinant immunogenic product obtained from a higher organism or from a microorganism, for example a bacterium, a virus, a parasite, a protozoa, a fungus, etc., containing one or more antigenic determinants, for example, structural components of said organism; toxins, for example exotoxins, etc. Practically, any antigen can be used in preparing the antigen-loaded nanoparticles of the invention; nevertheless, in a particular embodiment, said antigen consists of outer membrane vesicles (OMVs) from *E. coli.*

The term "immunomodulating agent" refers to a product that induces, amplifies, attenuates or suppresses specific pathways of the immune system (regulatory adjustment) and may help the body fight cancer, infection, allergies or other diseases. Specific immunomodulating agents, such as monoclonal antibodies, cytokines, and, affect specific parts of the immune system. Nonspecific immunomodulating agents, such as BCG and levamisole, affect the immune system in a general way. Virtually any agent capable of modifying the immune response can be used in preparing immunomodulating agent-loaded nanoparticles of the invention.

The term "immunostimulating agent" refers to a product that is able to specifically or non-specifically enhance an immune response, for example, proteins or peptides working as natural adjuvants stimulating immune system response to the allergen or antigen, bacterial lipopolysaccharides, components of the cell wall of Gram-positive bacteria (for example muramyl dipeptide (MDP)), DNA CpG sequences, plant extracts, mainly saponin plant extracts, etc. Virtually any immunostimulating agent can be used in preparing immunostimulating agent-loaded nanoparticles of the invention.

In a preferred embodiment, the conjugate (PVM/MA- mannosamine)/zein weight ratio does not exceed 0.2, preferably 0.1, more preferably 0.05. In a particular embodiment, the conjugate (PVM/MA-mannosamine)/zein weight ratio ranges between 0.005 and 0.2, preferably between 0.005 and 0.1, more preferably between 0.01 and 0.1, more preferably between 0.005 and 0.05, even more preferably between 0.0125 and 0.05, and even more preferably between 0.025 and 0.05.

In another particular embodiment of the invention, the compound having a reactive primary amine group is thiamine.

Thus, in a particular embodiment, the invention refers to a core-shell nanoparticle, wherein:

the core is solid and comprises a zein matrix and at least one basic amino acid; and
the shell comprises a conjugate obtained by reaction of a poly (methyl vinyl ether-co-maleic anhydride) copolymer with thiamine.

Said nanoparticles may further comprise in the core thereof a compound capable of controlling the glycaemia.
In a particular embodiment of the invention, the compound capable of controlling the glycaemia is selected from the group of insulin, incretins, glucagon-like peptide-1 receptor agonists (i.e., GLP-1, exenatide, etc) and mixtures thereof. In a more particular embodiment the compound capable of controlling the glycaemia is insulin.
In a preferred embodiment, the conjugate (PVM/MA-thiamine)/zein weight ratio does not exceed 0.2, preferably 0.1, more preferably 0.075, even more preferably 0.05. In another preferred embodiment, the conjugate (PVM/MA-thiamine)/zein weight ratio ranges between 0.02 and 0.2, more preferably from 0.005 and 0.1, more preferably from 0.025 and 0.1, even more preferably from 0.005 to 0.05, and even more preferably from 0.025 to 0.05.
A second aspect of the invention is directed at a process for producing a core-shell nanoparticle wherein the core is solid and comprises a zein matrix and at least one basic amino acid; and the shell comprises a conjugate obtained by reaction of a poly (methyl vinyl ether-co-maleic anhydride) copolymer with a compound having a reactive primary amine group; said process comprises the steps of:

a. preparing a hydroalcoholic solution comprising a zein and at least one basic amino acid;

b. adding water to the solution obtained in step a);

c. reacting a poly (methyl vinyl ether-co-maleic anhydride) copolymer with a compound having a reactive primary amine group in an organic solvent to form a conjugate; and

d. adding the conjugate obtainable in step c) to the solution of step b).

The process of the invention as described above is a process for the production of the core-shell nanoparticles of the invention. Therefore, the zein, the basic amino acid, the PVM/MA copolymer and the compound having a reactive primary amine group are as defined above.
As used herein, "hydroalcoholic solution" refers to a solution wherein the solvent comprises water and an alcohol, in variable relative ratios. In a particular embodiment, said hydroalcoholic solution comprises a solution of ethanol in water, in any relative ratio between said compounds. In a particular embodiment, said hydroalcoholic solution comprises between 25% and 80% (w/v) of alcohol, preferably between 30% and 75%, more preferably between 50 and 70%.
In step b) of the process of the invention, water is added in an amount sufficient for the formation of the core-only nanoparticles. Although the amount of water to be added can vary within a wide range, in a particular embodiment, water is added in an amount sufficient for the final proportion of alcohol in the medium to be comprised between 10% and 60% (w/v), preferably between 15% and 40%, more preferably between 25 and 35%.
In a particular embodiment of said process, step (c) leads to the formation of the shell. In another particular embodiment of said process, step (c) further comprises removing the organic solvent. In a preferred embodiment of said process, step (d) consists in adding an aqueous solution of the conjugate obtainable in step (c) to the solution of step (b).
As used herein, "aqueous solution" refers to a solution wherein the solvent comprises water. In a particular embodiment, the solvent essentially consists of water.
The nanoparticles of the invention can further contain a biologically active compound. In this way, in a particular embodiment of the invention, a process for producing a core-shell nanoparticle wherein the core is solid and comprises a zein matrix, at least one basic amino acid and further comprises a biologically active compound; and the shell comprises a conjugate obtained by reaction of a poly (methyl vinyl ether-co-maleic anhydride) copolymer with a compound having a reactive primary amine group; comprises the steps of:

a. preparing a hydroalcoholic solution comprising a zein, at least one basic amino acid and a biologically active compound;

b. adding water to the mixture resulting from step (a);

c. reacting a poly (methyl vinyl ether-co-maleic anhydride) copolymer with a compound having a reactive primary amine group in an organic solvent to form a conjugate; and

d. adding the conjugate obtainable in step (c) to the solution of step (b).

In a particular embodiment, step (a) of said process comprises three steps of (a1) preparing a hydroalcoholic solution comprising a zein and at least one basic amino acid; (a2) preparing a solution of a biologically active compound and (a3) mixing the solutions prepared in steps (a1) and (a2).

In a particular embodiment of said process, step (b) leads to the formation of the core-only nanoparticles. Although the amount of water to be added can vary within a wide range, in a particular embodiment, water is added in an amount sufficient for the final proportion of alcohol in the medium to be comprised between 10% and 60% (w/v), preferably between 15% and 40%, more preferably between 25 and 35%.

In another particular embodiment of said process, step (c) further comprises removing the organic solvent. In a preferred embodiment of said process, step (d) consists in adding an aqueous solution of the conjugate obtainable in step (c) to the solution of step (b).

In yet another particular embodiment of the invention, a process for producing a core-shell nanoparticle wherein the core is solid and comprises a zein matrix, at least one basic amino acid which further comprises a biologically active compound; and the shell comprises a conjugate obtained by reaction of a poly (methyl vinyl ether-co-maleic anhydride) copolymer with a compound having a reactive primary amine group; comprises the steps of:

a. preparing a hydroalcoholic solution (i) containing a zein and at least one basic amino acid;

b. preparing a hydroalcoholic solution (ii) comprising a biologically active compound;

c. mixing said hydroalcoholic solution (i) containing a zein and at least one basic amino acid with said hydroalcoholic solution (ii) comprising a biologically active compound;

d. adding water to the mixture resulting from step (c);

e. reacting a poly (methyl vinyl ether-co-maleic anhydride) copolymer with a compound having a reactive primary amine group in an organic solvent to form a conjugate;

f. adding the conjugate obtainable in step (e), to the solution of step (d).

In the process of the invention, the zein, the basic amino acid and the BAC are diluted in a suitable hydroalcoholic solvent, which can be independently selected from an aqueous solution of the alcohols selected from methanol, ethanol, propanol, isopropanol, butanol, or combinations thereof. In a preferred embodiment, both the hydroalcoholic solutions (i) and the hydroalcoholic solution (ii) are aqueous solutions of ethanol.

In another particular embodiment of the invention, any of the processes disclosed in the previous paragraphs are processes wherein the poly (methyl vinyl ether-co-maleic anhydride) copolymer is reacted with a compound having a reactive primary amine group in an organic solvent, said organic solvent being selected from acetone, dimethylsulfoxide, N,N-dimethylformamide, isopropanol, or combinations thereof. In a preferred embodiment, the organic solvent is acetone.

Once the conjugate is formed, it can be subjected to a purification step (for example by means of filtering) and to a drying step in order to remove the organic solvent and to obtain a dry powder. The organic solvent can be removed by any suitable process, such as evaporation under reduced pressure, evaporation at room temperature, or centrifugation under vacuum. In a particular embodiment, organic solvent is removed by evaporation under reduced pressure. Then, the conjugate in powder form may be dissolved in water for addition to the solution comprising the core-only nanoparticles

In another particular embodiment, the process of the invention comprises additional steps of purifying and/or concentrating the formed core-shell nanoparticles, for example by filtration techniques or centrifugation until a pellet is obtained.

If desired, nanoparticles obtained according to the procedures described above can be dried for their long-term storage and preservation, optionally in the presence of a protective agent. In a particular embodiment of the invention, this additional step is made by means of spray-drying or freeze-drying (lyophilization), preferably by means of spray-drying. Standard cryoprotective agents can be used, preferably at a concentration comprised between 0.1 and 10% by weight with respect to the total composition weight, to facilitate drying.

In a third aspect, the invention relates to a core-shell nanoparticle obtainable by the process of the invention, as described above. The nanoparticles obtainable by the process of the invention fall within the definition of the nanoparticles of the invention.

The inventors have studied and shown herein that the nanoparticles of the invention are capable of carrying BACs and act on certain metabolic pathways of the host. Thus, the core-shell nanoparticles of the invention can be incorporated in a composition or product. Non-limiting, illustrative examples include dietary supplements, food, feed,

nutraceutical products, cosmetic or cosmeceutical products as well as medicinal products.

Therefore, in a fourth aspect, the invention is directed to composition, hereinafter composition of the invention, comprising at least one core-shell nanoparticle of the invention or a at least one core-shell nanoparticle obtainable by the process of the invention as described above and a food, nutraceutical, cosmetical, cosmeceutical, or pharmaceutically acceptable vehicle or carrier. In a particular embodiment, said composition of the invention comprises a plurality of nanoparticles of the invention and/or a plurality of nanoparticles obtainable by the process of the invention.

In a particular embodiment of the fourth aspect of the invention, the composition of the invention is a food or feed composition comprising at least one core-shell nanoparticle of the invention or at least one core-shell nanoparticle obtainable by the process of the invention as described above, further comprising a food or feed carrier. As used herein, the term "food" is any substance or product of any nature, solid or liquid, natural or processed which due to its characteristics, applications, components, preparation and state of preservation, can usually or ideally be used for some of the following purposes: a) as normal nutrition for human beings or animals or as pleasurable foods; or b) as dietetic products, in special cases of human or animal food. The term "feed" includes all the natural materials and finished products of any origin which, separately or conveniently mixed with one another, are suitable as animal food.

In another particular embodiment of the fourth aspect of the invention, the composition of the invention is a nutraceutical composition comprising at least one core-shell nanoparticle of the invention or at least one core-shell nanoparticle obtainable by the process of the invention as described above, further comprising a nutraceutically acceptable vehicle or carrier. As used herein, the term "nutraceutical composition" refers to a composition suitable for use in human beings or animals, comprising one or more natural products with therapeutic action which provide a health benefit or have been associated with disease prevention or reduction, and it includes dietary supplements presented in a non-food matrix (e.g., capsules, powder, etc.) of a concentrated natural bioactive product usually present (or not) in the foods and which, when taken in a dose higher than that existing in those foods, exerts a favorable effect on health which is greater than effect which the normal food may have.

In another particular embodiment of the fourth aspect of the invention, the composition of the invention is a cosmetic composition comprising at least one core-shell nanoparticle of the invention or at least one core-shell nanoparticle obtainable by the process of the invention as described above, further comprising a cosmetically acceptable vehicle or carrier. As used herein, the term "cosmetic composition" refers to a composition suitable for use in personal hygiene of human beings or animals, or in order to enhance the natural beauty or change the body appearance without affecting the structure or functions of the human or animal body, comprising one or more products providing such effects. If desired, the cosmetic composition provided by the invention can also contain one or more cosmetic products, i.e., substances or mixtures intended to be placed in contact with the external parts of the human or animal body

In yet another particular embodiment of the fourth aspect of the invention, the composition of the invention is a cosmeceutical composition comprising at least one core-shell nanoparticle of the invention or at least one core-shell nanoparticle obtainable by the process of the invention as described above, further comprising a cosmeceutically acceptable vehicle or carrier. As used herein, the term "cosmeceutical composition" refers to a composition suitable for use in the body or animal body comprising one or more cosmeceutical products (functional cosmetics, dermoceuticals or active cosmetics), i.e., topical hybrid products with cosmetical-pharmaceutical characteristics containing active ingredients having effect on user's skin, hair and/or nails, at higher and more effective concentrations, therefore they are located in an intermediate level between cosmetic and drug. Illustrative examples of cosmeceutical products include essential oils, ceramides, enzymes, minerals, peptides, vitamins, etc.

In another particular embodiment of the fourth aspect of the invention, the invention is directed to a pharmaceutical composition, hereinafter the pharmaceutical composition of the invention, comprising at least one core-shell nanoparticle of the invention or at least one core-shell nanoparticle obtainable by the process of the invention as described above, further comprising a pharmaceutically acceptable carrier. In a particular embodiment, said pharmaceutical composition of the invention comprises a plurality of nanoparticles of the invention and/or a plurality of nanoparticles obtainable by the process of the invention.

As it is used herein, the concept of acceptable in pharmacy means that a compound or combination of compounds is sufficiently compatible with the other ingredients of a formulation, and not deleterious to the subject up to those levels acceptable by the industry standards.

As it is used herein, the term "carrier" refers to a diluent with which the active ingredient or drug is administered. Examples of pharmaceutically acceptable carriers are known in the state of the art and include phosphate buffered saline solutions, water, emulsions, such as oil/water emulsions, different types of wetting agents, sterile solutions, etc. Preferably, the carriers of the invention are approved by a regulatory agency of the Federal or a state government or listed in the United States Pharmacopoeia or another generally recognized pharmacopeia for use in animals, and more particularly in humans.

In a specific embodiment, said pharmaceutical composition is formulated as a pharmaceutical dosage form suitable for its administration by a route of access to mucosae. In a preferred embodiment, the pharmaceutical composition provided by this invention is orally administered; therefore, the carrier comprises one or more pharmaceutical excipients

acceptable for the administration by oral route. Oral formulations are conventionally prepared by methods known by persons skilled in the art. A review of the different forms of administration of active ingredients, of the excipients to be used, and of the processes for manufacturing them can be found in the book "Tratado de Farmacia Galénica", by C. Faulí i Trillo, 10 Edition, 1993, Luzán 5, S.A. de Ediciones.

The person skilled in the art will understand that the core-shell nanoparticles of the invention or the compositions containing them can be part of a food or feed, or of a nutraceutical, cosmetic, cosmeceutical or pharmaceutical product, which fall within the fourth aspect of the present invention. Said products can be in a liquid, semi-solid or solid form.

The invention also relates to a nanoparticle of the invention or to a nanoparticle obtainable by the process of the invention as defined herein above, or to a pharmaceutical composition of the invention for use in medicine.

It has been found that the nanoparticles of the invention have the ability to modulate the immune response when they are administered to a subject, while maintaining their mucus-penetrating properties, as pointed out in the examples provided herein, which allows their use in immunotherapy and vaccines by different routes of administration.

More particularly, said nanoparticles have shown to be capable of offering protection against a challenge in an animal model of peanut allergy. Additionally, the experimental data have also pointed out that the encapsulation of antigens, such as outer membrane vesicles from *E. coli,* in these nanoparticles increases the elicited antibodies as compared to the administration of the free antigen. Furthermore, the antibody response against a toxin such as heat labile toxin (LT) is significantly higher.

Thus, the nanoparticles of the invention can be used as an adjuvant in immunotherapy and vaccination.

Accordingly, a further aspect of the invention refers to a vaccine or an immunotherapeutic composition comprising at least one core-shell nanoparticle, wherein the core is solid and comprises a zein matrix, at least one basic amino acid, and an allergen or antigen and/or an immunomodulating agent, and the shell comprises a conjugate obtained by reaction of a poly (methyl vinyl ether/maleic acid) copolymer with a compound having a reactive primary amine group. The vaccine or immunotherapeutic composition of the invention can be incorporated in a food or animal feed such as fodder or the like, or directly administered to an animal or human being. Thus, in a preferred embodiment, the vaccine or immunotherapeutic composition of the invention further comprises a pharmaceutically acceptable carrier or excipient.

In a preferred embodiment of this aspect of the invention, the compound having a reactive primary amine group is mannosamine.

In a particular embodiment, said vaccine or immunotherapeutic composition is in a suitable formulation for oral administration, whereas in another particular embodiment, said vaccine or immunotherapeutic composition is in a suitable formulation for parenteral administration.

In another particular embodiment, the vaccine or immunotherapeutic composition of the invention is a composition for use in the prevention and/or treatment of an immune system impairment; said immune system impairment can be a natural (genetic) impairment or an induced impairment, such as an impairment induced by an infectious process, stress, etc.

In another particular embodiment, the vaccine or immunotherapeutic composition of the invention is a composition for use in the prevention and/or treatment of:

- Th1 response-mediated transplant rejection,
- allergies and allergy-associated diseases,
- immunodeficiencies and pathologies derived from said immunodeficiencies,
- infections caused by intracellular pathogens, or
- mucosal infections.

Another aspect of the invention relates to:

- the core-shell nanoparticle of the invention further comprising a biologically active compound selected from the group consisting of allergens, antigens, immunomodulating agent and combinations therefrom; or
- the core-shell nanoparticle obtainable by the process of the invention, further comprising a biologically active compound selected from the group consisting of allergens, antigens, immunomodulating agents and combinations therefrom; or
- a vaccine or an immunotherapeutic composition containing at least one of said nanoparticles as described above;

for use in immunotherapy.

In another aspect, the invention refers to the use of:

- the core-shell nanoparticle of the invention further comprising a biologically active compound selected from the group consisting of allergens, antigens, immunomodulating agents and combinations therefrom; or
- the core-shell nanoparticle obtainable by the process of the invention, further comprising a biologically active compound selected from the group consisting of allergens, antigens, immunomodulating agents and combinations therefrom; or
- a vaccine or an immunotherapeutic composition containing at least one of said nanoparticles as described above;

in the manufacture of a medicament for the prevention and/or treatment of an immune system impairment.
In another aspect, the invention refers to a method of treating or preventing an immune system impairment, method which comprises administering to a subject in need of such a treatment, a therapeutically effective amount of:

- the core-shell nanoparticle of the invention further comprising a biologically active compound selected from the group consisting of allergens, antigens, immunomodulating agent and combinations therefrom; or
- the core-shell nanoparticle obtainable by the process of the invention, further comprising a biologically active compound selected from the group consisting of allergens, antigens, immunomodulating agents and combinations therefrom; or
- a vaccine or an immunotherapeutic composition containing at least one of said nanoparticle as described above.

The characteristics of the immune system impairment referred to above are herein applicable *mutatis mutandi*.
In a preferred embodiment of these aspects of the invention, the compound having a reactive primary amine group is mannosamine.
On the other hand, the examples provided in the experimental part have pointed out that the nanoparticles of the invention are capable of controlling the glycaemia in an animal model of diabetes type 1.
Thus, another aspect refers to a pharmaceutical composition comprising a at least one core-shell nanoparticle, wherein the core is solid and comprises a zein matrix, at least one basic amino acid and a compound capable of controlling the glycaemia, and the shell comprises a conjugate obtained by reaction of a poly (methyl vinyl ether/maleic acid) copolymer with a compound having a reactive primary amine group; and a pharmaceutically acceptable carrier or excipient. In a particular embodiment of this aspect of the invention, the compound capable of controlling the glycaemia is insuline. In another particular embodiment of this aspect of the invention, the compound having a reactive primary amine group is thiamine.
Another aspect of the invention relates to a core-shell nanoparticle, or the a core-shell nanoparticle obtainable by the process of the present invention, wherein the core is solid and comprises a zein matrix, at least one basic amino acid and at least one compound capable of controlling the glycaemia, and the shell comprises a conjugate obtained by reaction of a poly (methyl vinyl ether/maleic acid) copolymer with a compound having a reactive primary amine group, or to a pharmaceutical composition containing at least one of said nanoparticles, for its use in the prevention or treatment of diabetes. In a particular embodiment of this aspect of the invention, the compound capable of controlling the glycaemia is insulin. In another particular embodiment, the compound having a reactive primary amine group is thiamine.
In another aspect, the invention refers to the use of a therapeutically effective amount of:

- the core-shell nanoparticle of the invention, further comprising a biologically active compound capable of controlling the glycaemia; or
- the core-shell nanoparticle obtainable by the process of the invention further comprising a biologically active compound capable of controlling the glycaemia; or
- the pharmaceutical composition containing at least one of said nanoparticles as described above;

in the manufacture of a medicament for the prevention or treatment of diabetes. In a particular embodiment, the compound capable of controlling the glycaemia is insulin. In another particular embodiment of this aspect of the invention, the compound having a reactive primary amine group is thiamine.
In another aspect, the invention refers to a method for the prevention or treatment of diabetes, said method comprises the administration of a therapeutically effective amount of core-shell nanoparticles, wherein the core is solid and comprises a zein matrix, at least one basic amino acid and a compound capable of controlling the glycaemia, and the shell comprises a conjugate obtained by reaction of a poly (methyl vinyl ether/maleic acid) copolymer with a compound having a reactive primary amine group, to a subject in need of such treatment. In a particular embodiment of this aspect of the invention, the compound capable of controlling the glycaemia is insulin.
In another particular embodiment the compound having a reactive primary amine group is thiamine.
The therapeutically effective dose of the nanoparticles to be administered depends on the individual case and, as customary, is to be adapted to the conditions of the individual case for an optimum effect. Thus it depends, of course, on the frequency of administration and on the potency and duration of action of the BAC employed in each case for therapy, but also on the nature and severity of the disease and symptoms, and on the sex, age, weight, co-medication and individual responsiveness of the subject to be treated and on whether the therapy is acute or prophylactic. Doses may be adapted depending on subject weight, and for pediatric administration.

As it is used herein, the term "preventing" refers to keep from happening, existing, or alternatively delaying the onset or recurrence of a disease, disorder, or condition to which such term applies, or of one or more symptoms associated with a disease, disorder, or condition. The term "prevention" refers to the act of preventing, as "preventing" is defined immediately above.

As it is used herein, the term "treating", refers to reversing, alleviating, or inhibiting the progress of the disorder or condition to which such term applies, or one or more symptoms of such disorders or condition. The term "treatment" refers to the act of treating, as "treating" is defined immediately above.

As it is used herein, the term "subject" means animals, in particular mammals such as dogs, cats, pigs, cows, horses, sheep, geese, and humans. Particularly preferred subjects are humans of both sexes.

## EXAMPLES

The present invention will now be described by way of examples which serve to illustrate the construction and testing of illustrative embodiments. However, it is understood that the present invention is not limited in any way to the examples below.

Experiments were performed in compliance with the regulation of the Ethics Committee of the Institution in line with the European legislation on animal experiments (approved protocol 006/15).

In the examples provided below, the following abbreviations are used:

### Empty nanoparticles

PA-NP: poly(anhydride) nanoparticles

GM-NP: "naked" nanoparticle from conjugate Gantrez-Mannosamine

NPZ: "naked" core-only zein nanoparticle

GM-NPZ: zein nanoparticle coated with a shell of Gantrez-Mannosamine conjugate

GT-NPZ: zein nanoparticle coated with with a shell of Gantrez-Thiamine conjugate

### Nanoparticles loading peanut extract

PE-GM-NP: "naked" nanoparticle from conjugate Gantrez-Mannosamine and loading PE

PE-NPZ: "naked" core-only zein nanoparticles loading PE

PE-GM-NPZ: zein nanoparticles coated with a shell of Gantrez-Mannosamine conjugate and loading PE

### Nanoparticles loading antigens from *E. coli*

F4-GM-NPZ: zein nanoparticles coated with a shell of Gantrez-Mannosamine conjugate and loading OMVs derived from the F4 serotype of *E. coli*

F18-GM-NPZ: zein nanoparticles coated with a shell of Gantrez-Mannosamine conjugate and loading OMVs derived from the F18 serotype of *E. coli*

NPI: nanoparticles of zein coated with a shell of Gantrez-Mannosamine conjugate loading 50 mg OMVs derived from the serotypes F4 and F18 of *E. Coli* and 0.2 mg β-toxin of *Clostridium perfringens.*

NPII: nanoparticles of zein coated with a shell of Gantrez-Mannosamine conjugate loading 100 mg OMVs derived from the serotypes F4 and F18 of *E. Coli* and 0.4 mg of β-toxin of *Clostridium perfringens.*

**Nanoparticles loading insuline**

I-NPZ: "naked" core-only zein nanoparticles loading insulin

I-GT-NPZ: zein nanoparticles coated with a shell of Gantrez-Thiamine conjugate and loading insulin

Chemicals

The copolymer of methyl vinyl ether and maleic anhydride or poly(anhydride) (Gantrez® AN 119) can be acquired by Ashland Inc. (Barcelona, Spain). Thiamine hydrochloride, mannosamine, zein, mannitol, lysine, tween20, concanavalin A, agarose, glutaraldehyde, EPON™, 2-Bromoethylamine-hydrobromide, BSA, recombinant human insulin 10 mg/mL (rhINS), citric acid monohydrated, tribasic sodium citrate and NaCl are, for example, from Sigma-Aldrich (Madrid, Spain). Lumogen red is commercially available from Kremer (Germany). Boric acid is available from Merck. Sucrose is available from Fagron (Spain). O-phtalaldehide is available from Invitrogen. All solvents and reagents are used without further purification. Deionized reagent water (18.2 Mcm resistivity) can be prepared by a water purification system (Wasserlab, Pamplona, Spain). Nitrogen gas (ultrapure, > 99) can be produced using an Alltech nitrogen generator (Ingeniería Analítica, Barcelona, Spain). Peanut extract (PE) can be obtained from Diater® Laboratories (Madrid, Spain). 2,2'azino-bis(3-ethylbenzothiazoline-6-sulfonic acid) diammonium salt (ABTS), PBS containing 0.05% Tween 20 (PBS-T) can be purchased from Sigma (Spain). Dulbecco's Phosphate Buffered Saline ($CaCl_2$, $MgCl_2$) is obtainable from Gibco (Germany). The peroxidase immunoconjugates (GAM/IgG1/PO, GAM/IgG2a/PO, GAM/IgA/PO) are available at Nordic Immunology (The Netherlands). Sodium Hydroxide is available from Merck (Darmstadt, Germany), Micro-BCA™ Protein Assay Reagent Kit is available from Pierce® (Rockford, USA) and BD Cytometric Bead Array (CBA) is available from BD Bioscience (USA). TSB can be obtained from bioMérieux (France). RPMI can be obtained from Gibco-BRL (UK). Coomassie brilliant blue and sample buffer can be purchased from Bio Rad (Madrid, Spain). Insulin EIA kit can be purchased from Arbor Assays (Ann Arbor, Michigan, USA).

FT-IR determinations

Spectra were collected with a Nicolet-FTIR Avatar 360 spectrometer (Thermo/Nicolet 360FT-IR E.S.P.Thermo FisherScientific, USA), using a MKII Golden Gate ATR device with resolution of 2 $cm^{-1}$ connected with OMNIC E.S.P. software (Thermo Fisher Scientific, USA). The spectrum obtained was an average of 32 scans.

Elemental analysis

Elemental analysis (C, H, O and N content) was determined in a CHN-900 apparatus from LECO Corporation (Michigan, USA). For this purpose, 1 milligram of each polymer was analyzed by triplicate and the results were expressed as percentage (% w/w).

Proton nuclear magnetic resonance ($^1$H-NMR)

$^1$H-NMR spectra were obtained in a Bruker UltraShield NMR 400 MHZ/ 54 mmÅ Avance 400 (Switzerland). For this purpose, exact amounts of samples were disolved in deuterated DMSO (dimethyl sulfoxide) and the spectra were obtained at ns=6400.

Thiamine quantification

The amount of thiamine covalently attached to the poly(anhydride) was calculated by a modification of a chromatographic method previously described (Salman H.H., et al. Vaccine 25 (2007) 8123-8132). For this purpose, 400 mg Gantrez® AN and 10 mg of thiamine were added to 20 mL acetone. The mixture was heated at 50°C, under magnetic agitation at 400 rpm, for 3 h. The organic solvent was eliminated under reduced pressure in a Buchi R-144 apparatus (BÜCHI Labortechnik AG, Flawil, Switzerland) until the conjugate was totally dry. Once dried, the resulting unpurified conjugate was dissolved in 20 mL of acetone. Then, 40 mL of deionized water were added until the formation of suspension. This suspension was centrifuged for 20 minutes at 21000 rpm and the supernatants were collected for the quantification of thiamine. The analysis was performed in a model 1100 series LC Agilent (Waldbornn, Germany) coupled with a UV diode array detection system. Data were analyzed using the Chem-Station G2171 program. The separation of thiamine was carried out at 40 °C on a reversed-phase Zobrax NH2 narrow-bore column (4.6 mm × 150 mm; particle

size 5 μm) obtained from Agilent (Waldbornn, Germany). The column was protected by a 0.45 μm filter. The mobile phase composition was potassium phosphate buffer 50 mM (pH 6) and methanol (80/20, v/v). The flow rate was set to 1 mL/min and the effluent was monitored with UV detection at 254 nm.

Standard curves were designed over the range of 10-600 μg/mL ($R2 \geq 0.999$) from a thiamine solution in deionized water.

For sample analysis, samples of 1 mL from the supernatants were transferred to auto-sampler vials, capped and placed in the HPLC auto-sampler. Then, 10 μL aliquot was injected onto the HPLC column. Finally, the amount of thiamine associated to the poly(anhydride) backbone was calculated as the difference between the initial amount of thiamine added and the amount of thiamine recovered in the supernatants.

Mucus

Freshly isolated pig intestinal ileum (2 m in length from proximal region) was obtained from a local abattoir (Cardiff, UK) and kept in ice-cold oxygenated phosphate buffered saline (PBS) (no longer than 2 h) prior to sample processing. The ileum was processed into 25 cm lengths with each length incised longitudinally to allow intestinal food and other waste debris to be gently rinsed away by ice-cold PBS. The mucus was then harvested following the approach described in R.A. Cone, Adv. Drug Deliv. Rev., 2009, 61, 75-85, recognised to optimise the yield of not only the loose mucus layer but critically also a high content of the adherent mucus layer. In short, it involved gentle scraping from the intestinal surface by spatula avoiding the shedding of significant intestinal epithelial tissue. Mucus was divided into aliquots (0.5 g) and kept at -20 °C prior to experimentation.

Particle size, zeta potential and yield

The particle size, polydispersity index (PDI) and zeta-potential were determined by photon correlation spectroscopy (PCS) and electrophoretic laser Doppler anemometry respectively, using a Zetasizer analyser system (Brookhaven Instruments Corporation, New York, USA). The diameter of the nanoparticles was determined after dispersion in ultrapure water (1/10) and measured at 25°C by dynamic light scattering angle of 90°C. The zeta potential was determined as follows: 200 μL of the samples were diluted in 2 mL of a 0.1 mM KCl solution adjusted to pH 7.4. The yield of the preparative process of nanoparticles was calculated by gravimetric procedure.

In order to quantify the amount of protein transformed into nanoparticles, 10 mg of the nanoparticle formulation was dispersed in water and centrifuged at 17,000 x g for 20 min. Supernatants were discarded and the pellets were digested with ethanol 75%. Then, the amount of protein was quantified by UV spectrophotometry at 278 nm in an Agilent 8453 system (Agilent Technologies, USA). For analysis, calibration curves were constructed between 90 and 1200 μg/mL ($R2 > 0.999$; quantitation limit = 143 μg/mL). The amount of protein forming nanoparticles in the formulation was estimated as the ratio between the amount of the protein quantified in the pellet of the centrifuged samples and the total amount of protein used for the preparation of nanoparticles and expressed in percentage.

Morphology and shape

The morphology and shape of nanoparticles were evaluated by transmission electron microscopy (TEM). In brief, 20 mg of the spray dried powder containing the nanoparticles were dispersed in 2 mL of cocodylate 0.1 M containing glutaraldehyde 4%. After one hour of incubation, nanoparticles were centrifuged at 1000 rpm (5 min). The pellet was re-suspended in 2 mL water and centrifuged again. Then, 2 mL of osmium 1% was added to the nanoparticles and kept at 4°C during 1 h. The excess of osmium was eliminated by centrifugation at 1000 rpm for 5 minutes. Nanoparticles were re-suspended in 2 mL water and centrifuged again. Then, 200 μL of agarose 2% were added to the nanoparticles, vortexed for 1 minute and kept at 4°C overnight. From this sample, 1 mL was inserted in an embedding flask and dehydrated with alcohols of increasing graduation for 3 h. Then, gelatin capsules were filled with a solution of propylene oxide-EPON™ (1:1) and the samples were inserted. These capsules were incubated at increasing temperatures (37°C, 45°C and 60°C) for the polymerization of the EPON™. Finally, 50-70 nm sections of the samples were obtained with a Leica Ultracut R ultramicrotome (Wetzlar, Germany). The sections were placed in a copper grid and treated with 3% uranil acetate-lead for 5 minutes and completely dried at room temperature. For the visualization of nanoparticles, a Zeiss Libra 120 Transmission Electron Microscope (Oberkochen, Germany) coupled with a digital imaging system Gatan Ultrascan 1000 2k x 2k CCD was used.

Alternatively, the particles of the invention were prepared for TEM as follows: 1.2 mg of formulations were suspended in 1 mL of water and a drop was placed in a copper grid coated with formvar for 30 seconds at room temperature. Samples were cleaned by the addition of deionised water for three times. Then, nanoparticles were treated with 3% uranil acetate for 5 minutes and completely dried at room temperature. For the visualisation of nanoparticles a Zeiss Libra 120 Transmission Electron Microscope (Oberkochen, Germany) coupled with a digital imaging system Gatan

Ultrascan 1000 2k x 2k CCDwas used.

Shape and morphology were also examined by scanning electron microscopy (SEM) in a Zeiss DSM940 digital scanning electron microscope (Oberkochen, Germany) coupled with a digital image system (Point Electronic GmBh, Germany). Prior to examination, nanoparticles were diluted with deionized water and centrifuged at 17000 rpm (Sigma 3 K30, Germany) for 20 minutes. The pellet was dried and shaded with a 9 nm gold layer in a Emitech K 550 Sputter-Coater (Ahsford, UK).

Mannosamine binding assessment

In order to confirm the presence of mannosamine on the surface of the polymer and nanoparticles an *in vitro* agglutination assay was carried out.

The *in vitro* agglutination assay of mannosylated nanoparticles using concanavalin A (Con A), a mannose specific lectin, was applied to confirm the biological activity of the mannosamine linked to the surface of the nanoparticles and the amount of conjugate on the surface of zein modified nanoparticles, using a procedure already described (Salman, Hesham H., et al. "Bioadhesive mannosylated nanoparticles for oral drug delivery." Journal of nanoscience and nanotechnology 6.9-10 (2006): 3203-3209). Indeed, 50 μL of concanavalin A (1 mg/mL) dissolved in Dulbecco's Phosphate-Buffered Saline (DPBS) containing Calcium and Magnesium were added to 200 μL of nanoparticles (1 mg/mL) re-suspended in ultrapure water. The turbidity change was monitored in a spectrophotometer at 405 nm in continuous kinetic measurements (Labsystems iEMS Reader MF, Finland).

Quantification of Lumogen® F red 305

The amount of Lumogen® F Red 305 red loaded in the nanoparticles was quantified by UV-Vis spectrometry at wavelength 580 nm (Labsystems iEMS Reader MF, Vantaa, Finland). For this purpose, the difference between its initial added concentration and the concentration found in the supernatant after the centrifugation of the samples in water (17,000 rpm for 20 min) was calculated. For quantification, standard curves of Lumogen® F Red in ethanol 75% were used (concentration range of 5-30 μg/mL; $R^2 \geq 0.999$).

Radiolabeling of nanoparticles with $^{99m}$Tc

Nanoparticles were radiolabelled with technetium-99m by reduction with stannous chloride as described in P. Areses, et al., Mol. Imaging Biol., 2011, 13, 1215-1223. For this purpose, 0.8-1.0 mg nanoparticles were pre-tinned with 0.05 mg/mL of $SnCl_2$ and subsequently labelled for 30 minutes with 1-2 mCi of freshly eluted $^{99m}$Tc-pertechnetate. The overall procedure was carried out in helium-purged vials. The radiochemical purity was analysed by radiochromatography (Whatman 3MM, NaCl 0.9%). The radiolabelling yield was always over 95%.

Gastro-intestinal transit studies with $^{99m}$Tc-nanoparticles

The gastro-intestinal transit studies were carried out in female Wistar rats weighing 250-300 g. All the procedures were performed following a protocol previously approved by the "Ethical and Biosafety Committee for Research on Animals" at the Institution in line with the European legislation on animal experiments.

Animals were stunned with 2% isoflurane gas for administration of nanoparticles (above 1 mL) by oral gavage, and then quickly awakened. Each animal received one single dose of radiolabelled nanoparticles (1 mCi; 0.8-1.0 mg radiolabelled nanoparticles that were completed with up to 10 mg with unlabelled nanoparticles). SPECT/CT studies were performed three hours after administration of $^{99m}$Tc-nanoparticles, animals were anaesthetised with 2% isoflurane gas and placed in prone position on the gamma-camera (Symbia T2 Truepoint; Siemens Medical System, Malvern, USA). The acquisition parameters for SPECT studies were: 128 x 128 matrix, 90 images, 7 images per second and CT: 110 mAs and 130 Kv, 130 images, slice thickness 3 mm. Fused images were processed using the Syngo MI Applications TrueD software.

Statistical analysis

The physico-chemical characteristics of the nanoparticles were compared using the Student's t test p values<0.05 were considered as significant. For the evaluation of the mMCP-1, statistical comparisons and temperature decrease were performed using the one-way analysis of the variance (ANOVA) and Tukey HSD test p<0.05 was considered as a statistically significant difference. All calculations were performed using Graphpad Prism v6 (Graphpad Software, San Diego, CA).

Multiple particle tracking (MPT) in mucus

The diffusion of nanoparticles through porcine intestinal mucus barrier was assessed by Multiple particle tracking technique following the method described in M. Abdulkarim, et al., Eur. J. Pharm. Biopharm., 2015, 97, 230-238 and J. Rohrer, et al., Eur. J. Pharm. Biopharm., 2016, 98, 90-97. Samples (0.5 g) of porcine intestinal mucus were incubated in glass-bottom MatTek imaging dishes at 37 °C. The fluorescently labelled nanoparticles were inoculated into each 0.5 g mucus sample in a 25 μL aliquot at a suspension concentration of 0.002%. To ensure effective particle distribution following inoculation within the mucus, a 2 h period of equilibration was adopted prior the capture of nanoparticle movements by video microscopy. Video capture involved 2- dimensional imaging on a Leica DM IRB wide-field epifluorescence microscope (x63 magnification oil immersion lens) using a high speed camera (Allied Vision Technologies, Stadtroda, Germany) running at a frame rate of 33 ms i.e. capturing 30 frames $sec^{-1}$; each completed video film comprised 300 frames. For each 0.5 g mucus sample approximately 120 nanoparticles were simultaneously tracked and their movements captured. Videos were imported into Fiji ImageJ software to convert the movement of each nanoparticle into individual nanoparticles trajectories across the full duration of the 10 sec. videos. However, for the analysis of particle diffusion only a 30 frame video period (1 sec) was used, with the criterion that any individual particle tracked must display a continuous presence in the X-Y plane 8 throughout the respective 30 sequential frames. The individual particle trajectories were converted into numeric pixel data (Mosaic Particle Tracker within Fiji ImageJ) which, based on the microscope and video capture settings, was converted into metric distance. The distances moved by every individual particle over a selected time interval (Δt) in the X-Y trajectory were then expressed as a squared displacement (SD). The mean square displacement (MSD) of any single particle (n) represents the geometric mean of that particle"s squared displacements throughout its entire 30-frame trajectory. MSD was determined as follows:

$$MSD_{(n)} = (X\Delta t)\ 2 + (Y\Delta t)\ 2$$

For each nanoparticle type under study an "ensemble mean square displacement" (defined by <MSD>) was then determined for each of the three replicate studies. The Effective Diffusion Coefficient (<Deff>) for a particular nanoparticle type was then calculated by:

$$<Deff>=<MSD> / (4 \times \Delta t)$$

where 4 is a constant relating to the 2-dimensional mode of video capture and Δt is the selected time interval.

The proportion of diffusive particles through the mucus matrix was evaluated by measuring particle diffusion across various time intervals. The following equation was used to determine a Diffusivity Factor (DF) which expresses the effective diffusion coefficient for each individual particle (Deff) across the time intervals (Δt) of 1 second and 0.2 sec, when it is considered as Brownian motion.

$$DF = Deff\ \Delta t{=}1\ sec\ /\ Deff\ \Delta t{=}0.2\ sec$$

Where the individual particle Deff = MSD/ (4 x Δt). Particles with a DF value of 0.9 and greater were defined as diffusive. The proportion of the diffusive particles within a given NP type under study was then calculated and expressed as %Diffusive particles.

In parallel, the particles' diffusion coefficient (D°) in water was calculated by the Stokes-Einstein equation at 37°C. For this purpose, the following was applied:

$$D^{\circ} = \kappa T / 6\pi\eta r$$

Where k is Boltzmann constant, T is absolute temperature, η is water viscosity and r is radius of the particle.

The diffusion of all particles was also expressed as the parameter, %ratio [Deff] / [D°] which provided a measure of the relative efficiency of particle diffusion through mucus when particles intrinsic free Brownian motion in water is taken into account. As such it affords comparison of particle diffusion in mucus after accounting for the impact of a particles surface composition upon its unrestricted diffusion in solution. It is essentially a measure that more directly addresses the relative impact between particles of differing surface physico-chemical properties and the interactions, and the steric hindrance of the mucin network.

**Example 1.** Preparation of Gantrez-Thiamine conjugates (GT)

The conjugate was obtained by the covalent binding of thiamine to the poly(anhydride) backbone. For this purpose, 5 g Gantrez® AN were dissolved in 200 mL acetone. Thiamine (125 mg) was added and the mixture was heated at 50 °C, under magnetic agitation at 400 rpm, for 3 h. Then, the mixture was filtered through a pleated filter paper and the organic solvent was eliminated under reduced pressure in a Buchi R-144 apparatus (BÜCHI Labortechnik AG, Flawil, Switzerland). Finally, the resulting powder was stored at room temperature. The conjugate was named GT.

Figure 1 shows the IR spectra of the GT conjugate as compared with the IR spectra of the Gantrez polymer. The infrared spectroscopy study of the conjugates showed the formation of a new binding at ~1650 $cm^{-1}$ believed to be associated with the stretching of the new amide group v (C=O) originated as a result of the amine group of the thiamine and the anhydride groups of Gantrez AN.

Elemental analysis shows a decrease in C% and O%, confirming a change in the composition of Gantrez-Thiamine derivative compared to unmodified Gantrez (Table 1). On the other hand, the titration of the hydrated polymer and conjugates confirmed a reduction in the amount of free carboxylic groups by the binding of thiamine to Gantrez AN (Table 1). In fact, under the experimental conditions used here, about 13% of the carboxylic acid groups from hydrated poly(anhydride) would be used for the covalent binding of thiamine. In other words, the % of substitution meant that 13 molecules of the maleic anhydride groups of each 100 residues in Gantrez AN had reacted with thiamine to generate amide group and carboxylic acid.

By HPLC, the amount of thiamine associated to the poly(anhydride) backbone was calculated to be 8.7 μg/mg. Finally, with this data, the MW of the conjugate (GT) was 96.33 kDa.

Table 1. Physico-chemical characterization of Gantrez AN and its conjugate with thiamine (GT). For titration and HPLC experiments, data expressed as mean ± SD (n=3).

| Polymer | C% | H% | O% | % Free -COOH | DS (%) | MW (kDa) | Thiamine content (μg/mg G) |
|---|---|---|---|---|---|---|---|
| G | 53.49 | 5.18 | 41.33 | 100 ± 0 | 0 | 95.50 | - |
| GT | 53.19 | 5.58 | 41.23 | 87 ± 1 | 13 | 96.33 | 8.7 ± 0.6 |

DLS showed the values of hydrodynamic radius of Gantrez and Gantrez-Thiamine derivative. In the modified conjugate the hydrodynamic radius increases respect of starting Gantrez copolymer which confirms the presence of thiamine in the polymer.

Table 2. Determination of hydrodynamic radius (Rh) of Gantrez AN conjugate using DLS.

| Polymer | $R_h$ |
|---|---|
| G | 18.96 |
| GT | 41.02 |

**Example 2.** Preparation of Gantrez-Mannosamine conjugates (GM)

The conjugate was obtained by the covalent binding of mannosamine to the poly(anhydride) backbone by two alternative methods.

Method 1 (GM). The first method was based on the spontaneous binding of mannosamine under stirring and heat conditions. Briefly, 1 g Gantrez® AN poly(anhydride) were dissolved in 120 mL acetone. Then, 50 mg mannosamine were added and the mixture was heated at 50°C under magnetic agitation at 400 rpm, for 3 h. Then, the mixture was filtered through a pleated filter paper and the organic solvent was eliminated under reduced pressure in a Buchi R-144 apparatus (BÜCHI Labortechnik AG, Flawil, Switzerland) until the conjugate was totally dry (Yield = 78.37% ± 11.15). Finally, the resulting powder was stored. A mannosamine content of 20.86 ± 0.84 μg/mg of polymer was obtained by OPA assay. A molecular weight (kDa) of 97.54 for the conjugate against 95.50 for the Gantrez polymer was calculated by titration. Zetasizer measurements reveal a mean size of 204 ± 1 nm (PDI of 0.113 ± 0.009) and a zeta-potential of -35.25 ± 0.67.

Characterization of GM conjugates

FTIR analysis was performed to confirm the binding of the reactive functional groups of mannosamine (-OH or -NH2) to the anhydride groups of the polymer. Figure 2 shows the IR spectra for Gantrez AN119 and Gantrez-Mannosamine conjugate. Both compounds presented bands at 1770-1850 $cm^{-1}$ (number 1, Figure 2), that are associated with a typical u(C=O) signal characteristic of anhydride groups. For GM, the IR spectra showed an additional peak at 1707 $cm^{-1}$ (number 2, Figure 2), corresponding to an amide group. This signal confirmed that mannosamine was bound to the Gantrez through the amine functional group (number 3, Figure 2).

Elemental analysis was also performed to verify the modification of Gantrez after grafting Mannosamine which illustrates the existence of new bonds in the synthesized Gantrez-Mannosamine conjugate. The elemental analysis (Table 3) showed differences in the %C, %H, and %O values of the Gantrez-Mannosamine conjugate compared with those of Gantrez. The binding of mannosamine to the polymer backbone slightly decreased the percentage of carbon, whereas the hydrogen and oxygen content increased. In the same way, a very low amount of nitrogen was detected in GM samples (Table 3). The increase in %N resulted from the presence of mannosamine in the new polymer conjugate.

Table 3. Physico-chemical characterization of Gantrez AN (G) and its conjugate with mannosamine (GM). For titration and OPA experiments, data expressed as mean ± SD (n=3). M: mannosamine.

| | **C%** | **H%** | **O%** | **N%** | **% Free-COOH** | **DS (%)** | **MW (kDa)** | **Ligand content (μg/mg M)** |
|---|---|---|---|---|---|---|---|---|
| G | 53.49 | 5.18 | 41.33 | 0 | 100 | 0 | 95.50 | - |
| GM | 52.84 | 5.40 | 41.73 | 0.03 | 80.0 ± 0.7 | 20 | 97.54 | 20.90±0.84 |

Figure 3 shows the $^1$H-NMR spectra of Gantrez and GM. Nevertheless, this technique was not sensitive enough to identify differences between the spectra of both compounds. On the contrary, the titration of the hydrated polymer and conjugates confirmed a reduction in the amount of free carboxylic groups by the binding of mannosamine to Gantrez AN (Table 3). In fact, under the experimental conditions used here, about 20% of the carboxylic acid groups from hydrated poly(anhydride) would be used for the covalent binding of mannosamine. In other words, the percentage of substitution means that 20 molecules of the maleic anhydride groups of each 100 residues in Gantrez AN had reacted with mannosamine to generate amide groups and carboxylic acids. From the OPA analysis, the amount of mannosamine associated with the poly(anhydride) backbone was calculated to be about 21 μg/mg. Finally, with these data, the estimated MW of the conjugate (GM) was 97.54 kDa.

The bioactivity of mannosamine upon binding to Gantrez was assessed by a turbidity assay. Figure 4 shows the increment on the turbidity after incubation of Gantrez modified polymer in presence of Concanavalin A.

Method 2 (GM2). The second method was based on the carbodiimide activation of the carboxylic groups present in the Gantrez molecule. Briefly, 100 mg of Gantrez® AN were suspended in 6 mL anhydride DMSO with a ratio of 1:2 EDC:NHS in $N_2$ atmosphere. The suspension was then incubated for 24 hours at room temperature in the presence of 12.5 mg of mannosamine. The obtained polymer was then dialyzed in PBS and dried by freeze-dryer.

Characterization of GM2 conjugate

FTIR analysis was performed to confirm the binding of the reactive functional groups of mannosamine (-NH2) to the anhydride groups of the Gantrez polymer. Figure 5 shows the IR spectra for mannosamine, Gantrez AN and Gantrez-Mannosamine conjugate (GM2). Both compounds presented bands at 1770-1850 $cm^{-1}$, that are associated with a typical u(C=O) signal characteristic of anhydride groups. For GM2, the IR spectra showed an additional peak at 1706.77 $cm^{-1}$, corresponding to an amide group. This signal confirmed that mannosamine was bound to the Gantrez through the amine functional group.

Regarding elemental analysis (Table 4), the binding of mannosamine to the polymer backbone slightly decreased the percentage of carbon, whereas the hydrogen and oxygen contents increased. In the same way, a small amount of nitrogen was detected in GM2 samples (1.15%). Although $^1$H-NMR spectra of Gantrez and GM2 were done, this technique was not suitable to identify the amount of mannose binding to the polymer backbone due to the reasonably increase in solubility of GM2 in deuterium oxide (data not show). On the contrary, the titration of the hydrated polymer and conjugates confirmed a reduction in the amount of free carboxylic groups by the binding of mannosamine to Gantrez AN (Table 4). In fact, under the experimental conditions used here, about 97% of the carboxylic acid groups from hydrated poly(anhydride) would be used for the covalent binding of mannosamine. In other words, the % of substitution meant that 97 molecules of the maleic anhydride groups out of each 100 residues of Gantrez AN had reacted with mannosamine to generate amide groups and carboxylic acids.

Table 4. Physico-chemical characterization of Gantrez AN (G) and its conjugate with mannosamine (GM2). Content of C, H, O, and N were obtained by elemental analysis. Substitution degree (DS) and % of free carboxylic groups (-COOH) obtained by titration. The substitution degree is expressed as the total amount of Gantrez anhydride groups modified by the presence of mannosamine. For titration experiments, data expressed as mean ± SD (n=3).

| | C% | H% | O% | N% | % Free - COOH | DS (%) |
|---|---|---|---|---|---|---|
| **G** | 53.49 | 5.18 | 41.33 | 0 | 100 ± 0 | 0 |
| **GM2** | 42.46 | 6.20 | 50.19 | 1.15 | 2.4 ± 0.27 | 97 |

**Example 3.** Preparation of zein nanoparticles coated with a shell of the GT conjugate (GT-NPZ)

Zein nanoparticles were prepared following the desolvation procedure previously described by Peñalva R, et al (Journal of Drug Delivery Science and Technology, 2015, 30, 450-457) and then coated with the Gantrez -Thiamine (GT) conjugate of Example 1. Then the resulting nanoparticles were purified, concentrated and, finally, dried. In brief, 200 mg zein and 30 mg lysine were dissolved in 20 mL ethanol 55% and incubated under agitation at RT for 15 minutes. In parallel, a 2% aqueous solution of the Gantrez -Thiamine conjugate of Example 1 was prepared by dispersing the polymer in purified water till complete solubilisation. Nanoparticles were obtained after the addition of 20 mL purified water to the hydroalcoholic solution of zein and lysine. Then, a determined volume of GT solution (0.25, 0.5 or 1 mL) was added and the mixture was maintained under agitation at room temperature (RT) for 30 minutes. The resulting suspension of nanoparticles was purified and concentrated to 20 mL by ultrafiltration through a polysulfone membrane cartridge of 500 kDa pore size (Medica SPA, Medolla, Italy). Finally, 10 mL of a mannitol aqueous solution (4% w/v) was added to the suspension of nanoparticles and the mixture was dried in a Buchi Mini Spray Drier B-290 apparatus (Buchi Labortechnik AG, Switzerland). For this purpose, the following parameters were selected: inlet temperature of 90 °C, outlet temperature of 60 °C, spray-flow of 600 L/h, and aspirator at 100 % of the maximum capacity.

The first approach was to optimize the coating process of zein nanoparticles. For this purpose, the influence of the GT/zein ratio on the physico-chemical properties of nanoparticles was evaluated (Table 5). As expected, by increasing the GT/zein ratio, the mean size and the negative zeta potential of the resulting nanoparticles increased. On the other hand, and under the experimental conditions tested, all the nanoparticle formulations displayed homogeneous characteristics with a PDI below 0.2. All the nanoparticles displayed negative surface charges, however, formulations presenting the GT coating showed slightly more negative surface charges than "bare" nanoparticles.

Table 5. Influence of the GT-to-zein ratio (expressed in percentage) on the physico-chemical properties of the resulting nanoparticles. Data expressed as mean ± SD (n=3).

| FORMULATION | GT/zein ratio (%) | Size (nm) | PDI | Zeta Potential (mV) |
|---|---|---|---|---|
| NPZ | 0 | 235 ± 3 | 0.120 ± 0.014 | -35 ± 4 |
| GT-NPZ1 | 2.5 | 258 ± 2 | 0.091 ± 0.033 | -45 ± 2 |
| GT-NPZ2 | 5.0 | 271 ± 1 | 0.151 ± 0.016 | -45 ± 3 |
| GT-NPZ3 | 10.0 | 345 ± 8 | 0.182 ± 0.078 | -55 ± 5 |

Figure 6 shows TEM photographs of bare ("naked") zein nanoparticles and zein nanoparticles coated with a shell of GT at a GT/zein ratio of 5% (GT-NPZ2). These nanocarriers displayed spherical shape and similar sizes to those obtained by photon correlation spectroscopy. Interestingly, core-shell nanoparticles showed a clear corona (shell-layer) that was missing in uncoated (core-only) zein nanoparticles. The diameter of the corona was calculated to be about 28 nm which represented about 10% of the total size.

**Example 4.** Zein nanoparticles coated with the GM conjugate (GM-NPZ and GM2-NPZ)

Zein nanoparticles were prepared following the desolvation procedure previously described by Peñalva R, et al (Journal of Drug Delivery Science and Technology, 2015, 30, 450-457) and then coated with the Gantrez -Mannosamine conjugate of Example 2 either GM or GM2. Then, the resulting nanoparticles were purified, concentrated and, finally, dried.

Two exemplary procedures are described, both of which yield the desired nanoparticles.

Procedure 1. 200 mg zein and 30 mg D-Lysine were dissolved in 20 mL ethanol 55% and incubated under agitation at RT for 15 minutes. In parallel, a 2% aqueous solution of the Gantrez -Mannosamine conjugates (GM or

GM2) of Example was prepared by dispersing the polymer in purified water till complete solubilisation. Nanoparticles were obtained after the addition of 20 mL purified water to the hydroalcoholic solution of zein and lysine. Then, 0.5 mL of GM or GM2 solution were added and the mixture was maintained under agitation at RT for 30 minutes. The resulting suspension of nanoparticles was purified and concentrated to 20 mL by ultrafiltration through a polysulfone membrane cartridge of 500 kDa pore size (Medica SPA, Medolla, Italy). Finally, 10 mL of a mannitol aqueous solution (4% w/v) was added to the suspension of nanoparticles and the mixture was dried in a Buchi Mini Spray Drier B-290 apparatus (Buchi Labortechnik AG, Switzerland).

Procedure 2. 400 mg zein were dissolved in 40 mL hydroalcoholic solution (ethanol:water 1:1 v/v) in presence of 60 mg of D-Lysine. After complete solubilization of the protein, 40 mL ultrapure water were added in order to form nanoparticles. In parallel, 1 mL of a solution of Gantrez-Mannosamine conjugate in water (10 mg/mL) were added to the suspension of zein nanoparticles and incubated for 30 minutes.

Then, the suspension was purified and concentrated by ultrafiltration through a polysulfone membrane cartridge of 50 kDa pore size (Medica SPA, Italy). Finally, 13.33 mL of an aqueous solution of mannitol (200 mg mannitol per 100 mg Zein) was added to the suspension of zein nanoparticles in order to prevent irreversible aggregation of nanoparticles during the drying step, and the mixture was dried in a Buchi Mini Spray Drier B-290 apparatus (Buchi Labortechnik AG, Switzerland).

For the purpose of spray drying the mixture obtained in either procedure 1 or 2, the following parameters were selected: inlet temperature of 90 °C, outlet temperature of 60 °C, spray-flow of 600 mL/h, and aspirator at 100 % of the maximum capacity.

Table 6 shows the main properties of the zein nanoparticles coated with the GM and GM2 conjugate (GM-NPZ and GM2-NPZ) compared to the zein nanoparticles of Example 4. In the case of GM1, the mean size and the negative zeta potential of the resulting nanoparticles increased. Remarkably, all nanoparticle formulations displayed homogeneous characteristics with a PDI below 0.2. All the nanoparticles displayed negative surface charges, however, formulations presenting the GM coating showed slightly more negative surface charges than the "naked" core-only zein nanoparticles.

Table 6. Influence of the GM/zein ratio (expressed in percentage) on the physico-chemical properties of the resulting nanoparticles. Data expressed as mean ± SD (n=3).

| FORMULATION | GM/zein ratio (%) | Size (nm) | PDI | Zeta Potential (mV) | Yield(%) |
|---|---|---|---|---|---|
| NPZ | 0 | 235±3 | 0.120±0.014 | -35±4 | 60±3 |
| GM-NPZ | 2.5 | 275±3 | 0.096±0.032 | -54±2 | 60±4 |
| GM2-NPZ | 2.5 | 193±8 | 0.128±0.024 | -40±4 | 61±6 |

Figure 7 shows the IR spectra of zein nanoparticles (NPZ) as prepared in Example 5 and Gantrez-Mannosamine coated particles (GM-NPZ). As it can be observed, when coated with Gantrez-Mannosamine, the intensity of the bands in the fingerprint region varies because of the chemical interaction of the polymer with the amine groups of zein aminoacids.

In order to confirm the presence of active mannosamine, an agglutination assay was performed. The turbidity change at 405 nm was measured before and after the addition of Concanavalin A (Con A). Figure 8 shows a modification of the turbidity after adding Con A, which confirms the activity of mannosamine.

**Example 5.** Preparation of zein nanoparticles (NPZ)

As control, "naked" core-only zein nanoparticles (NPZ) were prepared in the same way as described in Example 3 or in Example 4, but in the absence of the coating layer GT or GM, respectively. Zetasizer measurements reveal a mean size of 235 ± 3 nm (PDI of 0.120 ± 0.014) and a zeta-potential of -35 ± 4.

**Example 6.** Preparation of empty Gantrez-Mannosamine nanoparticles (GM-NP)

Nanoparticles based on the above described Gantrez-Mannosamine conjugates (GM) were prepared as follows: 400 mg GM were dissolved in 20 mL acetone. Then, nanoparticles were obtained by the addition, under stirring, of 40 mL of a hydroalcoholic mixture (10 mL water and 29.92 mL EtOH) containing 80 μL $CaCl_2$ (0.8% p/v). The organic solvents were eliminated by evaporation under reduced pressure (Buchi R-144, Switzerland) and the nanoparticles were purified by ultracentrifugation (Sigma 3K30 Rot. 12150-H, UK) at 4°C and 21000 rpm for 20 minutes. Finally, the nanoparticles were re-suspended in 40 mL mannitol 2% and dried by spray-drying in a Buchi Mini Spray Drier B-290 apparatus (Buchi Labortechnik AG, Switzerland) using the following parameters: inlet temperature of 90 °C, outlet temperature of

60 °C, spray-flow of 600 L/h, and aspirator at 100 % of the maximum capacity.

Mannosamine quantification

The amount of mannosamine bound to the polymer structure of Gantrez AN was indirectly quantified by the O-phtalaldehyde (OPA) assay for primary amines OPA method (Salman, Hesham H., et al. "Bioadhesive mannosylated nanoparticles for oral drug delivery." Journal of nanoscience and nanotechnology 6.9-10 (2006): 3203-3209; J R Benson, P E Hare, "O-phthalaldehyde: fluorogenic detection of primary amines in the picomole range. Comparison with fluorescamine and ninhydrin", Proceedings of the National Academy of Sciences Feb 1975, 72 (2) 619-622).

For this purpose 100 mg of un purified Gantrez-Mannosamine conjugate prepared according to Example 2 (GM1) was dissolved in 5 mL acetone. Then, nanoparticles were obtained by the addition of 10 mL of a hydroalcoholic mixture (2.5 mL water and 7.48 mL EtOH) containing 20 μL $CaCl_2$ (0.8% p/v). The organic solvents were eliminated by evaporation under reduced pressure (Buchi R-144, Switzerland) and the nanoparticles were purified by centrifugation (Sigma 3K30 Rot. 12150-H, UK). Supernatants were collected for further characterization.

The amount of mannosamine associated to Gantrez structure was estimated by the difference between the initial amount added and the amount quantified in the supernatant after the purification step using the OPA assay and the molecular weight of the polymer was recalculated according to the percentage of mannosamine linked to Gantrez structure.

**Example 7.** Preparation of fluorescently labelled nanoparticles

For different *in vitro* and *in vivo* studies, fluorescent-labelled nanoparticles were used. For this purpose, 2.5 mL of a 0.04% Lumogen® F red 305 solution in pure ethanol was added to the hydroalcoholic solution containing zein and lysine described in Example 3 or in Example 4. The mixture was maintained under agitation. Then, the desired nanoparticles were prepared, purified and dried following the procedures described in the corresponding Examples. All formulations encapsulated around 3 μg Lumogen® F Red 305 per mg dried powder.

**Example 8.** Preparation of poly(anhydride) nanoparticles (PA-NP)

Nanoparticles based on Gantrez were prepared following the procedures disclosed at P. Arbós, et al., Int. J. Pharm., 2002, 242, 129-136. Briefly, 400 mg of Gantrez® AN were dissolved in 20 mL acetone. Then, the nanoparticles were formed by the addition of 40 mL ethanol followed of the addition of 40 mL purified water. The organic solvents were eliminated under reduced pressure in a BÜCHI R-144 apparatus (BÜCHI Labortechnik AG, Flawil, Switzerland). Then, the nanoparticle suspensions were purified by centrifugation at 5000 × g for 20 min (SIGMA Lab. centrifuges, Osterode am Harz, Germany) using dialysis tubes Vivaspin® 300,000 MWCO (Sartorius AG, Madrid, Spain). Finally, 800 mg lactose dissolved in 40 mL deionized water was added to the pellet and vortexed for 5 minutes. The resulting suspension of nanoparticles was dried in a Buchi Mini Spray Drier B-290 apparatus (BÜCHI Labortechnik AG, Flawil, Switzerland) under the following experimental conditions: inlet temperature of 90 °C, outlet temperature of 60 °C, spray-flow of 600 L/h, and aspirator at 100 % of the maximum capacity. These nanoparticles were named PA-NP. The nanoparticles displayed a size of 213 ± 2 nm and a zeta potential of -53 ± 2 mV.

**Example 9.** Effect of varying the GT-to-zein ratio on particle diffusion

The influence of the GT/zein ratio used for the preparation of nanoparticles on the diffusion through porcine intestinal mucus was assessed by multiple particle tracking (MPT) technique (Table 7).

GT-coated nanoparticles prepared as disclosed in Example 3 at a GT/zein ratio of 2.5% (GT-NPZ1) and 5% (GT-NPZ2) displayed higher ability to diffuse through the mucus than "naked" core-only nanoparticles of Example 5 (NPZ), which showed a similar capacity to diffuse as poly(anhydride) nanoparticles of Example 8 (PA-NP). Actually, the diffusion coefficient in mucus (<Deff>) of GT-NPZ1 was 2.2-fold higher than NPZ, demonstrating their slippery properties. Surprisingly, the <Deff> of GT-NPZ2 was 24-fold higher than "naked" core-only nanoparticles.

Table 7. Diffusion behaviour of the different formulations tested. Data expressed as mean ± SD (n=3). D°: diffusion coefficient in water; <Deff>: diffusion coefficient in mucus; ratio %<Deff>/ D°: relative efficiency of particles diffusion; R: ratio of %<Deff>/ D° of the different formulations tested and %<Deff>/ D° of naked zein nanoparticles; PA-NP: poly(anhydride) nanoparticles; NPZ: zein nanoparticles; GT-NPZ1: GT-coated zein nanoparticles at a GT/zein ratio of 0.025 (2.5%); GT-NPZ2: GT-coated zein nanoparticles at a GT/zein ratio of 0.05 (5%).

| Formulation | D° (water) $cm^2.S^{-1}$ $x10^{-9}$ | <Deff> (mucus) $cm^2. S^{-1}$ $x10^{-9}$ Mean (± SD) | %<Deff>/ D° | R |
|---|---|---|---|---|
| PA-NP | 20.71 | 0.00167 (± 0.096) | 0.0081 | 0.91 |
| NPZ | 19.12 | 0.00171 (± 0.034) | 0.0089 | 1.00 |
| GT-NPZ1 | 17.42 | 0.00376 (± 0.095) | 0.0216 | 2.42 |
| GT-NPZ2 | 16.58 | 0.04129 (± 1.639) | 0.2490 | 27.98 |

**Example 10.** Gastro-intestinal transit studies with $^{99m}$Tc-GT-nanoparticles

Figure 9 shows the comparison of the biodistribution of the nanoparticles of Example 3 (after radiolabelling with $^{99}$mTc) when administered by the oral route to laboratory animals. In all cases, 2 hours post-administration, nanoparticles were visualized in the stomach and the small intestine of animals. However, the intensity of the radioactivity in the stomach of animals was higher for NPZ than for GT-NPZ1 (data not shown) and GT-NPZ2. On the contrary, nanoparticles containing GT as coating material appeared to move faster than uncoated ones because the radioactivity was more intense in the small intestine than in the stomach of animals. Interestingly, no activity was observed in the liver or the lungs of the animals.

**Example 11.** Biodistribution studies with fluorescently labeled nanoparticles

The tissue distribution of nanoparticles in the gastrointestinal mucosa was visualized by fluorescence microscopy. For that purpose, 25 mg of Lumogen® F Red-labelled nanoparticles of Example 3 as prepared according to Example 7 were orally administered to rats as described above. Two hours later, animals were sacrificed by cervical dislocation and the guts were removed. Ileum portions of 1 cm were collected, cleaned with PBS, stored in the tissue proceeding medium OCT™ and frozen at -80°C. Each portion was then cut into 5-μm sections on a cryostat and attached to glass slides. Finally, these samples were fixed with formaldehyde and incubated with DAPI (4',6-diamidino-2-phenylindole) for 15 minutes before the cover assembly. The presence of both fluorescently loaded nanoparticles in the intestinal mucosa and the cell nuclei dyed with DAPI were visualized in a fluorescence microscope (Axioimager M1, Zeiss, Oberkochen, Germany) with a coupled camera (Axiocam ICc3, Zeiss, Oberkochen, Germany) and fluorescent source (HBO 100, Zeiss, Oberkochen, Germany). The images were captured with the software ZEN (Zeiss, Oberkochen, Germany). As control, a suspension of Lumogen® F Red 305 was administered.

Figure 10. shows fluorescence microscopy images of ileum samples from the animals treated with the Lumogen® F Red-labelled nanoparticles of Example 3 according to Example 7. Control formulation (an aqueous suspension of the fluorescent marker) was visualized as large aggregates in the lumen of animals or in contact with the external mucus layer (data not shown). NPZ "naked" core-only nanoparticles are mucoadhesive and displayed a localization mainly restricted to the mucus layer protecting the epithelium in the ileum (Figure 10A and Figure 10B). On the contrary, for nanoparticles containing GT as coating material it was evident that these carriers were capable of reaching the epithelium and interact broadly with the intestinal cells (Figure 10C and Figure 10H). This interaction was higher for GT-NPZ2 than for GT-NPZ1.

**Example 12.** Effect of varying the GM-to-zein ratio on particle diffusion

The influence of the GM/zein ratio used for the preparation of nanoparticles on the diffusion through porcine intestinal mucus was assessed by multiple particle tracking (MPT) technique (Table 8).

GM-coated zein nanoparticles (GM-NPZ) displayed a higher ability to diffuse through the mucus than the "naked" core-only nanoparticles (NPZ), which showed a similar capacity to diffuse to the poly(anhydride) nanoparticles (PA-NP). Actually, the <Deff> of the zein nanoparticles coated with a shell of GM conjugate at a GM/zein ratio of 2.5% (GM-NPZ2) was 4.7-fold higher than "naked" nanoparticles NPZ. Surprisingly, the <Deff> of the zein nanoparticles coated with a shell of GM conjugate at a GM/zein ratio of 5% (GM-NPZ3) was 30-fold higher than "naked" core-only nanoparticles.

Table 8. Diffusion behaviour of the tested formulations. Data expressed as mean ± SD (n=3). D°: diffusion coefficient in water; <Deff>: diffusion coefficient in mucus; ratio %<Deff>/ D°: relative efficiency of particles diffusion; R: ratio of %<Deff>/ D° of the different formulations tested and %<Deff>/ D° of "naked" core-only zein nanoparticles; PA-NP: poly(anhydride) nanoparticles; NPZ: core-only zein nanoparticles; GM-NPZ1: zein nanoparticles coated with a shell of GM conjugate at a GM/zein ratio of 1.25% (w/w); GM-NPZ2: zein nanoparticles coated with a shell of GM conjugate at a GM/zein ratio of 2.5% (w/w); GM-NPZ3: zein nanoparticles coated with a shell of GM conjugate at a GM/zein ratio of 5.0% .

| Formulation | GM/Zein ratio (%) | Water diffusion (D°) $cm^2. S^{-1} x10^{-9}$ | Mucus diffusion (Deff) $cm^2. S^{-1} x10^{-9}$ | % Diffusion coefficient | R |
|---|---|---|---|---|---|
| PA-NP | - | 20.71 | 0.00167 (±0.096) | 0.0081 | 0.91 |
| NPZ | - | 19.12 | 0.00171 (±0.034) | 0.0089 | 1.00 |
| GM-NPZ1 | 1.25 | 21.09 | 0.002 (±0.001) | 0.0102 | 1.14 |
| GM-NPZ2 | 2.50 | 17.23 | 0.007 (±0.006) | 0.0419 | 4.71 |
| GM-NPZ3 | 5.00 | 19.04 | 0.052(±0.041) | 0.2731 | 30.68 |

**Example 13.** Gastro-intestinal transit studies with $^{99m}$Tc-GM-nanoparticles

Figure 11 shows the biodistribution of the nanoparticles of Example 4 (after radiolabeling with $^{99m}$Tc) when administered by the oral route to laboratory animals. In all cases, 2 hours post-administration, nanoparticles were visualized in the stomach and the small intestine of animals. The intensity of the radioactivity in the stomach of the animals was higher for GM-NPZ2 than for NPZ. Surprisingly, the GM-NPZ2 nanoparticles seem to have a higher retention time in the stomach than the uncoated particles and a slower motion towards the intestine. In fact, it is only at 8 hours post-administration when half of the administered nanoparticles of the invention can be found in the intestine. Interestingly, no activity was observed in the liver or the lungs of the animals.

**Oral allergen immunotherapy**

A new allergen delivery system for mucosal immunotherapy based on the nanoparticles of the invention with adjuvant properties was evaluated. The allergen delivery system is capable of offering protection against a challenge in an animal model of peanut allergy. The nanoparticles of the invention allow fulfilling at least the following requirements:

- Accommodate an important payload of allergen proteins;
- Offer protection to the loaded proteins against degradation;
- Facility to decorate the surface with ligands capable to specifically target immunological cells within the mucosa; and
- Controlled release properties of the loaded proteins.

Assessment of the peanut loading capacity of the nanoparticles

Two types of analysis were carried out. In the former, the presence of the different protein components in the nanoparticles was evaluated by SDS-PAGE. This analysis was carried out in order to evaluate the effect of the manufacturing process on the integrity of peanut proteins loaded in nanoparticles. In the latter, the presence of peanut proteins and the payload of nanoparticles were quantified by either microfluidic electrophoresis and a sandwich enzyme-linked immunosorbent assay (ELISA) kit purchased for peanut allergens (Veratox®, Neogen, Scotland, UK).
SDS-PAGE. SDS-Page was performed in 12% acrylamide slabs (Criterion XT, Bio Rad Laboratories, CA) with the discontinuous buffer system of Laemmli and gels stained with Coomasie blue.

Peanut extract (8 mg/mL) was dissolved in SDS-sample buffer 4x and boiled for 10 minutes at 100°C and centrifuged for 10 minutes at 10000 x g. Then, the supernatant was collected and loaded in the electrophoretic gel. The apparent molecular masses of components of the samples proteins were determined by comparison with standard molecular weight markers (Rainbow RPN756, Amersham Pharmacia Biotech).

For zein based nanoparticles, 12 mg of nanoparticles were re-suspended in 1 mL water and centrifuged (28000

x g) for 20 minutes at 4°C in order to remove the sugar. Then, the pellet was dissolved in 1 mL EtOH 70% and stored for 1 hour at - 80°C. Samples were centrifuged at 28000 x g (20 minutes at 4°C) and the sediment was washed with EtOH 70% and stored for 30 minutes at -80°C. Under the same conditions, pellets were re-suspended in electrophoretic buffer (4X Laemmli Sample Buffer) and boiled for 10 minutes at 100°C. Then, centrifuge for 10 minutes at 10000 x g. The obtained supernatant was collected and loaded in the electrophoretic gel. The apparent molecular masses of components of the samples proteins were determined by comparison with standard molecular weight markers (Rainbow RPN756, Amersham Pharmacia Biotech).

For Gantrez based nanoparticles, 12 mg of nanoparticles were re-suspended in 1 mL water and centrifuged (28000 x g) for 20 minutes at 4°C in order to remove the sugar. Then, the pellet was dissolved in 1 mL acetone/dimethylformamide (3:1 v/v) and stored for 1 hour at -80°C. Samples were centrifuged at 28000 x g (20 minutes at 4°C) and the sediment was washed with acetone and stored for 30 minutes at -80°C. Under the same conditions, pellets were resuspended in electrophoretic buffer (4X Laemmli Sample Buffer) and boiled for 10 minutes at 100°C. Then, centrifuge for 10 minutes at 10000 x g. The obtained supernatant was collected and loaded in the electrophoretic gel. The apparent molecular masses of components of the samples proteins were determined by comparison with standard molecular weight markers (Rainbow RPN756, Amersham Pharmacia Biotech).

ELISA and microfluidic electrophoresis. In order to quantify the amount of Peanut proteins loaded in the nanoparticles two different strategies were used: microfluidic electrophoresis and a sandwich enzyme-linked immunosorbent assay (ELISA) kit purchased for peanut allergens (Veratox®, Neogen, Scotland, UK).

Firstly, the supernatant obtained in the purification step of the synthesis of the nanoparticles was used for microfluidic electrophoresis as an indirect method of detection of peanut in the particles using an Experion™ Automated Electrophoresis System (Bio Rad, US). Samples were evaluated under reducing conditions using 2-mercaptoethanol. The data obtained was processed using the software Experion System. Samples of free protein (peanut and zein) were used as controls. All of these samples were evaluated after treatment with β-mercaptoethanol and heat. Then, the samples were treated following the protocol of the Experion System Pro260 Analysis Kit (Bio-Rad Lab., USA). Once the samples and controls were loaded into the chip, they were analysed by the Experion™ Automated Electrophoresis System (Bio Rad, US). The results were obtained as densitometric bands in a virtual gel. Each band corresponded to a different sample. The Experion software identifies the different size peaks and expressed them in kilodaltons ("kDa") in the system control band.

Secondly, in order to have an accurate quantification, peanut extract loaded in the particles was measured by a sandwich enzyme-linked immunosorbent assay (ELISA) kit purchased from Veratox® for peanut allergy (Neogen, Scotland, UK) following manufacturer’s instructions.

**Example 14.** Peanut extract-loaded GM-NP nanoparticles (PE-GM-NP)

400 mg of GM obtained according to Example 2 were dissolved in 20 mL acetone and incubated for 45 minutes with 12 mg of peanut extract suspended in 100 μL of acid water (pH: 3). Then, nanoparticles were obtained by the addition, under magnetic stirring, of 40 mL of a hydroalcoholic mixture (10 mL $H_2O$+29.920 mL EtOH) containing 80 μL $CaCl_2$ (0.8% p/v). The organic solvents were eliminated by evaporation under reduced pressure (Buchi R-144, Switzerland) and the nanoparticles were purified by ultracentrifugation (Sigma 3K30 Rot. 12150-H, UK) at 4 °C and 21000 rpm for 20 minutes. Finally, the resulting pellet was dispersed in 40 mL of an aqueous solution of mannitol 2% and dried by spray-drying in a Buchi Mini Spray Drier B-290 apparatus (Buchi Labortechnik AG, Switzerland). The parameters selected for this purpose were: inlet temperature of 90 °C, outlet temperature of 60 °C, spray-flow of 600 L/h, and aspirator at 100% of the maximum capacity.

**Example 15.** Peanut extract-loaded GM-NPZ nanoparticles (PE-GM-NPZ) and controls

400 mg of zein and 40 mg of dialyzed peanut extract were dissolved and incubated for 1 hour, under magnetic stirring at RT, in 40 mL of a hydroalcoholic solution (ethanol: water 1:1 by vol.) in presence of 60 mg of D-Lysine. Nanoparticles were then produced by desolvation after the addition of 40 mL of water. Once the nanoparticles were obtained, 1 mL of hydrolyzed GM conjugate (obtained by dissolving 100 mg conjugate in 10 mL water) was added and the mixture was maintained under agitation at RT for 30 minutes. The suspension was concentrated and purified by ultrafiltration through a polysulfone membrane cartridge of 50 kDa pore size (Medica SPA, Italy) and then dried by spray-dryer (Mini-Spray Dryer; Büchi®, Switzerland) with 800 mg of mannitol as protector.

The parameters selected for this purpose were: inlet temperature of 90 °C, outlet temperature of 60 °C, spray-flow of 600 L/h, and aspirator at 90 % of the maximum capacity.

As controls, PE-loaded "naked" core-only zein nanoparticles (PE-NPZ) were prepared with 40 mg PE in the same way as described above but in the absence of GM. In a similar way, PE-loaded nanoparticles from GM (PE-GM-NP) were prepared as described in Example 6.

The yields of polymer being converted into nanoparticles according to gravimetric analysis were approximately 80%, decreasing to 66% when peanut extract was present.

Table 9. Physical and chemical characteristics of the nanoparticles of Example 15. PE-GM-NP: nanoparticles of Gantrez-Mannosamine conjugate loading PE; PE-NPZ: "naked" core-only zein nanoparticles loading PE; PE-GM-NPZ: zein nanoparticles coated with a shell of Gantrez-Mannosamine (GM) conjugate and loading PE.

| | Mean size (nm) | PDI | Zeta Potential (mV) | PE loading (μg/mg NP) | Encapsulation Efficiency (EE, %) |
|---|---|---|---|---|---|
| PE-GM-NP | 223±4 | 0.19±0.01 | -35.7±0.7 | 25.2 | 83 |
| PE-NPZ | 236±1 | 0.12±0.01 | -49.0±0.8 | 101.2 | 88 |
| PE-GM-NPZ | 225±1 | 0.14±0.01 | -43.2±0.5 | 19.1 | 92 |

The size of the loaded zein-based nanoparticles coated with Gantrez-Mannosamine conjugate (PE-GM-NPZ) was slightly smaller than the empty particles (GM-NPZ), however no significant statistical differences are observed in any case ($p<0.05$). Overall, nanoparticle-batches were found to be homogeneous. However, the encapsulation of peanut extract decreased to some extent the negative charge of empty nanoparticles.

**Example 16.** *In vitro* release studies with peanut extract-loaded GM-NPZ nanoparticles

The release studies were performed under sink conditions by using simulated gastric fluid (SGF, pH 1.2) and simulated intestinal fluids (SIF, pH 6.8), supplemented with Tween 20 (1% w/v). For these purposes, Float-A-Lyzer devices with a Molecular Weight Cut-Off (MWCO) of 300 kDa (Spectrum Labs.com) were used. After washing the dialysis bags with ethanol 10% for 10 min and, then, with water, the system was filled with 5 mL of nanoparticles dispersed in SGF and, then, placed into a vessel containing 660 mL SGF. The vessel was maintained under magnetic agitation and 200 μL samples were withdrawn at fixed time intervals and replaced with equal volumes of SGF. After two hours of incubation in this gastric fluid, the system was placed into a second vessel with 660 mL SIF. Again, at fixed times, 200 μL were withdrawn and replaced with free SIF.

The amount of PE released from nanoparticles was quantified with the sandwich enzyme-linked immunosorbent assay (ELISA) kit for peanut allergy (Veratox®, Neogen, Scotland, UK).

Peanut extract release kinetics from zein particles decorated with Gantrez-Mannosamine conjugate (PE-GM-NPZ) were evaluated in two different media: simulated gastric fluid (SGF, pH 1.2) and simulated intestinal fluid (SIF, pH 6.8) for 24 hours at 37°C.

There was no release of PE from the particles in SGF. In contrast, the release in SIF started in the first 15 minutes. Figure 12 shows that both formulations present a similar profile characterized by a burst effect followed by a sustained period of slow release.

**Example 17.** Sensitization, Immunotherapy and Challenge

Experiments were performed in compliance with the regulation of the Ethics Committee of the Institution in line with the European legislation on animal experiments (approved protocol 006/15).

ICR female mice (20 grams) were sensitized by oral administration of a mixture of peanut butter (4.35 mg with an approx. content of 1 mg protein) and 5 μg cholera toxin in a total volume of 200 μL of saline solution on days 1, 7, 15 and 21. Moreover, a tape stripping was applied to obtain a higher sensitization. Mice were shave and barrier-disrupted on back skin. Percutaneous sensitization in the damaged skin was performed by topical application of 100 μg peanut extract in 100 μL saline solution onto the barrier-disrupted skin.

Thus, on days 25, 28 and 35 the animals received one oral dose of 1 mg peanut extract either re-suspended in water or incorporated into the nanoparticles of Example 14 (PE-GM-NP) and of Example 15 (PE-NPZ and PE-GM-NPZ, respectively).

A group of non-immunized mice (No sensitization) and a group of sensitized animals but not treated (no OIT) were also included in the study as controls. Finally, on days 44 and 45 animals were challenged by an injection of 2 mg peanut extract by intraperitoneal route in order to provoke an anaphylactic shock in the sensitized animals. In order to analyze the intensity of the anaphylaxis shock, the following parameters were recorded before challenge and 30 min post-challenge: activity, bristly hair and cyanosis. Clinical anaphylactic reactions were scored by two independent observers. Piloerection was scored as follows: (-) normal mouse, (+) weak reaction and/or scratching of the nose and head,

(++) moderate, and (+++) strong piloerection. Cyanosis was classified as absent, weak, moderate and strong. In a similar way, the mobility of animals was scored as very low (no reaction after pushing), low (arched back and low movements) or normal.

Furthermore, levels of mouse mast cell protease-1 (mMCP-1) in serum were measured with an mMcp-1 enzyme-linked immunosorbent assay kit purchased from eBioscience, Affimetrix, following the manufacturer's instructions. A commercial BD™ Cytometric Bead Array Mouse Th1/Th2/Th17 Cytokine Kit (CBA) was used in order to determine levels of TNF-$\gamma$, which is consider a good marker of Th2 response.

Table 10. shows the overall anaphylactic symptoms score at time 30 minutes. Animals of the positive control group (no OIT) that did not receive any treatment, displayed low mobility and signs of bristly hair and cyanosis. On the contrary, animals treated with peanut-loaded zein nanoparticles coated with Gantrez-Manosamine conjugate displayed an almost normal behaviour and an evident better symptomatology than those animals receiving peanut as oral suspension, which appeared to be immobile or with high difficulty to coordinate any simple movement. Nevertheless, the most determinant data was in terms of mortality after the challenge.

Figure 13. shows the survival rate 40 minutes after challenge. Animals treated with PE2-GM-NPZ showed a survival of 70% whereas animals treated with free peanut extract have a survival of 36%.

Table 10. Anaphylactic symptoms score at time 30 minutes

| Time: 30 minutes | | | | |
|---|---|---|---|---|
| Treatment | T decrease (°C) | Piloerection | Reduction of the mobility | Cyanosis |
| No sensitization | 0.8±0.6 | - | - | - |
| No Oral IT | -4.7±2.6 | +++ | +++ | +++ |
| Free PE | -5.2±3.2 | ++ | +++ | ++ |
| PE-NPZ | -4.6±4.7 | + | - | + |
| PE-GM-NP | -3.6±2.7 | ++ | ++ | + |
| PE2-GM-NPZ | -5.4±3.7 | - | + | + |

*Severity of the symptoms: (-) absent, (+) weak, (++) moderate, (+++) strong*

Figure 14 shows mMCPT-1 (mouse mast cell protease-1) mMCPTt-1 sera levels, 15 minutes after challenge with peanut in the surviving animals. The protease is highly elevated in anaphylactic process and it is considered as a good marker to quantify the intensity of the anaphylaxis. The group treated with PE2-GM-NPZ showed a lower mMCPT-1 concentration than control groups, confirming the capability of this formulation to protect the sensitized animals against the challenge.

**Nanoparticles for oral vaccination**

Post-weaning diarrhoea (PWD) is a serious threat to the economic success of the swine industry, due both to losses as a result of mortalities, as well as reduced growth performance of surviving pigs. It is estimated that 50% of piglet mortality worldwide is associated to the causative agent of PWD, enterotoxigenic *Escherichia coli* (ETEC). Vaccination is the best choice to reduce the use of antibiotics in pig farming and likely the most effective approach to control pathogenic bacterial infections. Adequate vaccination of the sows before farrowing might protect piglets by passive immunization through colostrum and milk. In fact, *E. coli* vaccines have been used for many years to stimulate mucosal immunity of the sows and, hence, to elicit specific IgG and IgA antibodies in colostrum conferring protection to the neonatal piglets. However, the available parenteral vaccines to prevent post-weaning diarrhoea tend to stimulate the systemic rather than mucosal immune system. In contrast to parenteral vaccines, mucosal vaccines are safer and induce immunity in both, systemically, as well as in mucosal tissues. The use of outer membrane vesicles (OMVs) from *E. coli* as the source of relevant antigens and zein nanoparticles as the necessary oral adjuvant is disclosed herein. The adjuvant effect of OMVs encapsulated in zein nanoparticles comparing with oral administration of free OMVs was analysed in BALB/c mice and pregnant sows.

Preparation and characterization of the vaccine complex from *Escherichia coli*

The vaccine complex consisting in OMVs were extracted from the two serotypes mainly involved in bacterial enterocolotic in piglets of *E. coli:* F4 (71709 CECT, Valencia, Spain) and F18 isolated from pig (Agropecuaria Obanos, Spain). Vesicles were purified from a method adapted from Camacho *et al.* (Camacho AI, Souza-Reboucas J, Irache JM, Gamazo C. "Towards a non-living vaccine against Shigella flexneri: from the inactivation procedure to protection

studies." Methods 2013; 60:264-8) Bacteria were grown in TSB under shaking overnight to early stationary phase (37 °C, 125 rpm). Then, bacteria were inactivated during 6 hours with a solution of ethylenimine and formaldehyde (6 mM BEI-0.06% FA, 6 h, 37 °C). BEI was prepared as a 0.1 M solution by cyclization of 0.1 M 2-bromoethylamine hydrobromide (Sigma) in 0.175 M NaOH solution for 1 h (Camacho et al. "Towards a non-living vaccine against Shigella flexneri: from the inactivation procedure to protection studies." Methods 2013; 60:264-8). Cells were discarded by centrifugation at 10,000 g during 10 min. Supernatant was filtered through a 0.45 μm Durapore PVDF filter (Millipore) and purified by tangential filtration using a 300-kDa concentration unit (Millipore). The retenate was reduced into a volume of 100 mL, freezed and finally lyophilize. Total protein content was determined by the method of Lowry, with bovine serum albumin (BSA) as standard. Lypopolysaccharide (LPS) content was determined by KDO assay. The proteic profile was determined by SDS-PAGE and protemic analysis. The morphology of the vesicles was examined by Field Emission Scanning Electron Microscope. Scanning electron microscopy revealed that the OMVs released by *E. coli* F4 or F18 strains present a spherical shape with wide range of diameters from 15 to 200 nm.

The yield obtained in the process of isolating OMVs was 23.0±0.1 μg/mg, referred to the original cell culture wet weight. Protein content determined by Lowry's assay (Lowry O.H, R. N. J. (1951). "Protein measurement by the Folin phenol reagent." J. Biol.Chem. 193: 265-275) was 41.9 ± 2.6% for the OMVs-F4, and 43.2 ± 3.1% for OMVs-F18; whereas the LPS content measured by KDO assay (Osborn, M. J. (1963). "Studies on the gram negative cell wall. I. Evidence for the role of the 2-Keto, 3-deoxyoctonate in the lipopolysaccharide of Salmonella thiphymurium." Proc. Natl. Acad. Sci. USA 50: 499-506) was 15.8 ± 1.9% and 13.5 ± 2.2% for OMVs-F4 and OMVs-F18, respectively. To study the protein profile of the OMVs, an SDS-PAGE analysis was performed and, after that, the identification of each band was confirmed by proteomic analysis. The results revealed that the OMVs contained main immunodominant antigenic proteins of *E. coli* such as flagellin; OmpC/OmpA, FaeG, among others.

OMVs analysis content

To measure the amount of OMVs extract encapsulated in nanoparticles, the nanoparticles were re-suspended in water and centrifuged. The supernatant containing mannitol and free OMVs was removed and pellet containing nanoparticles re-suspended in 70% ethanol, which partly dissolved protein P. Another centrifugation resulted in pellet containing OMVs and proteic residues. This pellet was re-suspended in a mixture of 20% DMSO in 85% ethanol and a last centrifugation was performed. After removing the supernatant, the final pellet was obtained, which was used for further experiments.

Structural integrity and antigenicity of the loaded OMVs

To study the efficiency of encapsulation, SDS-PAGE and immunoblotting were performed. In brief, 10 mg/mL of loaded nanoparticles were suspended in ultrapure water and centrifuged (10,000 g, 20 min). Then, the supernatant was discarded and the pellet re-suspended in 10 mL of ethanol 70% and centrifuged at 10,000 g for 20 min. The supernatant was discarded and the sediment reconstituted in 10 mL of 20% DMSO- 85% ethanol. After centrifugation under the same conditions, the supernatant was discarded and the pellet re-constituted in 100 μL of sample buffer (Tris-HCl 62.5 mM, pH 6.8; 10 % glycerol; 2 % sodium dodecyl sulfate, SDS; 5% β-mercaptoethanol and bromophenol blue) and boiled for 5 min. SDS-PAGE was performed in 12% acrylamide gels (Criterion XT, Bio Rad Laboratories, CA). The gels where stained with Coomassie brilliant blue (Bio Rad). The apparent molecular masses of the proteins of the samples were determined by comparison with standard molecular weight markers (Rainbow, RPN756, Amersham Pharmacia Biotech).

For the antigenicity studies, an immunoblotting assay was performed. The components separated electrophoretically were transferred from the gel to a nitrocellulose membrane (Whatman® Protran®; pore size 0.45 μm) using a semidry blotting system at 0.8 mA/cm$^2$ for 30 min (Trans-Blot® SD Transfer Cell, BIO-RAD). After the blocking of protein-binding sites with a blocking solution (PBS with 5% skimmed milk, overnight, RT), the membranes were washed 4 times with PBS-T and then incubated with sera from pigs immunized with F4-GM-NPZ and y F18-GM-NPZ and diluted 1:500 (4 h at 4 °C). The membranes were washed 4 times with PBS-T and then peroxidase (PO)-conjugated secondary antibody (GAP/lgG (Fc), HRP conjugate, 1:1000) was added for 60 min at RT. Finally, membranes were extensively washed with PBS-T and the antibody-antigen complexes were visualized after addition of a substrate/chromogen solution ($H_2O_2$/cloro α-naftol).

Presence of OMVs on the surface

To study if the OMVs are encapsulated or exposed in the surface, the nanoparticles were labeled with 3,3-Dioctadecyloxacarbocyanine perchlorate (DiO). A stock suspension of OMVs in water (1 mg/mL) was prepared and sonicated. Then, 20 μL of DiO was added and followed by vortex. The samples were gently shaken at 37 °C for 20

minutes and let to recover for 10 minutes at RT. After, the labeled samples were re-suspended in water and then centrifuged at 10,000 g. Finally, the supernatants were removed and their fluorescence measured and compared.

Specific antibody response

Blood and fecal samples were collected before immunization (week 0) and weekly until 4 weeks post-immunization. Specific antibodies in serum (IgG1, IgG2a, IgA) and fecal samples (IgA) were determined by indirect ELISA. Briefly, microplate wells (Immuno-Maxisorp, Nunc®, Roskilde, Denmark) were coated with 10 μg/mL of OMVs (F4 or F18) diluted in sodium carbonate-bicarbonate buffer (0.05M, pH 9.6), and incubated overnight at 4 °C. The plates were blocked with PBS containing 3% bovine serum albumin (BSA) for 1 h at room temperature (RT). Sample feces were treated with a protease inhibitor cocktail (Invitrogen) and kept in PBS-3% milk at -20 °C until use. Serum or feces samples from mice were diluted in PBS in triplicates (1 h, RT). After five times washing with PBS Tween 20 buffer (PBS-T), the alkaline phosphatase-conjugated detection antibody, class-specific goat anti mouse IgG/IgG2a/IgA/ (Sigma), was added for 1 h at 37 °C. The detection reaction was carried out by incubating the sample with $H_2O_2$-ABTS substrate-chromogen solution for 20 min at room temperature. Absorbance was measured with an ELISA reader (Sunrise remote; Tecan-Austria, Groeding, Austria) at a wavelength of 405 nm.

Cellular immune response

In order to determine the sort of cellular immune response elicited after immunization the pattern of cytokines (IL-2, IL-4, IL-6, TNFα, IFNγ, IL-17a, IL-10 and IL-22) were determined from splenic cells. Naive and immunized mice were sacrificed by cervical dislocation at day 28 after immunization and their spleens were removed and placed in RPMI 1640 media (Gibco-BRL, UK) under sterile conditions. The spleens were smashed and cells within experimental groups were pooled in one flask. The cellular suspension was centrifuged at 380 x g for 10 min, the supernatant discarded and the pellet washed twice with PBS. The splenocytes were resuspended in lysis buffer ($NH_4Cl$ 0.15 M, $KHCO_3$ 10 mM, EDTA 14 0.1 mM) for 2 min in order to eliminate erythrocytes, and refilled with RPMI 1640 to stop the reaction. This suspension was centrifuged (380 g, 5 min) and the pellet was suspended in RPMI 1640 medium supplemented with 1 IU/mL penicillin, 1 μg/ml streptomycin and 10% fetal bovine serum (Gibco-BRL, UK). The lymphocyte suspension was added to 96-well round bottom microtitre plates (Iwaki, UK) (4 x $10^5$ cells/well) and receive one of the following different stimuli: OMVs-F4 (10 μg/mL), OMVs-F18 (10 μg/mL) and Cholera toxin (5 μg/mL) in a final volume of 200 μl per well). Negative control (PBS) and positive control (100 ng/mL + 4 μg/ml of PMA/Ionomicine used as mytogen) controls were used. The culture supernatants were collected for cytokine assay at 72 h after the stimulation and were kept frozen at -80°C. Cytokines were quantified by the kit BD Cytometric Bead Array Th1/ Th2/ Th17 CBA (BD, USA) using a flow cytometer (Acoustic Focusing Cytometer Attune®).

**Example 18.** Preparation of OMVs-loaded nanoparticles (F4-GM-NPZ & F18-GM-NPZ)

The F4-GM-NPZ (zein nanoparticles coated with a shell of Gantrez-Mannosamine conjugate and loading OMVs derived from the F4 serotype of *E. coli* i) and F18-GM-NPZ (zein nanoparticles coated with a shell of Gantrez-Mannosamine conjugate and loading OMVs derived from the F18 serotype of E. coli) nanoparticles were prepared by a solvent displacement method. Briefly, 400 mg of zein and 66.67 mg D-lysine were dissolved in 40 mL of 70% EtOH. In parallel, 15 mg of OMVs were dispersed in 40 mL of deionized water and added to the previous solution achieving the formation of nanoparticles. The suspension of nanoparticles was maintained in agitation during 5 min under magnetic stirring. After that, 1 mL of a solution of Gantrez-Mannosamine conjugate in water (10 mg/mL) was added to the suspension of zein nanoparticles and incubated for 30 minutes. Then, the suspension was purified and concentrated by ultrafiltration through a polysulfone membrane cartridge of 50 kDa pore size (Medica SPA, Italy). Finally, 13.33 mL of an aqueous solution of mannitol (200 mg mannitol per 100 mg zein) was added to the suspension of zein nanoparticles in order to prevent irreversible aggregation of nanoparticles during the drying step, and the mixture was dried in a Buchi Mini Spray Drier B-290 apparatus (Buchi Labortechnik AG, Switzerland). For this purpose, the following parameters were selected: inlet temperature of 90 °C, outlet temperature of 60 °C, spray-flow of 600 L/h, and aspirator at 100 % of the maximum capacity.

The yield of the OMVs-loaded nanoparticles (OMV-GM-NPZ) in relation to the initial amount of polymer employed was consistently high (>80%). OMV-GM-NPZ were homogenous and spherically shaped, as shown in Figure 15A.

Table 11. summarizes the main physicochemical properties. The mean diameter of F4-GM-NPZ and F18-GM-NPZ was 240 nm ± 11 and 231 ± 13 nm, respectively; empty nanoparticles showed a slightly lower size of 227 nm ± 6. The polydispersity index (PDI) showed to be lower than 0.3, indicating homogeneity in the formulations. The Z potential was -36.5 ±1.8 and -38.0 ±0.8 for F4-GM-NPZ or F18-GM-NPZ, respectively. Empty nanoparticles (GM-NPZ) presented similar Z values (-35.0 ±0.8). Free OMVs presented Z values of -7.4 ± 1.2 mV for F4, and -6.1 ± 0.9 mV for F18.

In order to study the presence of OMVs on the particles, OMVs were labeled with the lipophilic fluorescent 3,3-Dioctadecyloxacarbocyanine perchlorate (DiO) dye. Free OMVs, OMV-loaded nanoparticles and empty nanoparticles were incubated with DiO, suspended in water and centrifuged at 10.000 g. Then, the supernatants containing free OMVs were removed and particle fluorescence measured by flow cytometry. The results revealed the presence of fluorescent particles, indicating the presence of OMVs on the nanoparticles surface. 7.3% of the particles were positive for the empty formulation and 37.3% were positive for loaded particles.

Table 11. Properties of F4-GM-NPZ and F18-GM-NPZ nanoparticles

| | Mean size (nm) | PDI | Zeta Potential (mV) | OMV content (μg/mg NP) | Yield (%) |
|---|---|---|---|---|---|
| GM-NPZ | 227±6 | 0.11±0.04 | -35.08±0.79 | - | 88±2 |
| F4-GM-NPZ | 240±11 | 0.15±0.03 | -36.51±1.76 | 16.0±1.5 | 82±5 |
| F18-GM-NPZ | 231±13 | 0.13±0.03 | -37.92±0.78 | 16.0±1.5 | 81±3 |

To further confirm the OMVs encapsulation into the nanoparticles, an immunoblotting was performed using sera from immunized pigs with OMVs from *E. coli* F4 and F18 serotypes. Results confirmed that the OMVs encapsulation into nanoparticles did not alter its antigenic properties (Figure 15B).

**Example 19.** *In vivo* studies: immunogenicity in mice.

All mice were treated in accordance with institutional guidelines for treatment animals (Ethical Comity for the Animal Experimentation, of the Institution). Eight-week old BALB/c mice (20 ± 1 g) were randomized in groups of 6 animals and immunized orally. A single dose of phosphate-buffered saline (PBS), either free or encapsulated into nanoparticles OMVs (0.1 mg/mouse) from the F4 or F18 *E. coli* strains were administered. A control group of non-immunized mice (No.imm) was also included.

The evolution of the elicited serum (IgG1, IgG2a and IgA) and fecal IgA specific antibodies was determined by indirect ELISA at days 0, 7, 14, 21 and 28 post-immunization (Figure 16). High levels of lgG2a (Th1 response) and IgG1 (Th2 response) isotypes were detected. F4-GM-NPZ and F18-GM-NPZ presented a similar profile of antibodies levels along the 4 weeks of the experiment, significantly higher than those elicited by the non-encapsulated antigens. The adjuvant effect of nanoencapsulation was also significant with respect the specific IgA in serum (Figure 16A) as well as in feces (Figure 16B).

Furthermore, the antibody response was evaluated against the heat labile toxin (LT) (Figure 17). The LT specific IgG1, IgG2a, IgA antibodies levels elicited after immunization with the encapsulated antigens (F4-GM-NPZ and F18-GM-NPZ) were significantly higher than the ones who received the free OMVs.

In order to corroborate these data, levels of different cytokines (IL-2, IL-4, IL-6, TNFα, IFNγ, IL-10, IL-17a and IL-22) were determined in splenocytes of each group of mice (no immunization, free OMVs *E. coli* F4 or F18 strains, encapsulated OMVs into nanoparticles F4-GM-NPZ or F18-GM-NPZ) 28 days after immunization (Figure 18A and 18B). The encapsulation of OMVs in NPs induced an increase of levels in IL-2, IL-4 and IFN-γ respect to the administration of free antigen or non-immunized groups.

**Example 20.** *In vivo* studies: immunogenicity in pigs.

The sows were divided into 4 groups. The first group, NPI (n = 6), received orally a single dose of 50 mg of OMVs (25 mg of F4 and 25 mg of F18) + 0.2 mg of β-toxin encapsulated in zein nanoparticles coated with Gantrez-Manosamine. Five weeks post-primary immunization (ppi) the sows received a second immunization with the same amount of antigen. The second group, NPII (n=6), received a double dose (100 mg OMVs + 0.4 mg β-toxin), either entrapped in zein nanoparticles decorated with Gantrez-Manosamine. 5 weeks after the primary immunization, this group received a second immunization. The third group of sows (n = 6) received one immunization of the commercial vaccine called SUISENG. The remaining 6 pigs were used as a control and received only PBS orally. The born piglets were stabled with the corresponding sow until the weaning. Experimental procedures and animal management procedures were undertaken in accordance with institutional guidelines for treatment animals (Ethical Comity for the Animal Experimentation, of the Institution).

Sample collection

Blood and faecal samples from sows were taken from the jugular vein at week 0, 5, 7 and 8. Harvested sera were incubated at 56 °C for 30 min to inactivate complement and subsequently treated with kaolin (Sigma) to decrease

the background reading in ELISA. The colostrum samples were taken the day of birth and the blood samples of piglets were taken from the jugular vein 7 days after the birth.

Specific antibody response

Blood and fecal samples were collected before immunization (week 0), 5 and 7 weeks post-immunization. Specific antibodies in serum (IgG1, IgA) and fecal samples (IgA) were determined by indirect ELISA. Briefly, microplate wells (Immuno-Maxisorp, Nunc®, Roskilde, Denmark) were coated with 10 μg/ml of OMVs (F4 and F18) diluted in sodium carbonate-bicarbonate buffer (0.05M, pH 9.6), and incubated overnight at 4 °C. The plates were blocked with PBS containing 3% bovine serum albumin (BSA) for 1 h at room temperature (RT). Sample feces were treated with a protease inhibitor cocktail (Invitrogen) and kept in PBS-3% milk at -20 °C until use. Serum or feces samples from mice were diluted in PBS in triplicates (1 h, RT). After five times washing with PBS Tween 20 buffer (PBS-T), the alkaline phosphatase-conjugated detection antibody, class-specific goat anti mouse IgG/IgA/ (Sigma), was added for 1 h at 37 °C. The detection reaction was carried out by incubating the sample with $H_2O_2$- ABTS substrate-chromogen solution for 20 min at RT. Absorbance was measured with an ELISA reader (Sunrise remote; Tecan-Austria, Groeding, Austria) at a wavelength of 405 nm.

Evaluation of the immunogenicity acquired after oral OMVs administration

Groups of eight weeks pregnant sows were immunized orally with a new nanoparticle based vaccine (F4-F18-GM-NPZ), and compared with a commercial intramuscular vaccine (SUISENG). The nanoparticle based vaccines consisted in OMVs of *E. coli* strains F4 and F18, formulated with β-toxin of *Clostridium perfringens.* Two different doses were used of F4-18-GM-NPZ: NPI (50 mg OMV + 0.2 mg β-toxin) and NPII (100 mg OMV + 0.4 mg β-toxin). The first dose of vaccines was administered to the sows between 7 and 8 weeks gestation. The second dose was administered between 13 and 14 weeks gestation, 5 weeks after receiving the first dose. A control group of non-immunized sows was also included.

The evolution of the elicited serum (IgG, IgA and IgM) and faecal (IgG, IgA and IgM) specific antibodies against OMVs F4 or F18 (Figure 19A and 19B), or against β-toxin (Figure 20A and B), was determined by indirect ELISA 0, 5 and 7 weeks after the first immunization.

Five weeks after the first immunization, results presented high levels of IgG in serum and IgA in faeces. NPI reported lower levels comparing with NPII and SUISENG. Two weeks after the second immunization, the higher specific antibody levels were in the NPII vaccinated group. NPI and SUISENG groups showed lower and similar levels between them.

Analyzing the response in mucosa, SUISENG reported much higher levels of IgA 5 weeks after receiving the first dose. However, two weeks after the second immunization, the IgA levels decreased in the commercial vaccine while the encapsulated vaccines reached higher levels than this one.

Three weeks after receiving the second immunization, IgG and IgA antibodies were also studied in colostrum collected the day of birth. The analysis of the IgG and IgA titre in colostrum (Figure 21A and 21B) showed also the adjuvant effect of nanoencapsulation. NPII vaccine reached higher levels of IgG and IgA than the commercial vaccine and NPI. This level of antibodies is correlated with the passive immunization reported in piglets. IgG and IgA levels were studied in serum of weaned piglets one week after birth (Figure 22A and 22B). The piglets whose mothers were vaccinated with NPII reported higher levels of antibodies comparing with the SUISENG group and much higher levels comparing with NPI.

**Nanoparticles for oral administration of insulin**

A new insulin delivery system for diabetes symptomatology based on the nanoparticles of the invention was evaluated. More particularly, the insulin delivery system is capable of controlling the glycaemia in an animal model of diabetes type 1. The nanoparticles of the invention allow the fulfilment of at least the following requirements:

- Accommodate an important payload of protein;
- Offer protection to the loaded protein against their degradation;
- Facility to decorate the surface with a shell capable to cross the mucus layer within the mucosa;
- Controlled release properties of the loaded proteins.

Insulin analysis

In order to quantify the insulin amount loaded into zein nanoparticles coated with a shell of Gantrez-Thiamine conjugate (I-GT-NPZ), supernatants from the formulations and liquids from washing procedures of already dried formulations were analysed by a HPLC method. The apparatus was an Agilent model 1100 series LC and a diode-array detector set at 220 nm. The chromatographic system was equipped with a TSKgel4000 7,8 x 30cm TosoHaas column. The mobile phase was a 0.3 M NaCl solution in 0.05 M phosphate buffer. The flow-rate was 0.8 mL/min. The column was placed at 27°C and the injection volume was 40 μL. Standard curves were designed over the range of 2-100 μg/mL ($R^2 \geq 0.999$) from a human insulin solution and were prepared in supernatant of non-loaded nanoparticles.

To analyse the samples, 1.5 mL of supernatants were diluted with 6 mL of mobile phase and filtered through a 0.45 μm regenerated cellulose membrane in order to eliminate the zein before the HPLC analysis. For the evaluation of free insulin associated to the nanoparticles, 5 mg of dried formulations were re-suspended in 1 mL of water and filtered through a 0.22 μm pore size PTFE membrane. Samples were transferred to auto-sampler vials, capped and placed in the HPLC auto-sampler. The results were expressed as the amount of insulin (μg) per mg of nanoparticles.

Pharmacokinetic and pharmacodynamic studies

The determined pharmacodynamic parameters include the hypoglycaemic effect quantified by the area above the curve ($AAC_{0\text{-}24h}$) by linear trapezoidal method. Pharmacological availability (PA) calculated as the cumulative hypoglycaemic effect relative to 100% PA of sc free insulin, the time of minimum glycaemia ($T_{max}$) and the minimum glucose concentration in the blood ($C_{min}$).

Pharmacokinetic parameters include bioavailability of oral insulin calculated by the dose-corrected area under the curve of serum insulin levels overtime ($AUC_{0\text{-}8h}$) relative to 100% bioavailability of sc free-form insulin calculated by linear trapezoidal method, the maximum concentration of insulin in serum ($C_{max}$) and time that insulin is at the maximum concentration ($T_{max}$). The pharmacokinetic parameters were obtained using WinNonline© software.

The relative oral bioavailability based on either blood insulin level (F%) or glucose levels (PA%) were calculated using the following equations:

$$PA\% = \frac{AAC_{oral} \times Dose_{s.c.}}{AAC_{s.c.} \times Dose_{oral}} \times 100 \quad \text{Equation 1}$$

$$F\% = \frac{AUC_{oral} \times Dose_{s.c.}}{AUC_{s.c.} \times Dose_{oral}} \times 100 \quad \text{Equation 2}$$

In which, AUC is the total area under the curve of the serum insulin concentration versus time profile and AAC is the total area above the glycaemia curve. Both were calculated by the trapezoidal rule.

**Example 21.** Zein nanoparticles coated with the Gantrez-Thiamine conjugate and loading insulin (I-GT-NPZ).

First, 200 mg zein, 30 mg D-lysine and 20 mg of recombinant human insulin (I) were dissolved and incubated for 1 hour, under magnetic stirring at RT, in 20 mL of ethanol 55%. Nanoparticles were then produced by desolvation after the addition of 20 mL of water. Once the nanoparticles were obtained, 0.25, 0.5 or 1 mL of hydrolyzed conjugate (obtained by dissolving 200 mg conjugate in 10 mL water) were added and the mixture was maintained under agitation at RT for 30 minutes. The suspension was concentrated and purified and then dried by spray-dryer (Mini-Spray Dryer; Büchi®, Switzerland) with 400 mg of mannitol as protector. The parameters selected for this purpose were: inlet temperature of 90 °C, outlet temperature of 60 °C, spray-flow of 600 L/h, and aspirator at 90 % of the maximum capacity. As controls, insulin-loaded zein nanoparticles (I-NPZ) were prepared in the same way as described above but in the absence of GT.

The main physico chemical characteristics are summarized in Table 12.

Table 12. Physico-chemical characterization of the different nanoparticles. Data expressed as mean ± SD (n=3). Insulin loaded-"naked" zein nanoparticles 0.1 % w/w and zein nanoparticles coated with different quantities of Gantrez-Thiamine (GT) conjugate: 2.5% w/w (I-GT-NPZ1), 5% w/w (I-GT-NPZ2), and 10% w/w (I-GT-NPZ3) of conjugate vs. zein.

| FORMULATION | SIZE (nm) | PDI | Zeta Potential (mV) | Insulin Loading (μg/mg NP) | EE% |
|---|---|---|---|---|---|
| I-NPZ | 220±10 | 0.127±0.005 | -43 ± 5 | 85 ± 2 | 85 ± 2 |
| I-GT-NPZ1 | 257±13 | 0.088±0.030 | -38 ± 1 | 79 ± 1 | 79 ± 1 |
| I-GT-NPZ2 | 271±5 | 0.161±0.006 | -38 ± 1 | 77 ± 2 | 77 ± 2 |
| I-GT-NPZ3 | 321±15 | 0.157±0.020 | -39 ± 7 | 80 ± 3 | 80 ± 3 |

In first place, uncoated nanoparticles containing insulin (I-NPZ) presented the smallest size, 220 nm. However, nanoparticles coated with the Gantrez-Thiamine conjugate presented bigger sizes and the size increased when higher amounts of conjugate were incorporated in the formulation, 257 nm when 2.5% w/w of conjugate was incorporated to the formulation (I-GT-NPZ1), 271 nm in the case of a 5 % of conjugate (I-GT-NPZ2), and 321 nm in the case of 10% of conjugate in the formulation (I-GT-NPZ3). Regarding the zeta potential, the nanoparticles coated with the Gantrez-Thiamine conjugate presented a negative zeta potential of around -38 mV. However, "naked" core-only zein nanoparticles presented a slightly more negative surface charge around -43 mV. The insulin loading in all formulations was around 80 μg/mg NP which means an encapsulation efficiency of 80 % since the initial amount of insulin employed for the preparation of the nanoparticles was 10 mg per 100 mg of zein.
TEM microphotographs (Figure 23) revealed that all formulations consisted of homogeneous and spherical populations and of a similar size similar to that obtained by photon correlation spectroscopy.

**Example 22.** *In vitro* release studies

The insulin release kinetics from zein nanoparticles were evaluated in two different media: simulated gastric (SGF) and intestinal fluids (SIF). For these purpose Float-A-Lyzer devices with a MWCO of 300 kDa (Spectrum Labs.com) were used. After washing the dialysis bags with ethanol 10% for 10 min and then with water, the system was filled with 5 mL of nanoparticles dispersed in water and then, placed into a vessel containing 45 mg mL SGF. The vessel was maintained under magnetic agitation and 100 μL samples were withdrawn at fixed time intervals and replaced with equal volumes of SGF. After two hours of incubation in this gastric fluid, the system was placed into a second vessel with 45 mL SIF. Again, at fixed times, 100 μL were withdrawn and replaced with free SIF.

At different time points, samples were collected and the insulin in SFG and SIF were quantified by the HPLC method described above. Calibration curves in the simulated mediums (2-100 μg/mL; $R^2 \geq 0.999$ in both cases) were generated.

Figure 24 represents the release profiles of insuline from the different assayed formulations as cumulative percentage of drug release as a function of time. In SGF a 50 % of insulin was released from "naked" zein nanoparticles (I-NPZ) in the first 2 hours. However, around a 30 % of insulin was released in the first two hours from zein nanoparticles coated with Gantrez-Thiamine conjugate (I-GT-NPZ). In contrast, when nanoparticles were dispersed in SIF exhibited a sustained release up to 24 h, when a 75 % of the cargo was released from I-NPZ and around a 60 % of the payload was released from I-GT-NPZ formulations. As can be seen in Table 13, the kinetics from the release of the nanoparticles containing insulin follows a first order kinetic, in addition, according to Korsmeyer-Peppas, the insulin is delivered from uncoated nanoparticles following a diffusion mechanism. However, when zein nanoparticles are coated with Gantrez-Thiamine conjugate, an erosion mechanism governs the release and this influence of an erosion mechanism is higher when the percentage of conjugate coating the surface of the nanoparticles is increased.

Table 13. Analysis of the insulin release mechanism from "naked" zein nanoparticles (I-NPZ) and zein nanoparticles coated with a shell of Gantrez-Thiamine (GT) conjugate at a GT/zein ratio (w/w) of 2.5% (I-GT-NPZ1), 5% (I-GT-NPZ2) and 10% (I-GT-NPZ3)

| FORMULATION | Korsmeyer-Peppas | | | First Order | |
|---|---|---|---|---|---|
| | $k_{KP}(h^n)$ | n | $R^2$ | $K_1(h^1)$ | $R^2$ |
| I-NPZ (0-26 h) | 0,342 | 0,277 | 0,889 | 0,222 | 0,689 |
| I-NPZ (0-2 h pH 1.2) | 0,282 | 0,722 | 0,999 | 0,327 | 0,990 |

(continued)

| FORMULATION | Korsmeyer-Peppas | | | First Order | |
|---|---|---|---|---|---|
| | $k_{KP}(h^n)$ | n | $R^2$ | $K_1(h^1)$ | $R^2$ |
| I-NPZ (2-26 h pH 6.8) | 0,066 | 0,471 | 0,986 | 0,017 | 0,742 |
| I-GT-NPZ1 (0-26 h) | 0,192 | 0,356 | 0,946 | 0,074 | 0,468 |
| I-GT-NPZ1 (0-2 h pH 1.2) | 0,157 | 0,791 | 0,993 | 0,165 | 0,988 |
| I-GT-NPZ1 (2-26 h pH 6.8) | 0,047 | 0,598 | 0,997 | 0,017 | 0,912 |
| I-GT-NPZ2 (0-26 h) | 0,169 | 0,380 | 0,956 | 0,061 | 0,504 |
| I-GT-NPZ2 (0-2 h pH 1.2) | 0,143 | 0,815 | 0,982 | 0,151 | 0,983 |
| I-GT-NPZ2 (2-26 h pH 6.8) | 0,034 | 0,706 | 0,998 | 0,017 | 0,971 |
| I-GT-NPZ3 (0-26 h) | 0,207 | 0,360 | 0,940 | 0,090 | 0,566 |
| I-GT-NPZ3 (0-2 h pH 1.2) | 0,177 | 0,848 | 0,976 | 0,195 | 0,985 |
| I-GT-NPZ3 (2-26 h pH 6.8) | 0,026 | 0,814 | 0,996 | 0,018 | 0,993 |

**Example 23.** *In vivo* studies

Male Wistar rats weighing 180 g were housed under controlled temperature, humidity, and a 12-12h light-dark cycle. Diabetes was induced with intraperitoneal injection of 80 mg/kg streptozotocin (Sigma-Aldrich, Madrid, Spain) in citrate buffer 0.1M (pH 4.5). During 24h after STZ injection, rats were given 5% glucose physiologic solution to prevent hypoglycaemia due to pancreatic damage. After 8-10 days, rats with frequent urination, loss of weight and fasting plasma glucose levels higher than 250 mg/dL were randomized for *in vivo* studies. The diabetic rats were fasted for 12 h before experiments and, then divided in three groups. The first group of animals received a single subcutaneous dose of an aqueous solution of insulin in hepes buffer (5 U/kg). In the second group, animals received by oral gavage 50 U/kg as single dose of insulin-loaded nanoparticles (I-GT-NPZ2) dispersed in 1 mL water. Finally, the third group of animals received orally 50 U/kg of insulin formulated as aqueous solution (1 mL).

Blood samples were collected from the tail vein of the rats prior insulin administration in order to establish baseline glucose levels, and at different times after the administration of the different formulations. Glucose levels were measured with a glucometer (Accu-Check® Aviva glucometer; Roche Diagnostics, Basel, Switzerland). In a similar way, insulin levels were quantified from the sera of animals. Insulin was quantified using an ELISA (EIA insulin kit, Arbor Assays, Ann Arbor, USA).

Changes in the plasma glucose are shown in Figure 25 whereas Table 14 summarises the pharmacodynamic parameters.

As can be seen from Figure 25, the oral administration (p.o.) of insulin encapsulated in the nanoparticles of the invention generated a sustained decrease of glucose when compared to the subcutaneous (s.c.) administration of insulin which showed a quick decrease in the blood glucose levels within the first hour post-administration followed by a fast increase up to the 8 hours post-administration. The sustained decrease in the blood glucose levels achieved by the nanoparticles of the invention containing insulin was from 4 to 18 hours with a minimum of 32% at 10 hours. When free insulin was administered orally, no hypoglycaemic effect was detected.

As observed in Table 14, subcutaneously administered insulin was the most effective formulation to decrease the blood glucose levels down to 20.6 % respect to the basal glucose at $T_{max}$ of 2 h. It is important to note that the $C_{min}$ values of insulin encapsulated in nanoparticles and insulin orally administered were very similar to the reference formulation, but the value of encapsulated insulin was achieved at a $T_{max}$ of 10 h. From the curves in Figure 25 the areas above the curves (AACs) of the three different ways of administration of the insulin were obtained. The AACs were 642.41m, 794.62 m and 0.00 m for subcutaneous insulin, oral insulin administered encapsulated in nanoparticles and the oral solution of insulin, respectively. This would indicate that the bioavailability based on the hypoglycaemic effect respect to the subcutaneously administered insulin were 12.4% and 0.0% in the case of insulin administered in nanoparticles per oral route and insulin administered in solution also per oral route.

Table 14. Pharmacodynamic parameters after oral (p.o) delivery of 50 U/kg insulin formulated as nanoparticles (I-GT-NPZ) and insulin free-form, and subcutaneous (s.c.) injection of 5 U/kg insulin free-form.

| | Dose (UI/kg) | AAC (μU/hmL) | $T_{max}$ (h) | $C_{min}$ (% basal glucose) | PA (%) |
|---|---|---|---|---|---|
| s.c. insulin | 5 | 642.41 | 2 | 20.6 | 100.0 |
| p.o. insulin | 50 | 0.00 | 2 | 87.5 | 0.0 |
| p.o. I-GT-NPZ I | 50 | 794.62 | 10 | 32.2 | 12.4 |

The oral relative bioavailability was calculated on the basis of quantification of insulin levels with subcutaneous insuline as a reference. Figure 26 shows the results of blood insulin level after the oral administration of zein nanoparticles coated with Gantrez-Thiamine conjugate containing insulin and insulin administered per subcutaneous route. The insulin serum concentration increased rapidly up to 1 hour and decreased dramatically until not being detectable 6 hours when insulin was subcutaneously administered. The data were adjusted by non-compartmental model. The pharmacokinetic parameters calculated are presented in Table 15.

Table 15. Pharmacokinetic parameters of insulin. I-GT-NPZ: insulin-loaded zein nanoparticles coated with a shell of Gantrez-Thiamine conjugate (50 UI/kg) and, insulin free-form by oral route (p.o.) (50 UI/kg), and insulin free-form by subcutaneous injection (s.c.) (5 UI/kg).

| | Dose (UI/kg) | $C_{max}$ (μU/mL) | $T_{max}$ (h) | $t_{1/2}$ (h) | MRT (h) | Cl/F (mL/h) | K ($h^{-1}$) | AUC (μUh/mL) | F% |
|---|---|---|---|---|---|---|---|---|---|
| s.c. insulin | 5 | 354 | 1 | 0.70 | 5.08 | 976 | 0.979 | 550.5 | 100 |
| p.o. insulin | 50 | N.D. | N.D. | N.D. | N.D. | N.D. | N.D. | N.D. | - |
| p.o. I-GT-NPZI | 50 | 117 | 4 | 1.53 | 8.19 | 982 | 0.454 | 365.0 | 6.6 |

When insulin was administered subcutaneously, the peak plasma concentration ($C_{max}$) was around 354 μU/mL and the $T_{max}$ was achieved at 1h with a half-life ($t_{1/2}$) of 0.7h. The insulin clearance/F was calculated to be 975.95 μU/hmL (Table 15). For insulin formulated in nanoparticles orally administered the $C_{max}$ was around 117 μU/mL (Figure 26, Table 15).

Surprisingly, the half-life was 1.5h, double than when insulin is subcutaneously administered and the elimination constant was 0.4544 $h^{-1}$ exactly half of the constant value of insulin subcutaneously administered. This suggests that the elimination of insulin from the organism was governed by the absorption process, as seen in the slopes of the curve in Figure 26. Finally, the relative bioavailability of insulin when it was orally administered encapsulated in nanoparticles was 6.6% respect to the subcutaneous route and the relative bioavailability based on the hypoglycemic activity was 12.4%.

## Claims

1. A core-shell nanoparticle, wherein:

   the core is solid and comprises a zein matrix and at least one basic amino acid; and
   the shell comprises a conjugate obtained by reaction of a poly (methyl vinyl ether-co-maleic anhydride) copolymer with a compound having a reactive primary amine group.

2. The nanoparticle according to claim 1, wherein the basic amino acid is selected from the group formed by arginine, lysine, histidine and mixtures thereof.

3. The nanoparticle according to any one of claims 1 or 2, wherein the compound having a reactive primary amine

group is selected from mannosamine and thiamine.

**4.** The nanoparticle according to any one of claims 1 to 3, further comprising a biologically active compound.

**5.** The nanoparticle according to claim 4, wherein the biologically active compound is selected from allergens, antigens, immunomodulating agents and compounds capable of controlling the glycaemia.

**6.** The nanoparticle according to anyone of claims 4 or 5, wherein the compound having a reactive primary amine group is mannosamine and the biologically active compound is selected from allergens, antigens, immunomodulating agents or combinations therefrom.

**7.** The nanoparticle according to anyone of claims 4 or 5, wherein the compound having a reactive primary amine group is thiamine and the biologically active compound is a compound capable of controlling the glycaemia.

**8.** A process for producing a core-shell nanoparticle as defined in anyone of claims 1 to 7, said process comprises the steps of:

a. preparing a hydroalcoholic solution comprising a zein and at least one basic amino acid;
b. adding water to the solution obtained in step (a).
c. reacting a poly (methyl vinyl ether-co-maleic anhydride) copolymer with a compound having a reactive primary amine group in an organic solvent to form a conjugate; and
d. adding the conjugate obtainable in step (c) to the solution of step (b).

**9.** The process according to claim 8, wherein the solution of step (a) further comprises a biologically active compound.

**10.** A core-shell nanoparticle, obtainable by the process according to any one of claims 8 or 9.

**11.** A composition comprising at least one core-shell nanoparticle according to any one of claims 1 to 7 or claim 10, and a food, nutraceutical, cosmeceutical, cosmetic or pharmaceutically acceptable vehicle or carrier.

**12.** A core-shell nanoparticle as defined in anyone of claims 1 to 7 or claim 10 or a pharmaceutical composition as defined in claim 11 for use in medicine.

**13.** A vaccine or an immunotherapeutic composition comprising at least one core-shell nanoparticle, wherein:

the core is solid and comprises a zein matrix, at least one basic amino acid, and an allergen or an antigen and/or an immunomodulating agent; and
the shell comprises a conjugate obtained by reaction of a poly (methyl vinyl ether-co-maleic anhydride) copolymer with a compound having a reactive primary amine group.

**14.** A core-shell nanoparticle as defined in claim 4, wherein the biologically active compound is selected from the group consisting of allergens, antigens, immunomodulating agents and combinations therefrom, or as defined in claim 6, or a vaccine or immunotherapeutic composition as defined in claim 13 for use in immunotherapy.

**15.** A pharmaceutical composition comprising at least one core-shell nanoparticle, wherein:

the core is solid and comprises a zein matrix, at least one basic amino acid and a compound capable of controlling the glycaemia; and
the shell comprises a conjugate obtained by reaction of a poly (methyl vinyl ether-co-maleic anhydride) copolymer with a compound having a reactive primary amine group; and
a pharmaceutically acceptable carrier or excipient.

**16.** A core-shell nanoparticle as defined in anyone of claims 4, 5 or 7 or a pharmaceutical composition as defined in claim 15 for use in the prevention or treatment of diabetes.

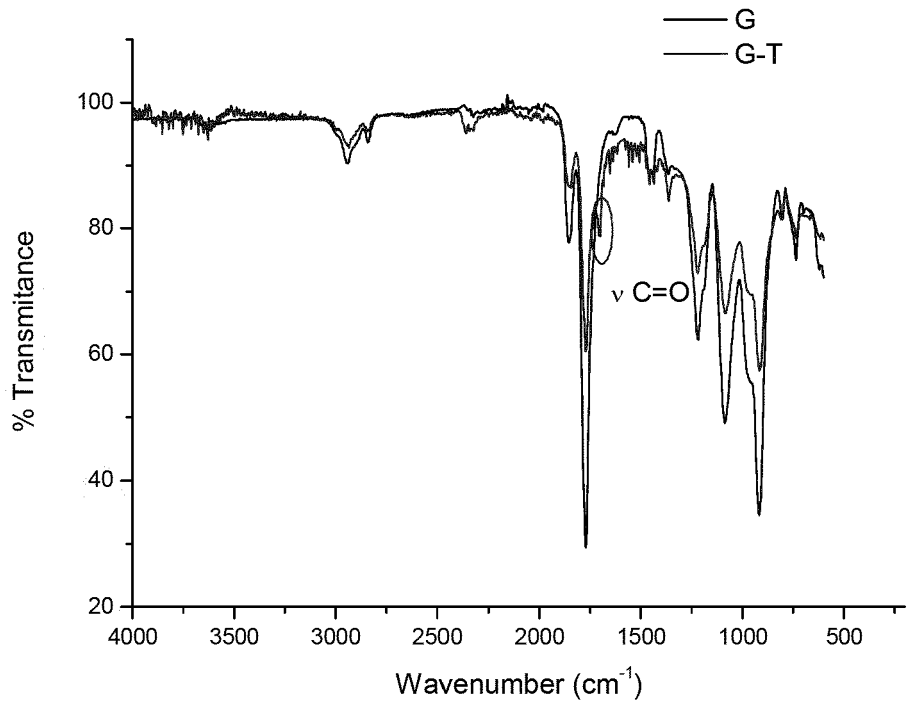


**Figure 1**

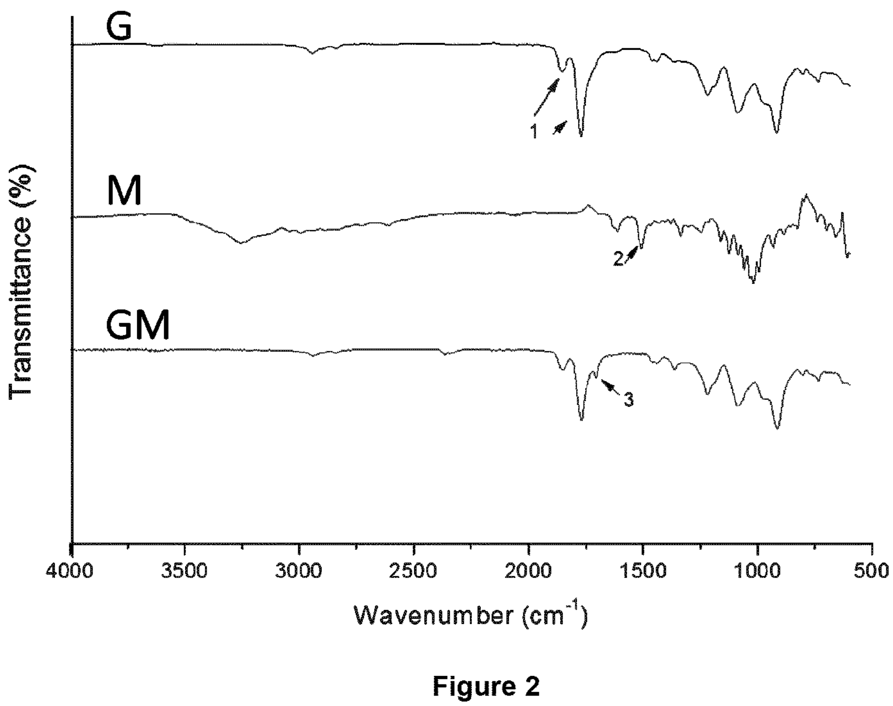


**Figure 2**

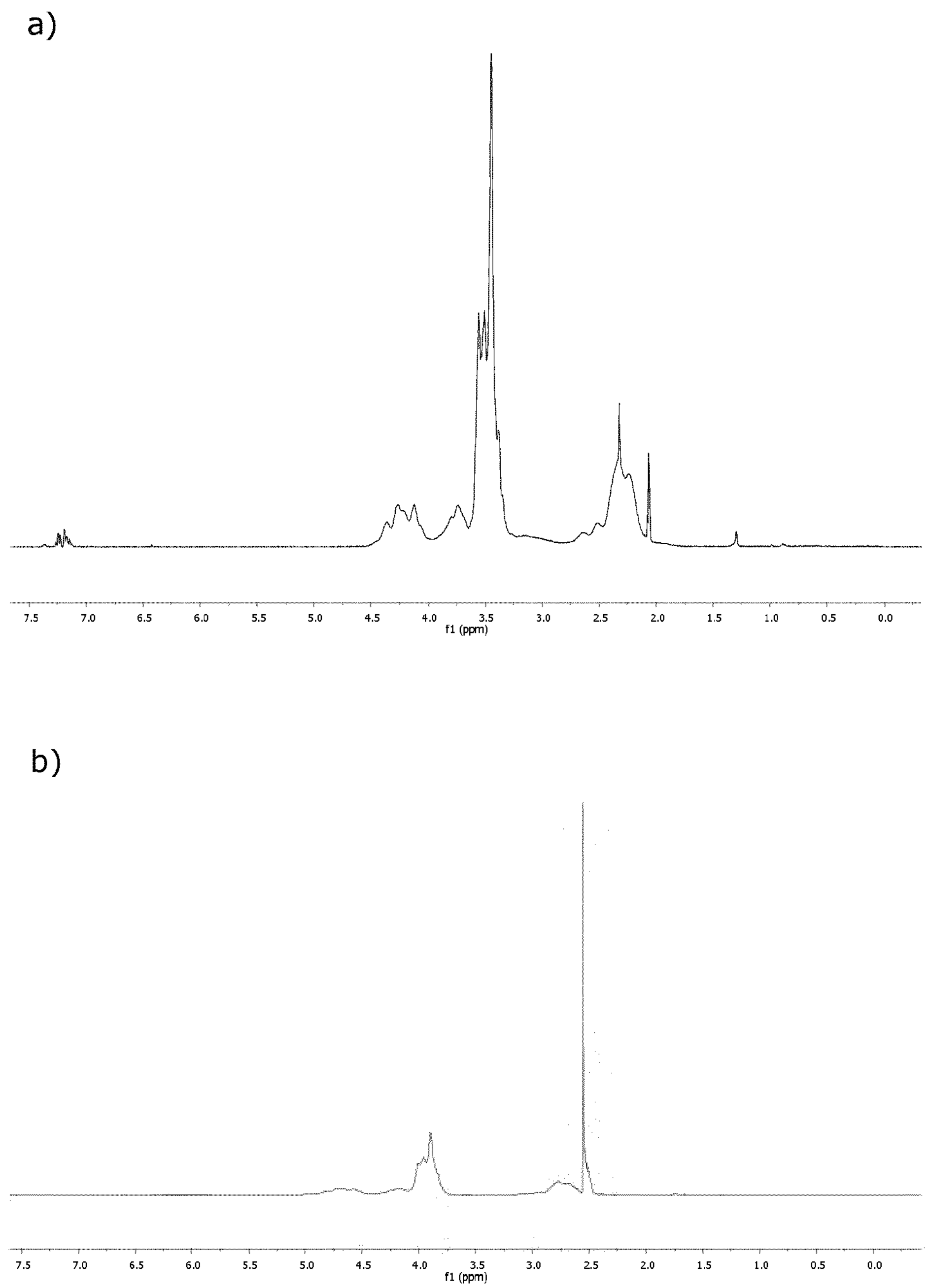
a)
7.5
7.0
6.5
6.0
5.5
5.0
4.5
4.0
3.5
3.0
2.5
2.0
1.5
1.0
0.5
0.0
f1 (ppm)
b)
7.5
7.0
6.5
6.0
5.5
5.0
4.5
4.0
3.5
3.0
2.5
2.0
1.5
1.0
0.5
0.0
f1 (ppm)

**Figure 3**

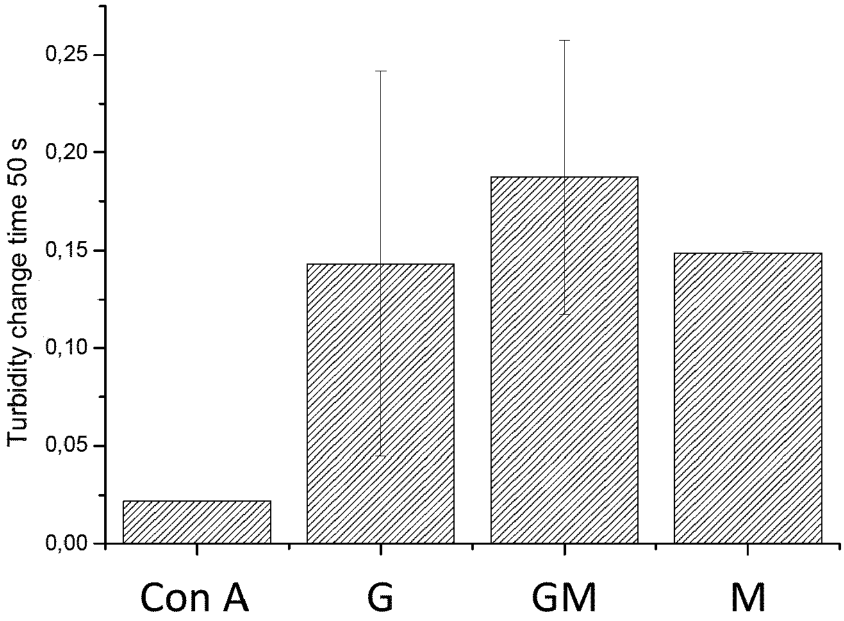


Figure 4

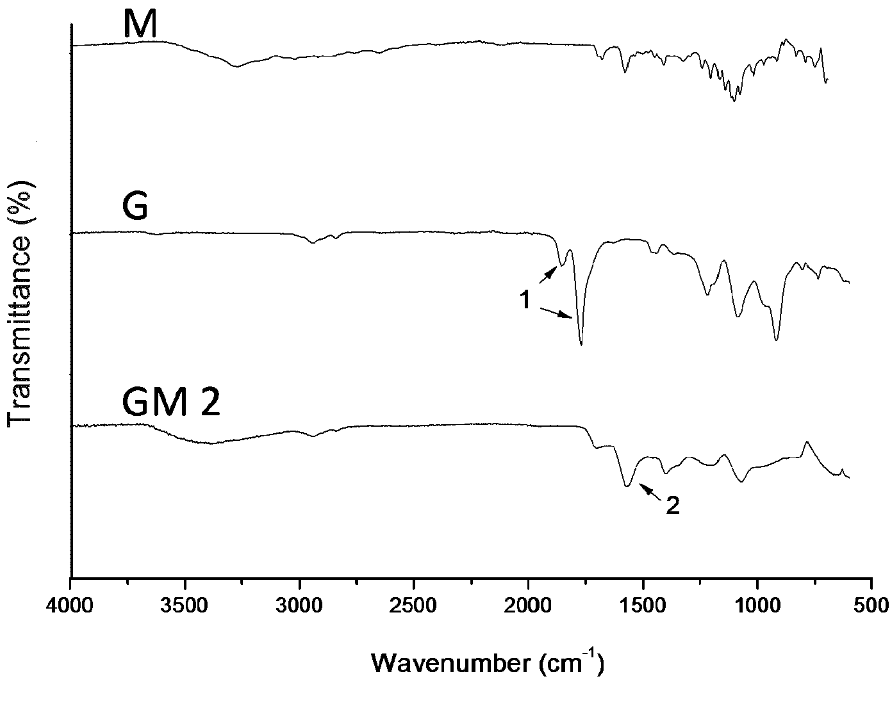


Figure 5

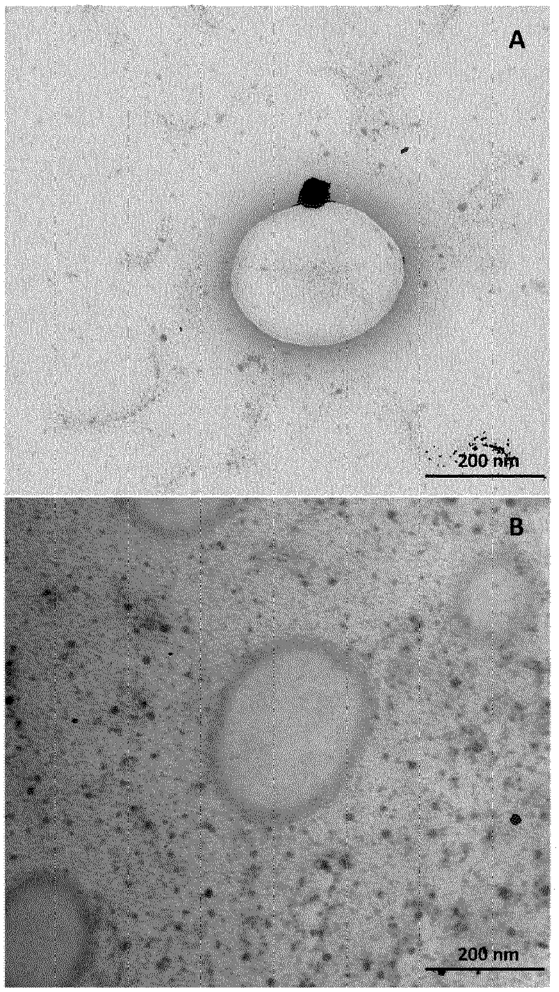


Figure 6

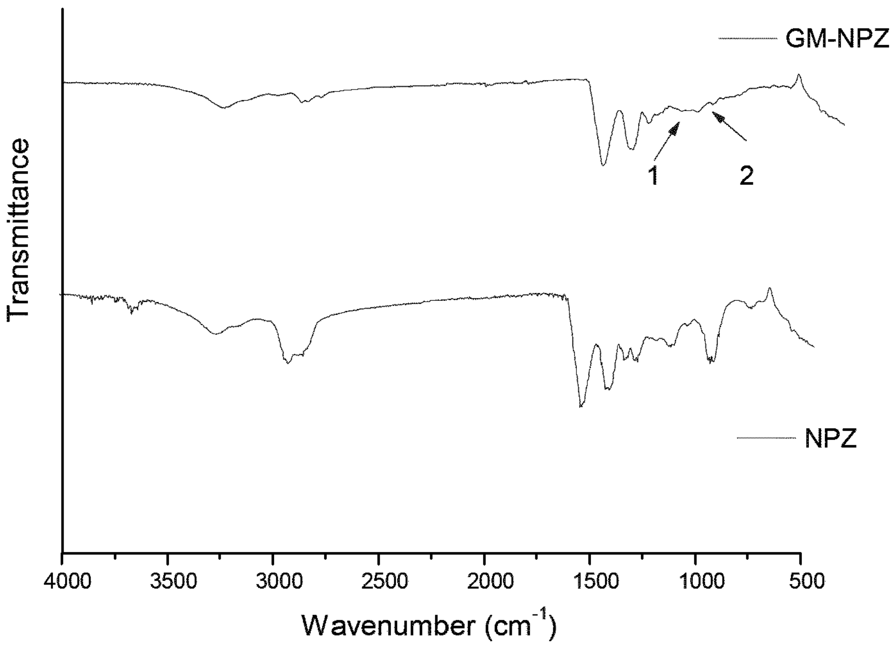


Figure 7

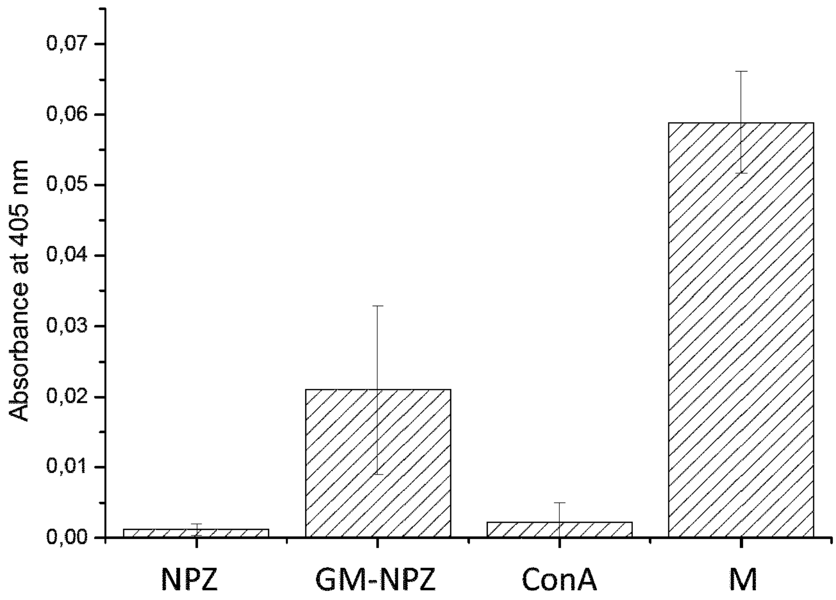


Figure 8

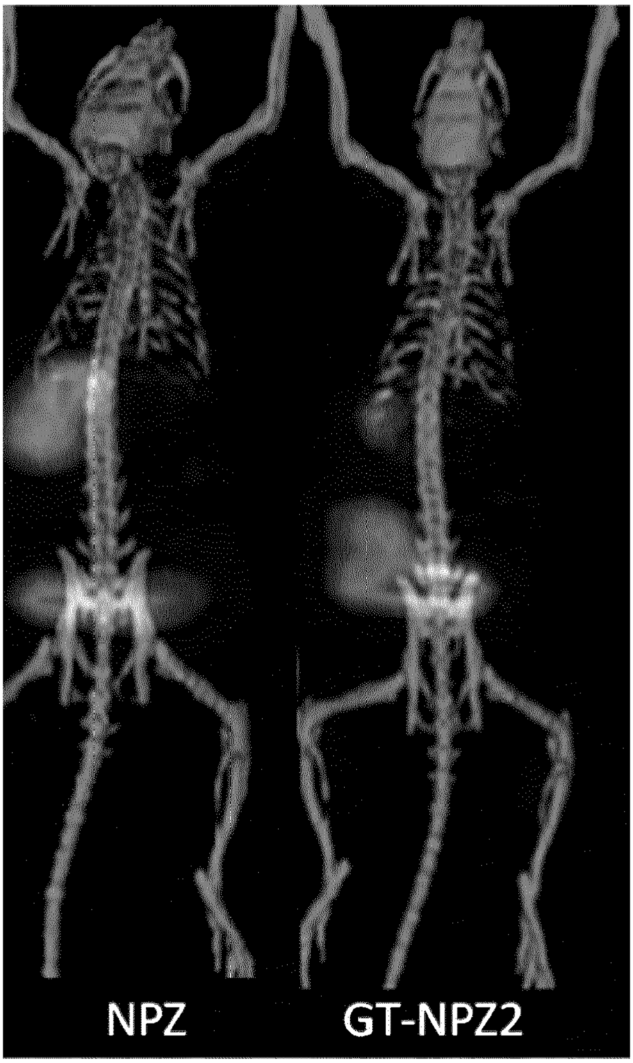


Figure 9

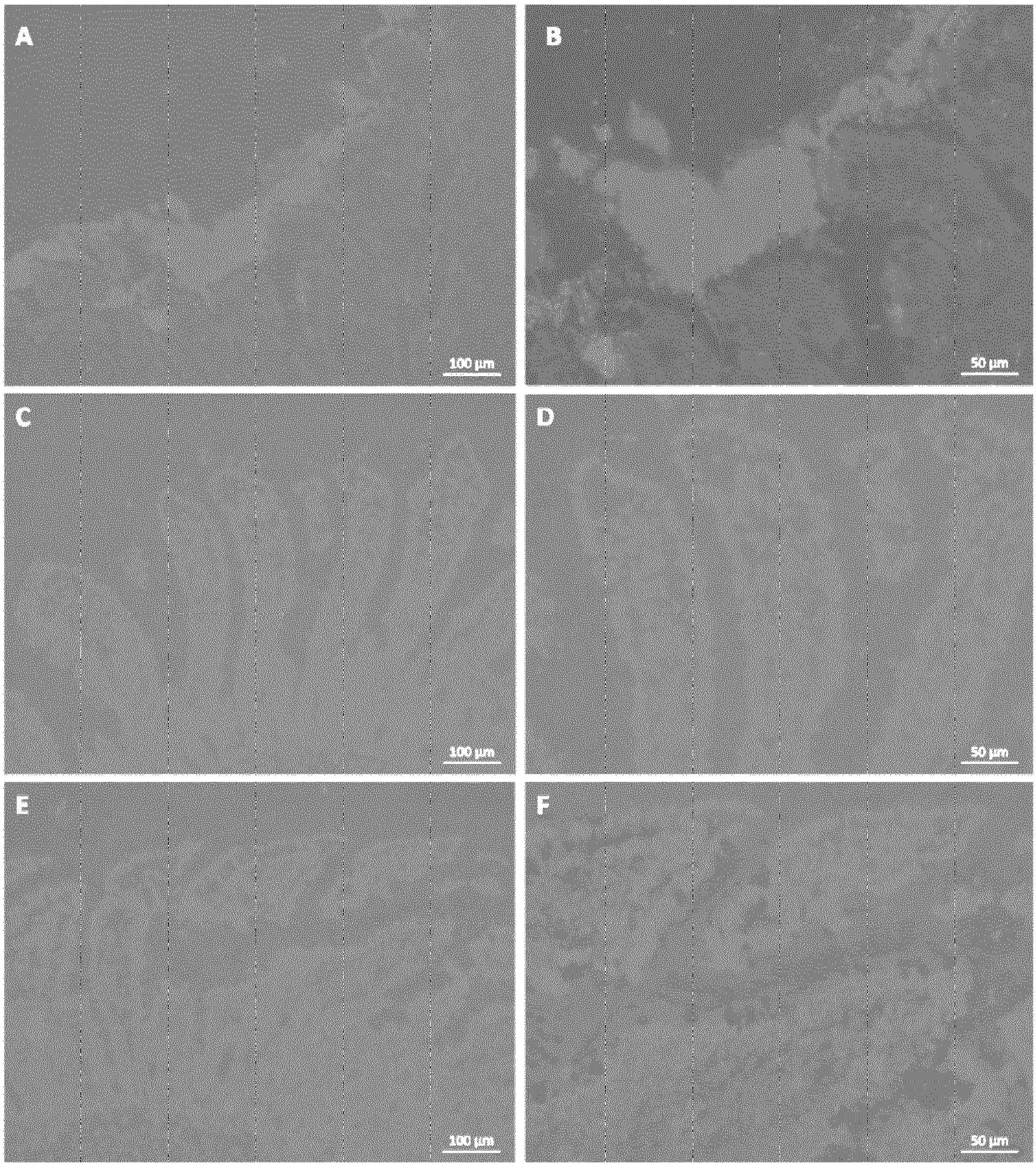


Figure 10

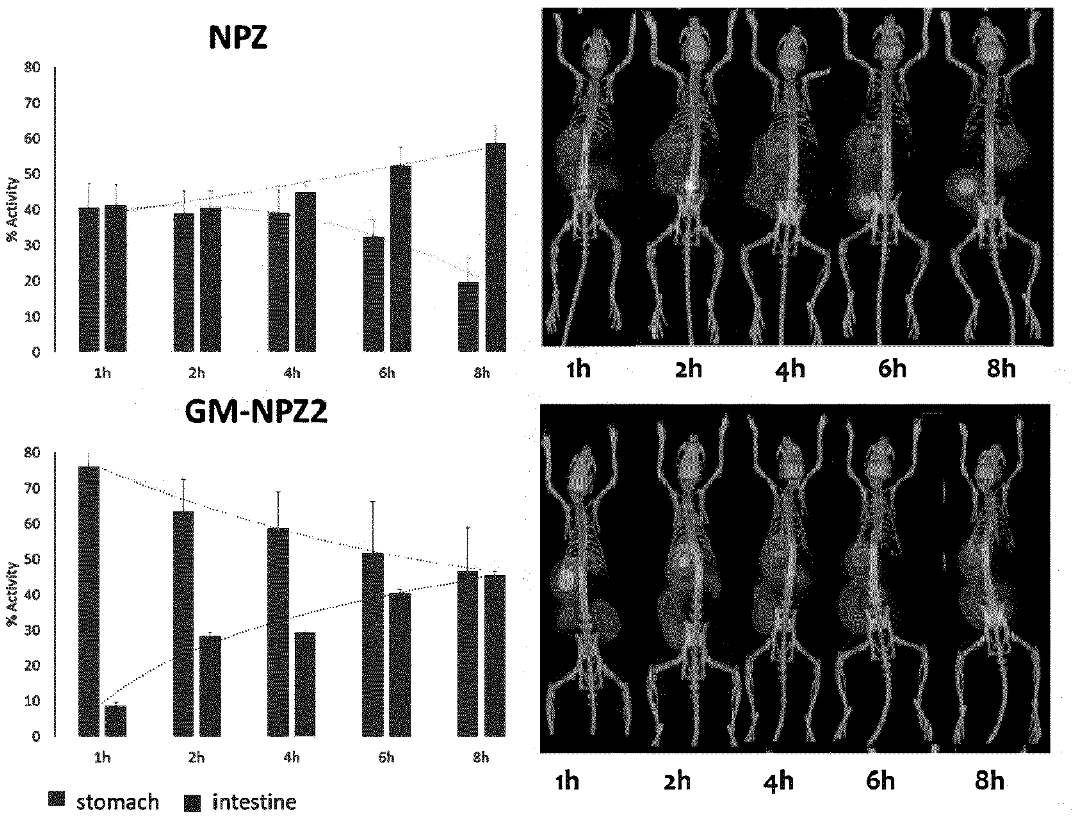

NPZ
% Activity
80
70
60
50
40
30
20
10
0
1h
2h
4h
6h
8h
GM-NPZ2
stomach
intestine


Figure 11

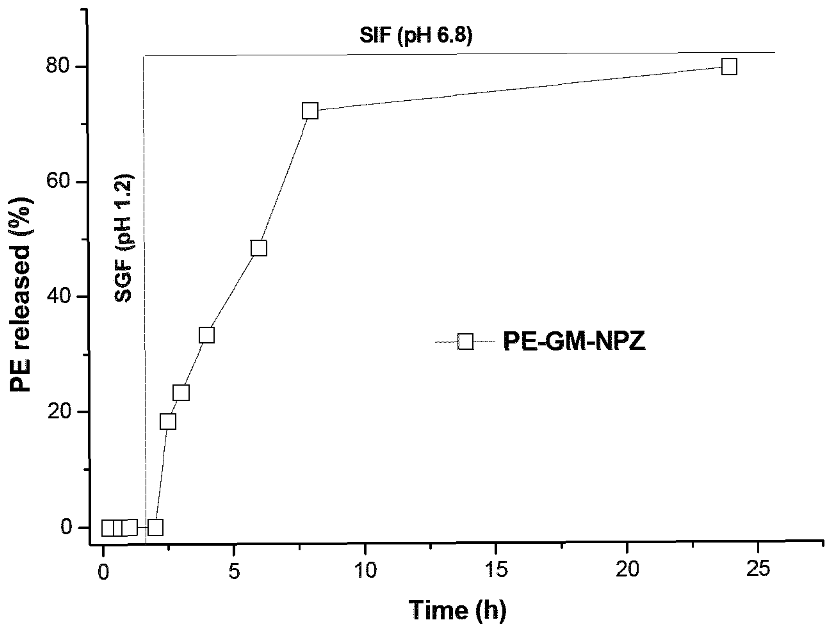


Figure 12

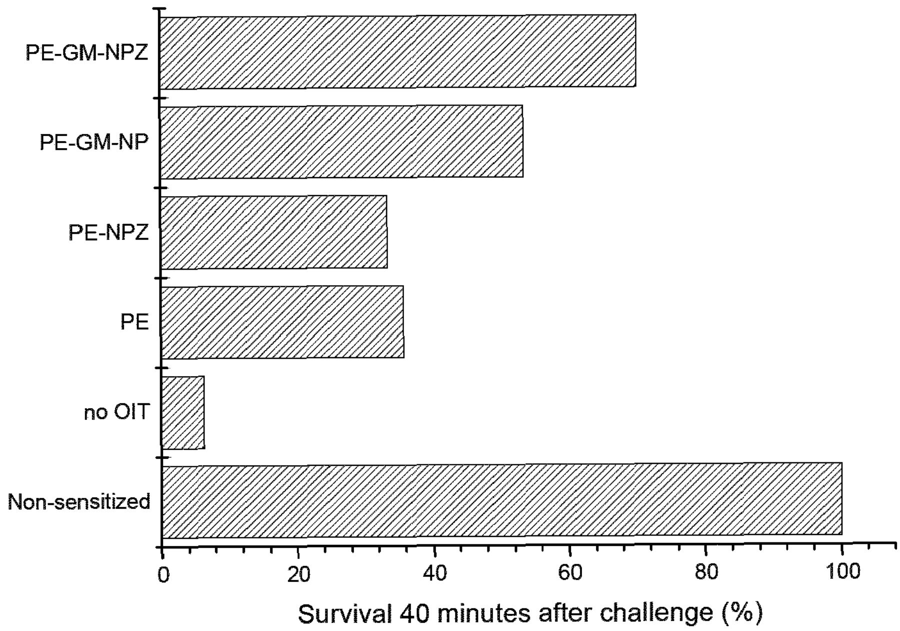


Figure 13

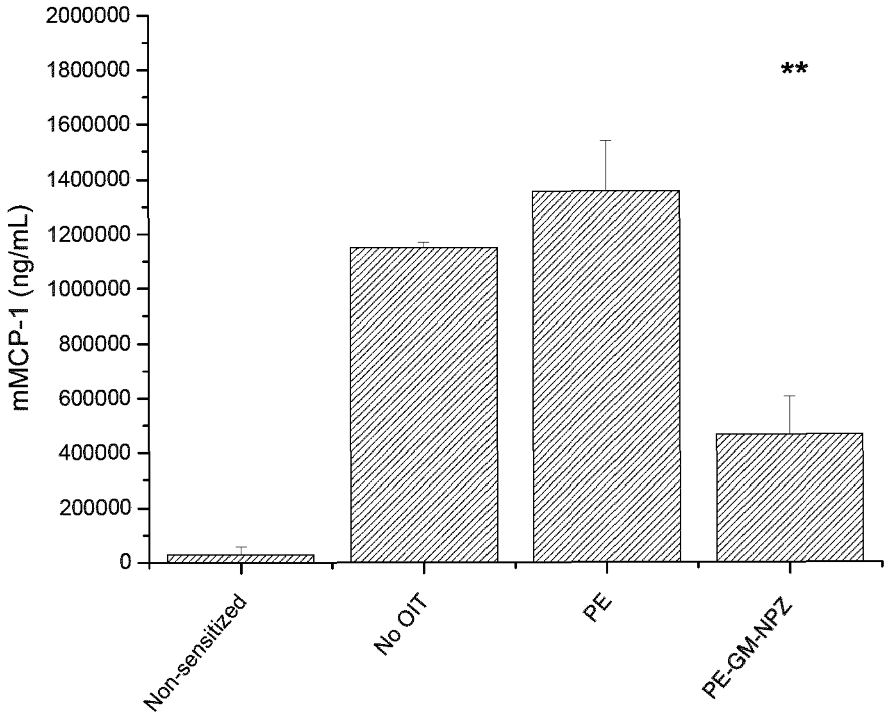


Figure 14

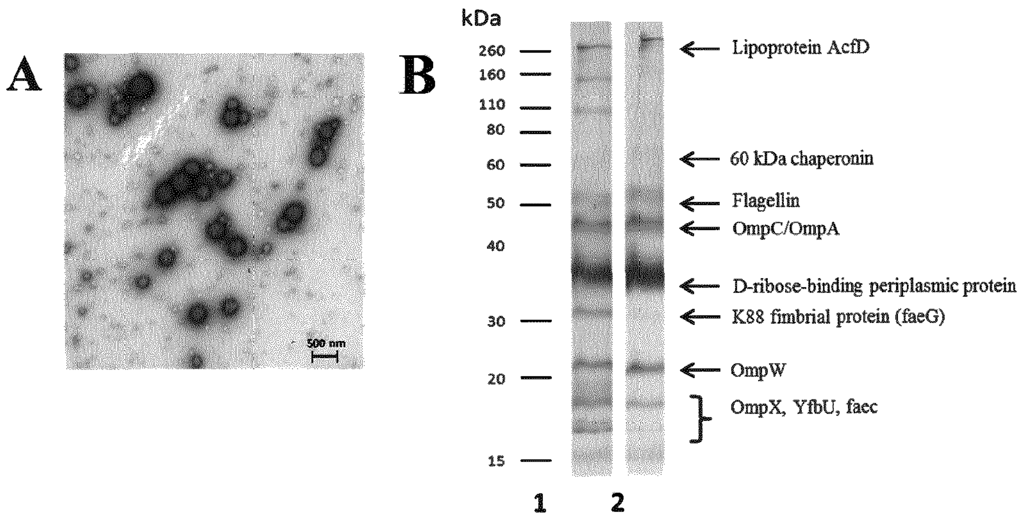


Figure 15

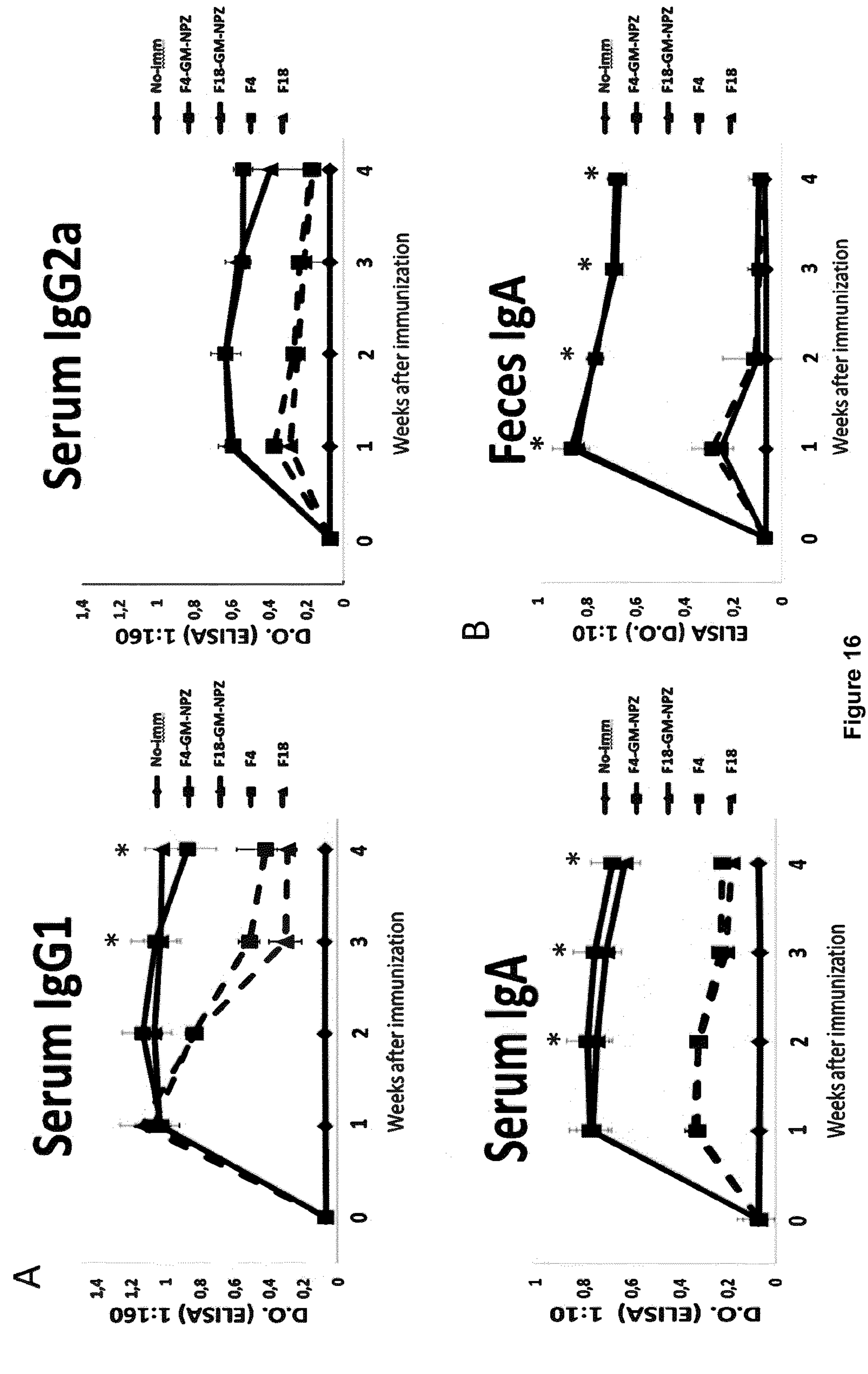
A
Serum IgG1
D.O. (ELISA) 1:160
Weeks after immunization
Serum IgG2a
D.O. (ELISA) 1:160
Weeks after immunization
Serum IgA
D.O. (ELISA) 1:10
Weeks after immunization
B
Feces IgA
ELISA (D.O.) 1:10
Weeks after immunization
No-imm
F4-GM-NPZ
F18-GM-NPZ
F4
F18

Figure 16

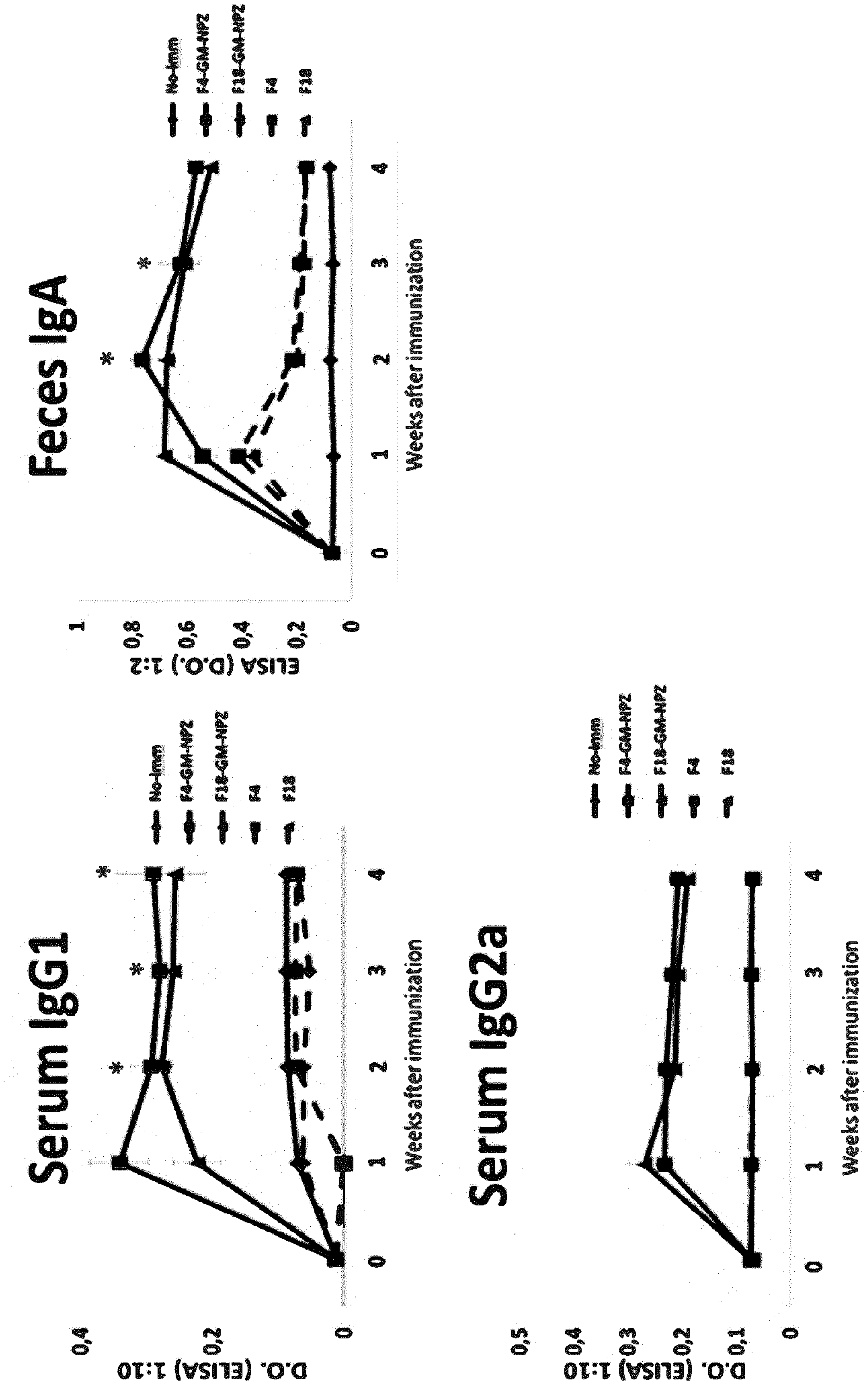
Serum IgG1
D.O. (ELISA) 1:10
0,4
0,2
0
0
1
2
3
4
Weeks after immunization
*
*
*
No-Imm
F4-GM-NPZ
F18-GM-NPZ
F4
F18
Feces IgA
ELISA (D.O.) 1:2
1
0,8
0,6
0,4
0,2
0
0
1
2
3
4
Weeks after immunization
*
*
No-Imm
F4-GM-NPZ
F18-GM-NPZ
F4
F18
Serum IgG2a
D.O. (ELISA) 1:10
0,5
0,4
0,3
0,2
0,1
0
0
1
2
3
4
Weeks after immunization
No-Imm
F4-GM-NPZ
F18-GM-NPZ
F4
F18

Figure 17

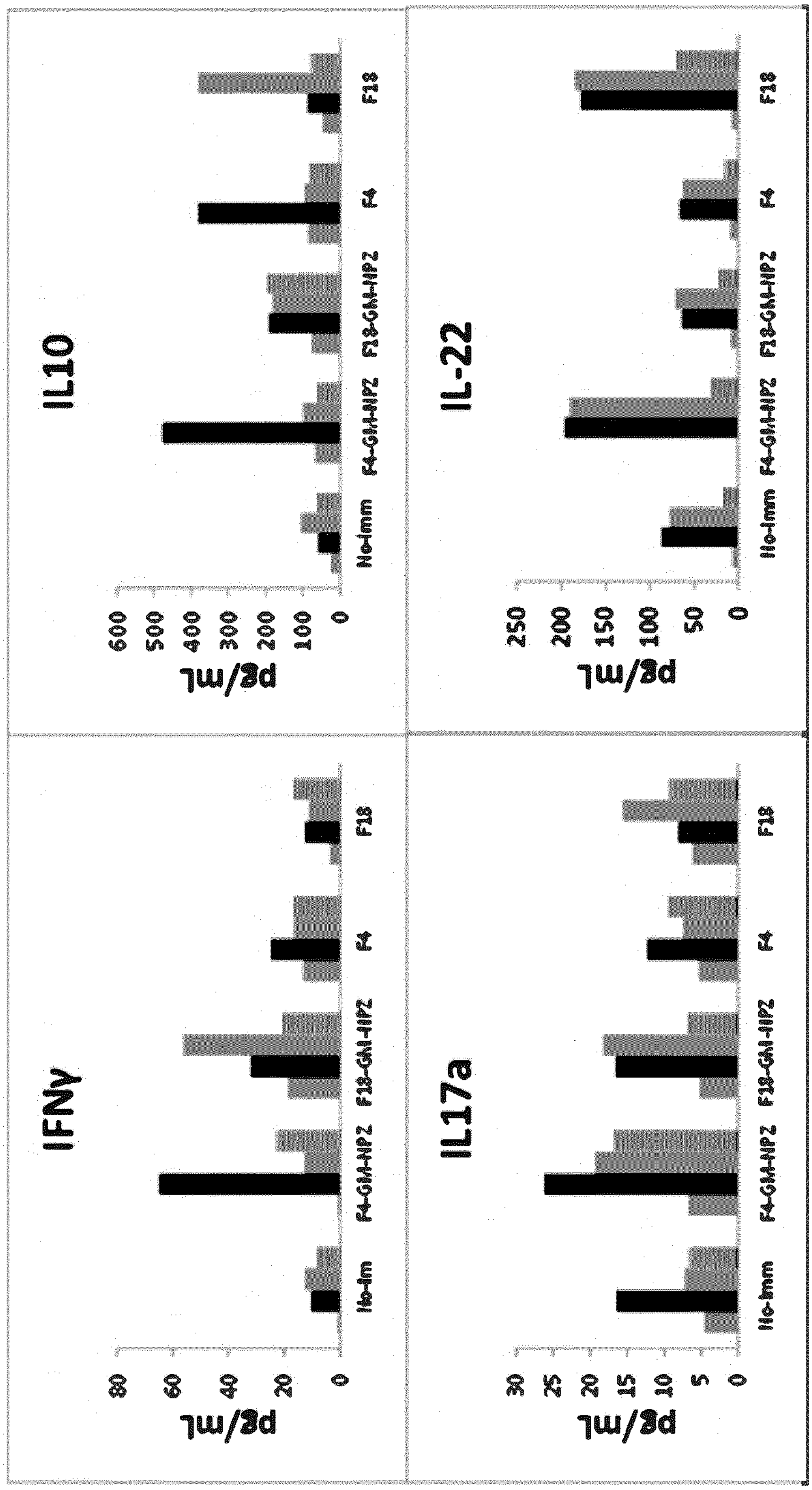
IFNγ
pg/mL
0
20
40
60
80
No-Imm
F4-GM-HPZ
F18-GM-HPZ
F4
F18
IL10
pg/mL
0
100
200
300
400
500
600
No-Imm
F4-GM-HPZ
F18-GM-HPZ
F4
F18
IL17a
pg/mL
0
5
10
15
20
25
30
No-Imm
F4-GM-HPZ
F18-GM-HPZ
F4
F18
IL-22
pg/mL
0
50
100
150
200
250
No-Imm
F4-GM-HPZ
F18-GM-HPZ
F4
F18

Figure 18A

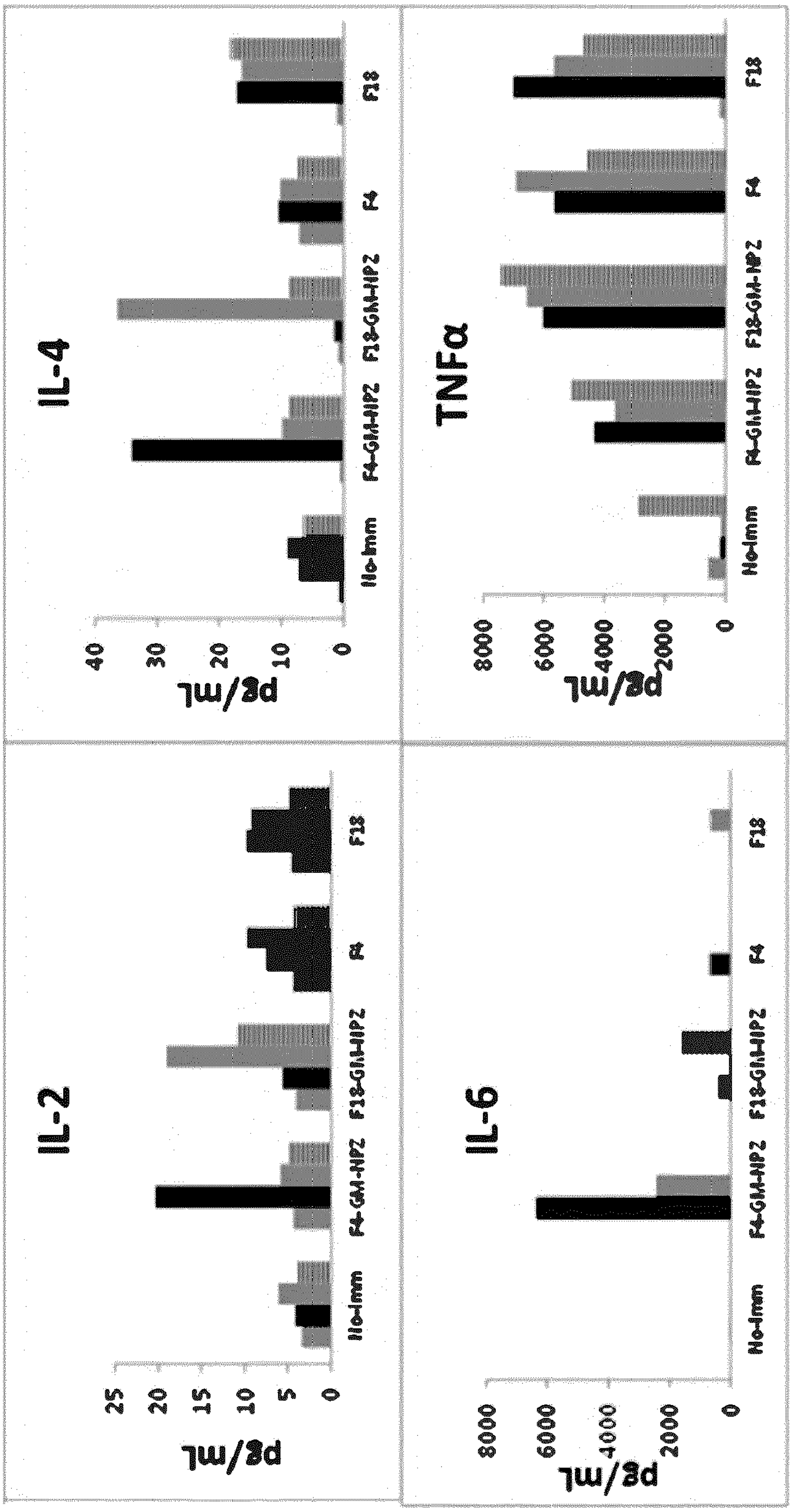


Figure 18B

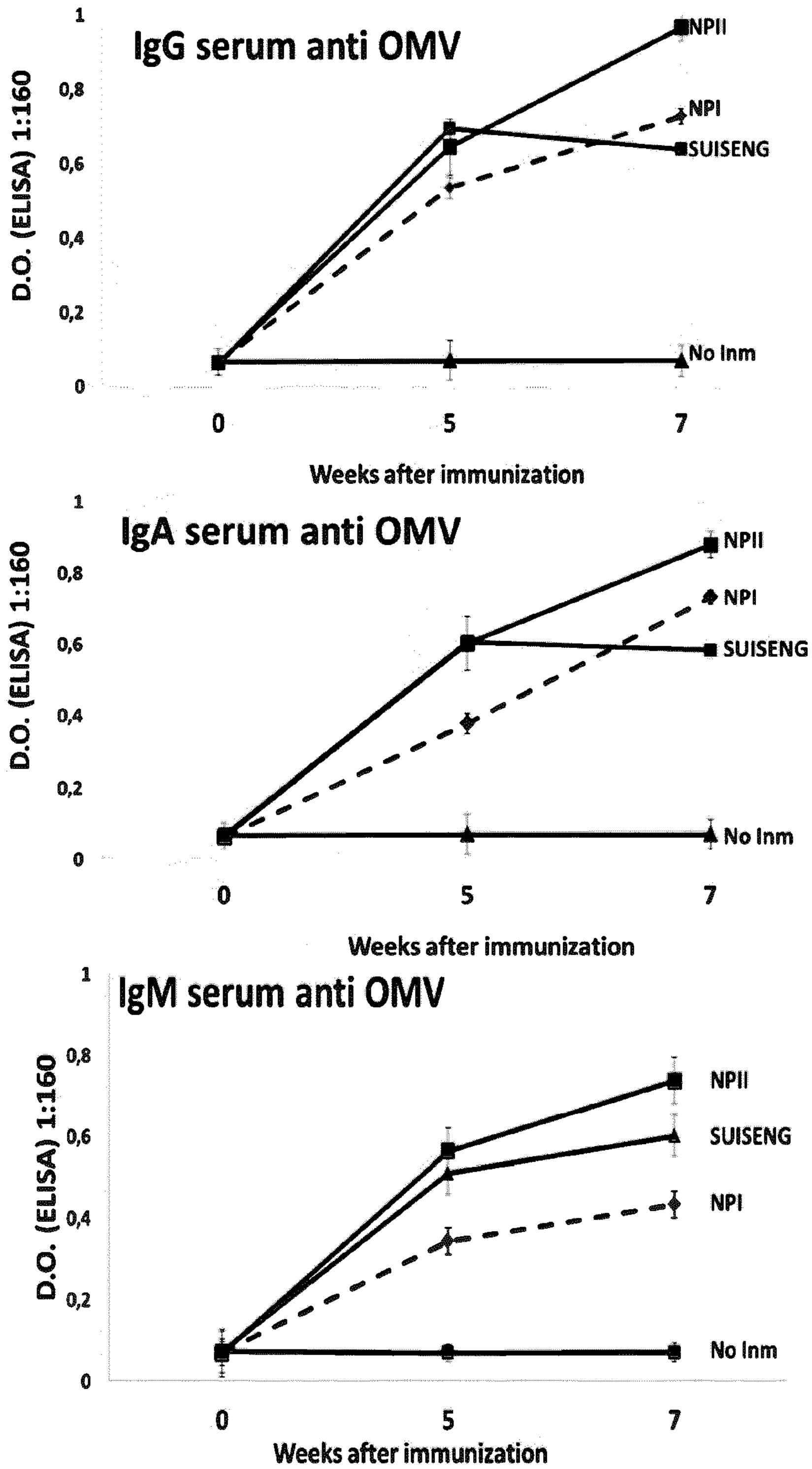
IgG serum anti OMV
D.O. (ELISA) 1:160
1
0,8
0,6
0,4
0,2
0
NPII
NPI
SUISENG
No Inm
0
5
7
Weeks after immunization
IgA serum anti OMV
D.O. (ELISA) 1:160
1
0,8
0,6
0,4
0,2
0
NPII
NPI
SUISENG
No Inm
0
5
7
Weeks after immunization
IgM serum anti OMV
D.O. (ELISA) 1:160
1
0,8
0,6
0,4
0,2
0
NPII
SUISENG
NPI
No Inm
0
5
7
Weeks after immunization

Figure 19A

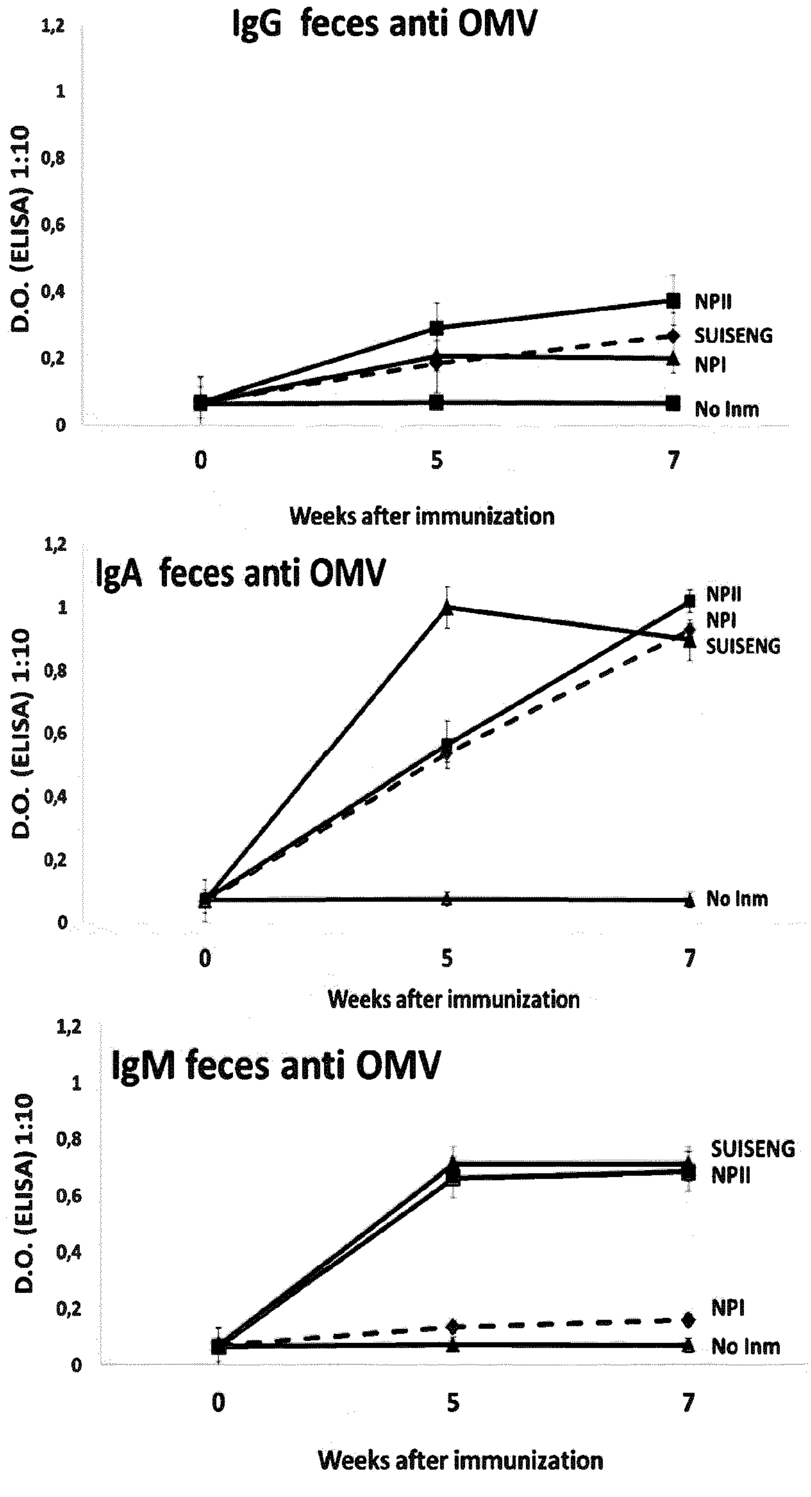
IgG feces anti OMV
D.O. (ELISA) 1:10
1,2
1
0,8
0,6
0,4
0,2
0
NPII
SUISENG
NPI
No Inm
0
5
7
Weeks after immunization
IgA feces anti OMV
D.O. (ELISA) 1:10
1,2
1
0,8
0,6
0,4
0,2
0
NPII
NPI
SUISENG
No Inm
0
5
7
Weeks after immunization
IgM feces anti OMV
D.O. (ELISA) 1:10
1,2
1
0,8
0,6
0,4
0,2
0
SUISENG
NPII
NPI
No Inm
0
5
7
Weeks after immunization

Figure 19B

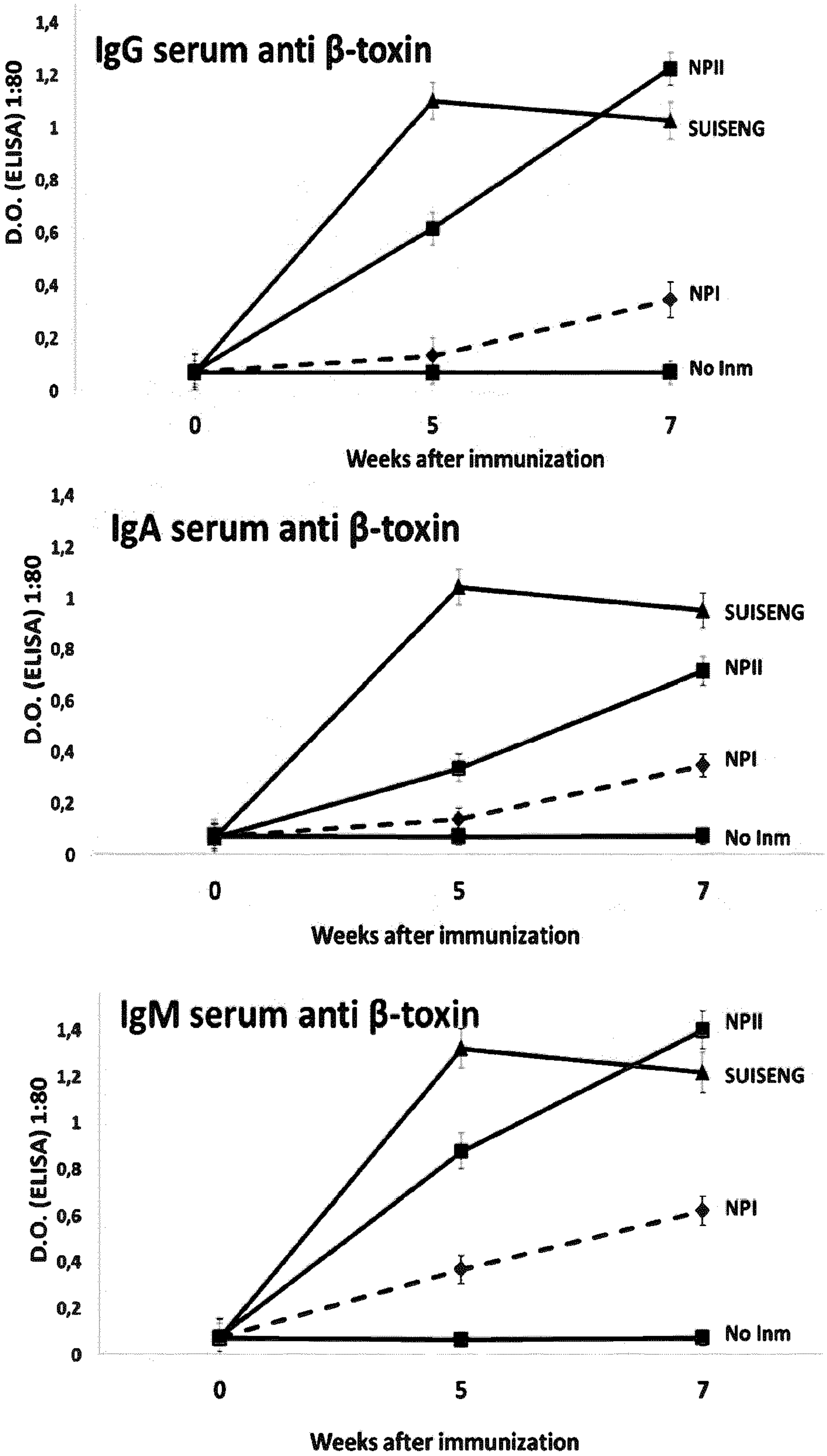
IgG serum anti β-toxin
D.O. (ELISA) 1:80
1,4
1,2
1
0,8
0,6
0,4
0,2
0
NPII
SUISENG
NPI
No Inm
0
5
7
Weeks after immunization
IgA serum anti β-toxin
D.O. (ELISA) 1:80
1,4
1,2
1
0,8
0,6
0,4
0,2
0
SUISENG
NPII
NPI
No Inm
0
5
7
Weeks after immunization
IgM serum anti β-toxin
D.O. (ELISA) 1:80
1,4
1,2
1
0,8
0,6
0,4
0,2
0
NPII
SUISENG
NPI
No Inm
0
5
7
Weeks after immunization

Figure 20A

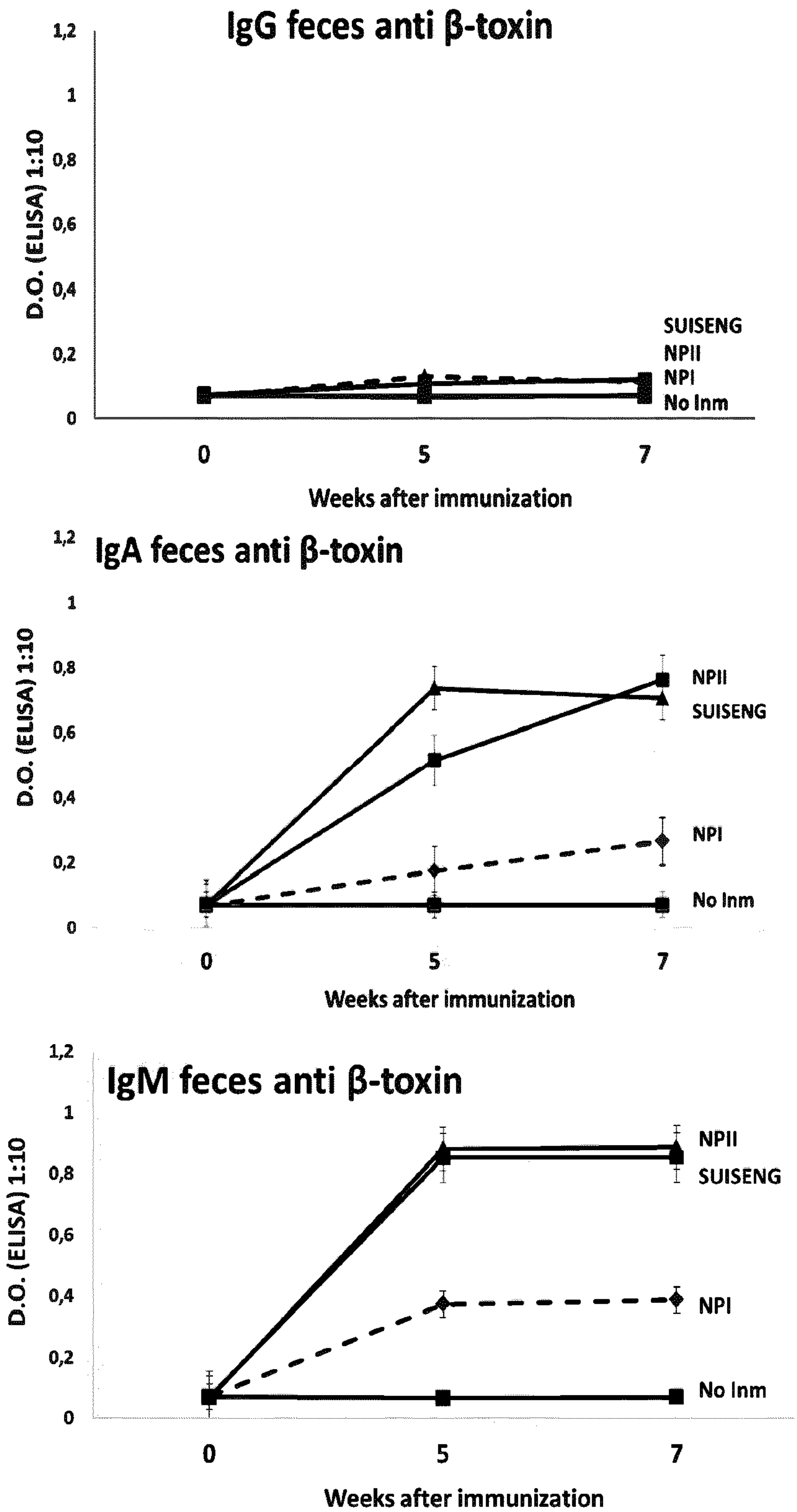
IgG feces anti β-toxin
D.O. (ELISA) 1:10
1,2
1
0,8
0,6
0,4
0,2
0
SUISENG
NPII
NPI
No Inm
0
5
7
Weeks after immunization
IgA feces anti β-toxin
NPII
SUISENG
NPI
No Inm
IgM feces anti β-toxin
NPII
SUISENG
NPI
No Inm

Figure 20B

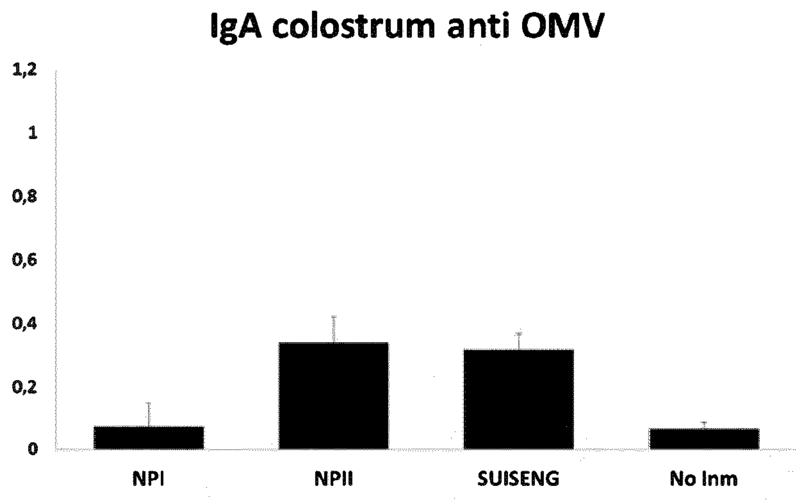
IgA colostrum anti OMV
1,2
1
0,8
0,6
0,4
0,2
0
NPI
NPII
SUISENG
No Inm

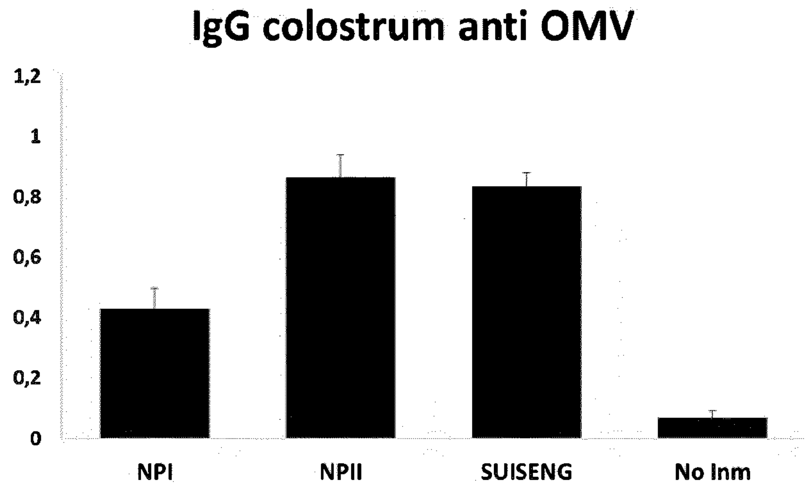
IgG colostrum anti OMV
1,2
1
0,8
0,6
0,4
0,2
0
NPI
NPII
SUISENG
No Inm

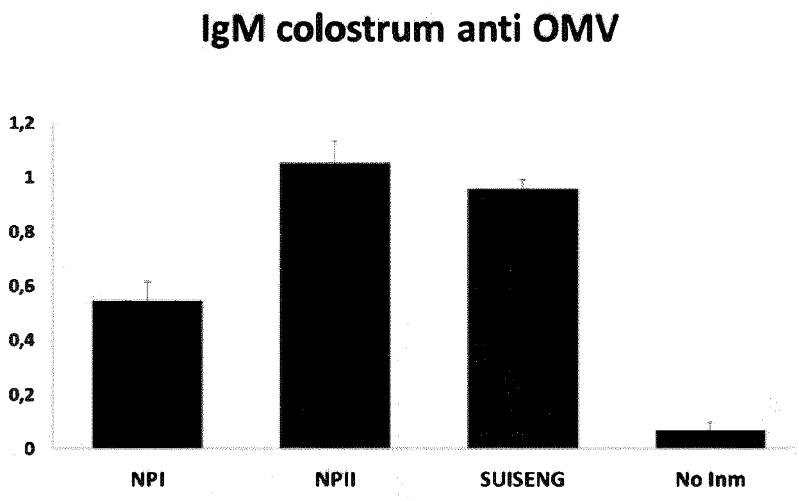
IgM colostrum anti OMV
1,2
1
0,8
0,6
0,4
0,2
0
NPI
NPII
SUISENG
No Inm

Figure 21A

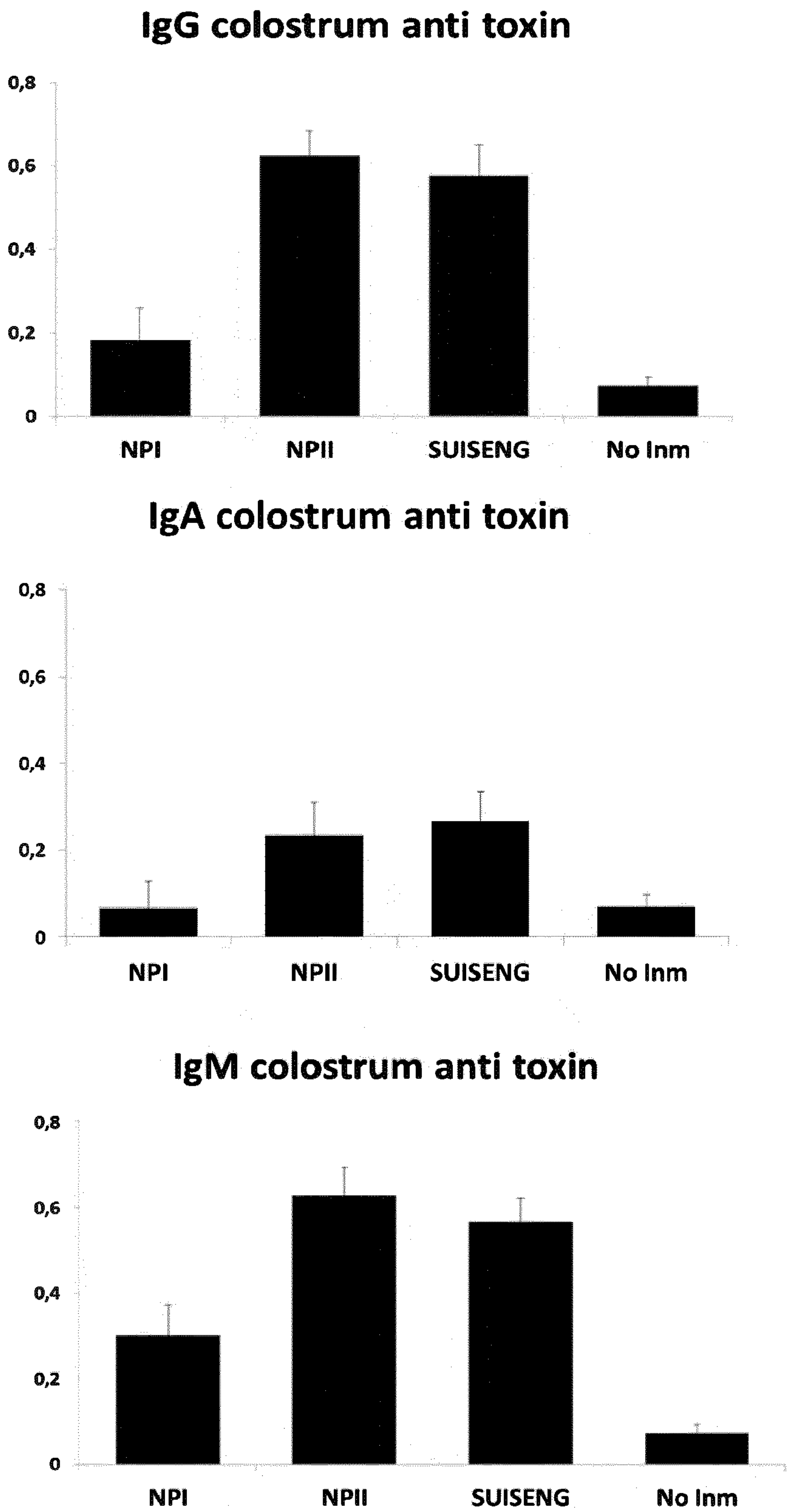
IgG colostrum anti toxin
0,8
0,6
0,4
0,2
0
NPI
NPII
SUISENG
No Inm
IgA colostrum anti toxin
0,8
0,6
0,4
0,2
0
NPI
NPII
SUISENG
No Inm
IgM colostrum anti toxin
0,8
0,6
0,4
0,2
0
NPI
NPII
SUISENG
No Inm

Figure 21B

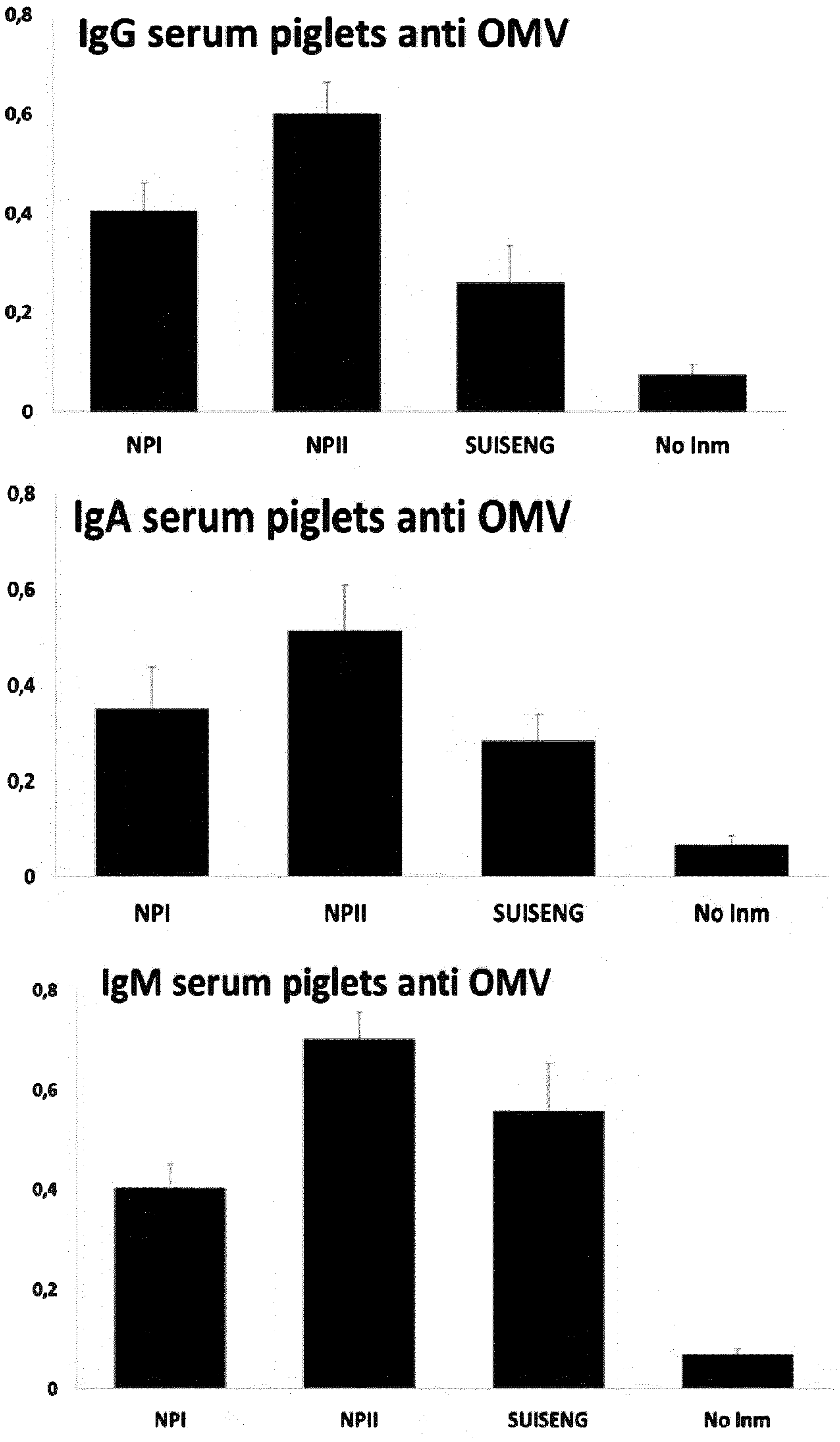
IgG serum piglets anti OMV
0,8
0,6
0,4
0,2
0
NPI
NPII
SUISENG
No Inm
IgA serum piglets anti OMV
0,8
0,6
0,4
0,2
0
NPI
NPII
SUISENG
No Inm
IgM serum piglets anti OMV
0,8
0,6
0,4
0,2
0
NPI
NPII
SUISENG
No Inm

Figure 22A

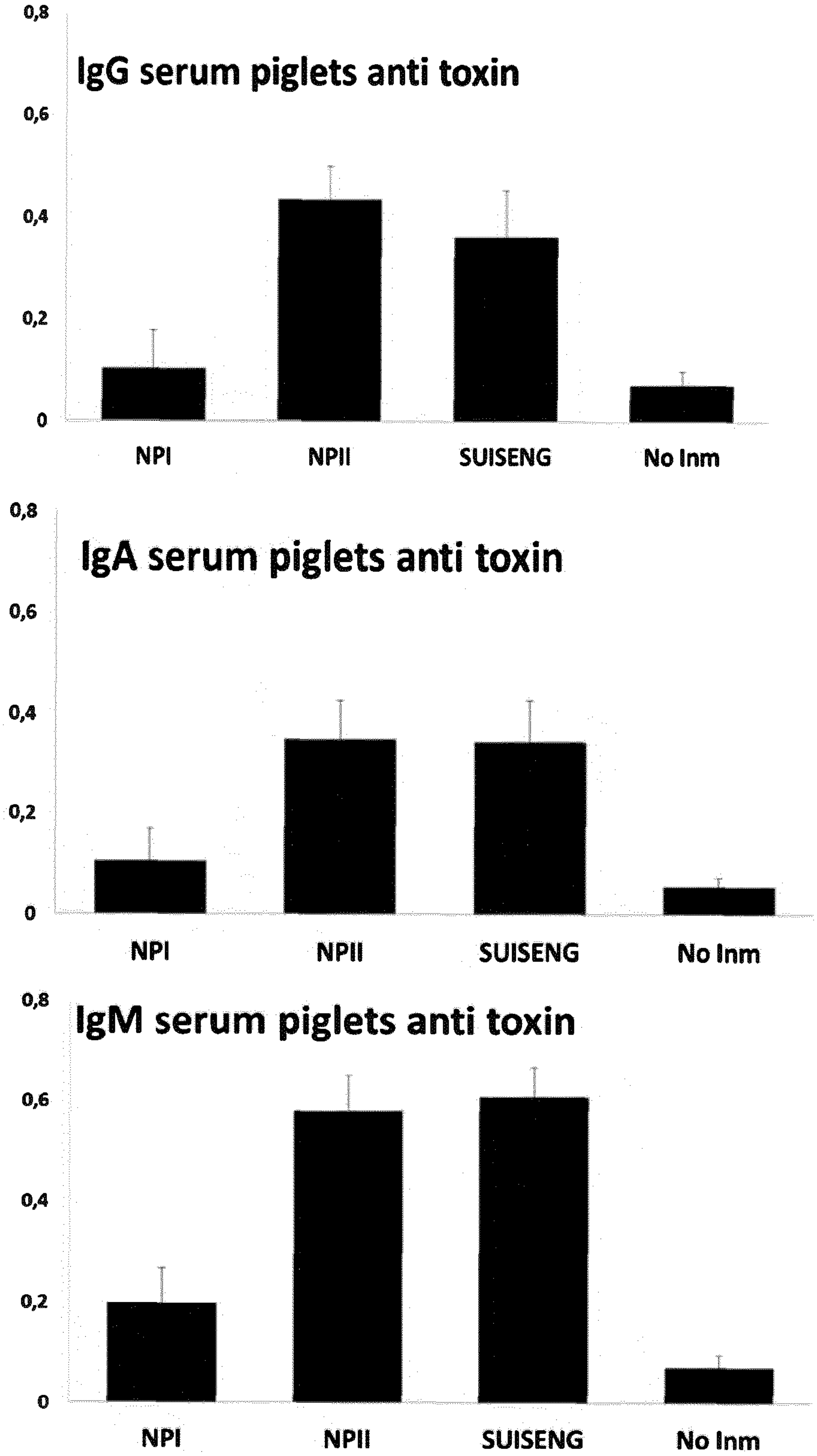
IgG serum piglets anti toxin
0,8
0,6
0,4
0,2
0
NPI
NPII
SUISENG
No Inm
IgA serum piglets anti toxin
0,8
0,6
0,4
0,2
0
NPI
NPII
SUISENG
No Inm
IgM serum piglets anti toxin
0,8
0,6
0,4
0,2
0
NPI
NPII
SUISENG
No Inm

Figure 22B

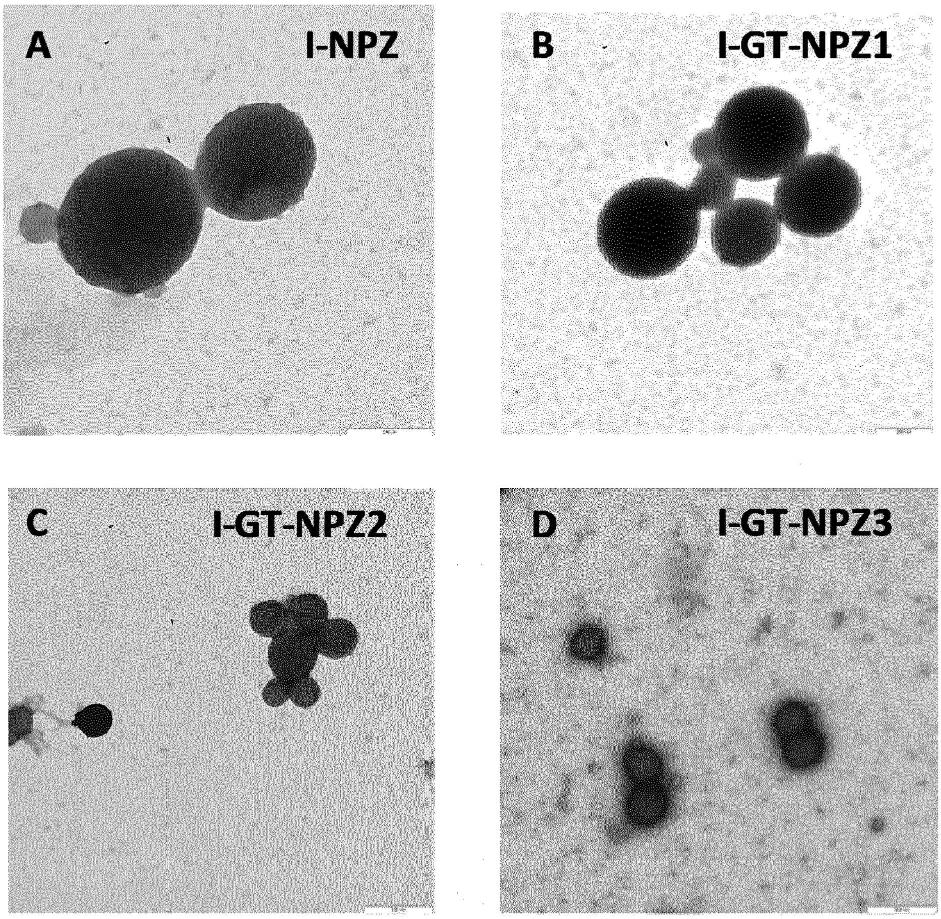


Figure 23

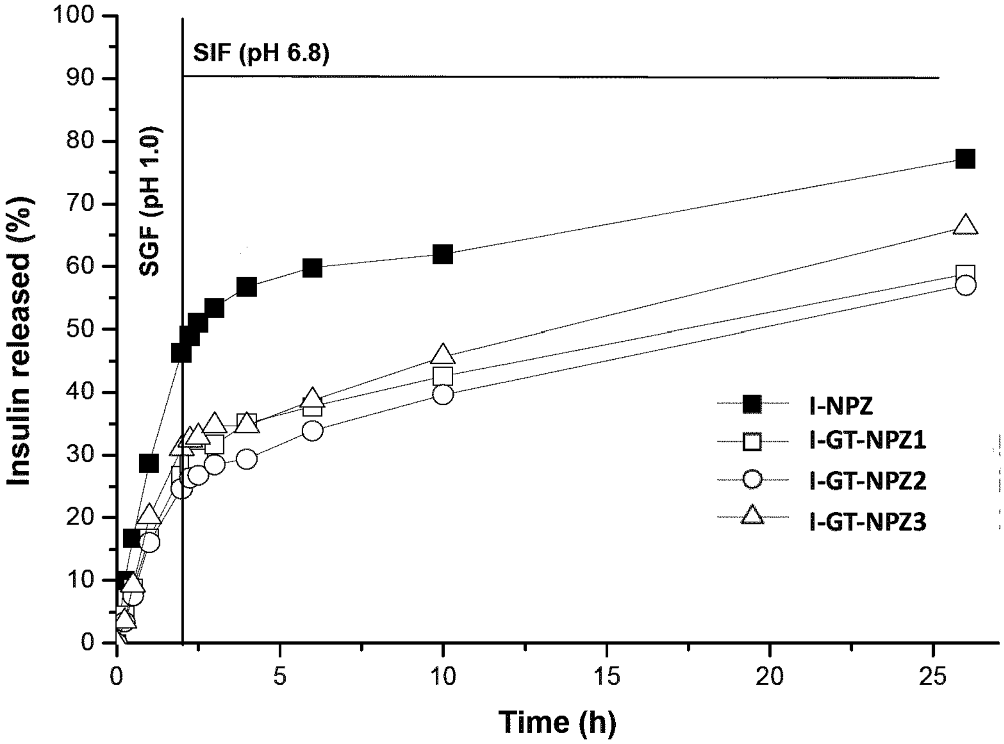


Figure 24

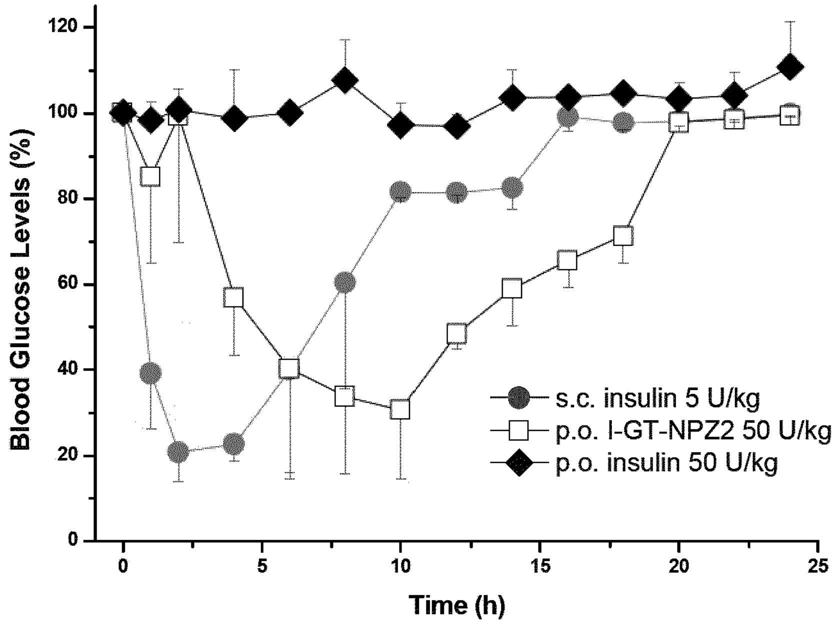


Figure 25

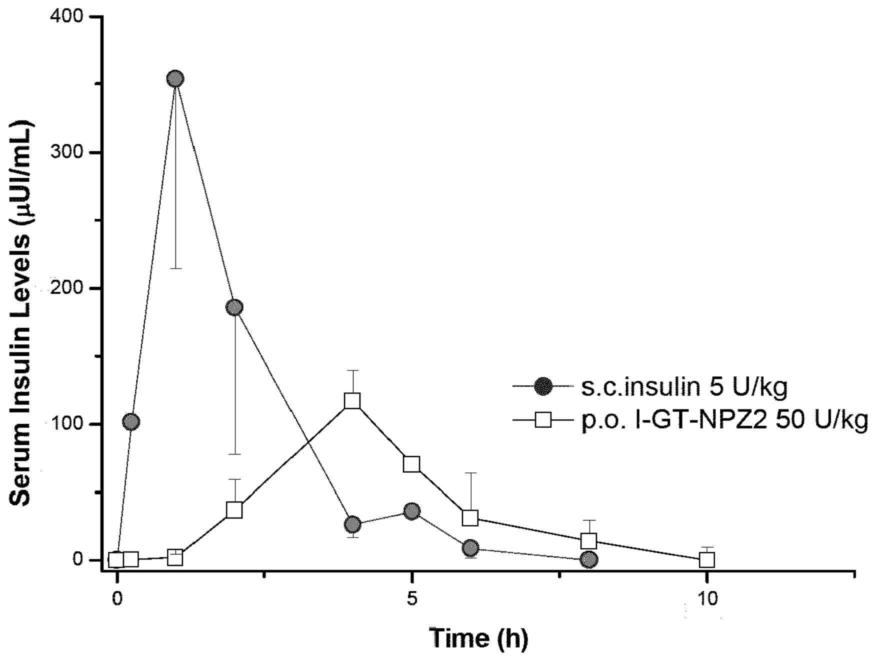

400
300
200
100
0
Serum Insulin Levels (μUI/mL)
0
5
10
Time (h)
s.c.insulin 5 U/kg
p.o. I-GT-NPZ2 50 U/kg


Figure 26

Europäisches Patentamt
European Patent Office
Office européen des brevets

# EUROPEAN SEARCH REPORT

Application Number
EP 18 38 2412

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SALMAN H.H. ET AL.: "Bioadhesive capacity and immunoadjuvant properties of thiamine-coated nanoparticles", VACCINE, ELSEVIER, AMSTERDAM, NL, vol. 25, no. 48, 23 November 2007 (2007-11-23), pages 8123-8132, XP026865307, ISSN: 0264-410X [retrieved on 2007-11-23] * the whole document * | 1-16 | INV. A61K9/51 A61K38/28 |
| A | IGLESIAS T. ET AL.: "In vitro evaluation of the genotoxicity of poly(anhydride) nanoparticles designed for oral drug delivery", INTERNATIONAL JOURNAL OF PHARMACEUTICS, vol. 523, March 2017 (2017-03), pages 418-426, XP002787222, * the whole document * | 1-16 | |
| A,D | PEÑALVA R. ET AL.: "Zein nanoparticles for oral folic acid delivery", JOURNAL OF DRUG DELIVERY SCIENCE AND TECHNOLOGY, vol. 30, 2015, pages 450-457, XP002787221, * the whole document * | 1-16 | TECHNICAL FIELDS SEARCHED (IPC) |
| A,D | US 5 679 377 A (BERNSTEIN HOWARD [US] ET AL) 21 October 1997 (1997-10-21) * examples * | 1-16 | A61K |

-/--

The present search report has been drawn up for all claims

2

| Place of search | Date of completion of the search | Examiner |
|---|---|---|
| Munich | 7 December 2018 | Giménez Miralles, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 38 2412

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | Inchaurraga L.: "Mucus-penetrating nanoparticles for the oral delivery of insulin", Tesis doctoral, Universidad de Navarra , 2017, XP002787224, Retrieved from the Internet: URL:http://dadun.unav.edu/handle/10171/43732 [retrieved on 2018-12-03] * abstract * ----- | 1-16 | |

TECHNICAL FIELDS SEARCHED (IPC)

The present search report has been drawn up for all claims

2

| Place of search | Date of completion of the search | Examiner |
|---|---|---|
| Munich | 7 December 2018 | Giménez Miralles, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 18 38 2412

This annex lists the patent family members relating to the patent documents cited in the above-mentioned European search report. The members are as contained in the European Patent Office EDP file on
The European Patent Office is in no way liable for these particulars which are merely given for the purpose of information.

07-12-2018

| Patent document cited in search report | Publication date | Patent family member(s) | Publication date |
|---|---|---|---|
| US 5679377 A | 21-10-1997 | AU 642932 B2 | 04-11-1993 |
| | | CA 2071867 A1 | 07-05-1991 |
| | | EP 0550436 A1 | 14-07-1993 |
| | | JP 2571874 B2 | 16-01-1997 |
| | | JP H05500961 A | 25-02-1993 |
| | | US 5679377 A | 21-10-1997 |
| | | WO 9106287 A1 | 16-05-1991 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5679377 A
- WO 2012007628 A1
- WO 2016087340 A1
- US 20090258050 A


### Non-patent literature cited in the description


- **WANG et al.** *Food Biophysics,* 2008, vol. 3, 174-181
- **MUTHUSELVI ; DHATHATHREYAN.** *Colloids and Surfaces B: Biointerfaces,* 2006, vol. 51, 39-43
- **SHUKLA ; CHERYAN.** *Industrial Crops and Products,* 2001, vol. 13, 171-192
- **PEÑALVA R et al.** *Journal of Drug Delivery Science and Technology,* 2015, vol. 30, 450-457
- **X. LI et al.** *Biomaterials,* 2013, vol. 34, 9678-9687
- **H.H. SALMAN et al.** *Vaccine,* 2007, vol. 25, 8123-8132
- **LAURA INCHAURRAGA et al.** *European Journal of Pharmaceutics and Biopharmaceutics,* 2015, vol. 97, 280-289
- **SOUSA I.P. et al.** *European Journal of Pharmaceutics and Biopharmaceutics,* 2015, vol. 97, 257-264
- **C. FAULÍ I TRILLO.** Tratado de Farmacia Galénica. 1993
- **SALMAN H.H. et al.** *Vaccine,* 2007, vol. 25, 8123-8132
- **R.A. CONE.** *Adv. Drug Deliv. Rev.,* 2009, vol. 61, 75-85
- **SALMAN ; HESHAM H. et al.** Bioadhesive mannosylated nanoparticles for oral drug delivery. *Journal of nanoscience and nanotechnology,* 2006, vol. 6.9-10, 3203-3209
- **P. ARESES et al.** *Mol. Imaging Biol.,* 2011, vol. 13, 1215-1223
- **M. ABDULKARIM et al.** *Eur. J. Pharm. Biopharm.,* 2015, vol. 97, 230-238
- **J. ROHRER et al.** *Eur. J. Pharm. Biopharm.,* 2016, vol. 98, 90-97
- **J R BENSON ; P E HARE.** O-phthalaldehyde: fluorogenic detection of primary amines in the picomole range. Comparison with fluorescamine and ninhydrin. *Proceedings of the National Academy of Sciences,* February 1975, vol. 72 (2), 619-622
- **P. ARBÓS et al.** *Int. J. Pharm.,* 2002, vol. 242, 129-136
- **CAMACHO AL ; SOUZA-REBOUCAS J ; IRACHE JM ; GAMAZO C.** Towards a non-living vaccine against Shigella flexneri: from the inactivation procedure to protection studies. *Methods,* 2013, vol. 60, 264-8
- **CAMACHO et al.** Towards a non-living vaccine against Shigella flexneri: from the inactivation procedure to protection studies. *Methods,* 2013, vol. 60, 264-8
- **LOWRY O.H ; R. N. J.** Protein measurement by the Folin phenol reagent. *J. Biol.Chem.,* 1951, vol. 193, 265-275
- **OSBORN, M. J.** Studies on the gram negative cell wall. I. Evidence for the role of the 2-Keto, 3-deoxyoctonate in the lipopolysaccharide of Salmonella thiphymurium. *Proc. Natl. Acad. Sci. USA,* 1963, vol. 50, 499-506